# EUROPEAN PATENT APPLICATION

(11) **EP 4 282 862 A1**
(43) Date of publication of application: **29.11.2023**
(21) Application number: 22175308.0
(22) Date of filing: 25.05.2022
(51) Int. Cl.: C07D 271/04, A61P 31/14

(54) **FLAVIVIRUS INHIBITORS**

(71) Applicant: IRBM S.P.A., 00071 Pomezia (RM) (IT); C.N.C.C.S. S.c.a.r.l. Collezione Nazionale Dei Composti Chimici e Centro Screening, 00071 Pomezia (RM) (IT)
(72) Inventor: ALLI, Cristina, Pomezia, Roma (IT); BRESCIANI, Alberto, Pomezia, Roma (IT); DI FABIO, Romano, Pomezia, Roma (IT); MONTALBETTI, Christian A.G.N., Pomezia, Roma (IT); ONTORIA ONTORIA, Jesus Maria, Pomezia, Roma (IT); PAONESSA, Giacomo, Pomezia, Roma (IT); QUOTADAMO, Antonio, Pomezia, Roma (IT); TORRENTE DE HARO, Esther, Pomezia, Roma (IT); BENCHEVA, Leda, Pomezia, Roma (IT); AMAUDRUT, Jerome, Pomezia, Roma (IT)
(74) Representative: Pace Napoleone, Maria

(57) **Abstract**

The invention relates to compounds that are inhibitors of Flavivirus NS2B-NS3 proteases and inhibit replication of flavivirus in cells. Compounds of this invention are useful alone or in combination with other agents for use in the treatment of infections caused by Flaviviruses, in particular by Dengue, West Nile, Zika, Japan Encephalitis viruses.

## Description

### SUMMARY OF THE INVENTION

The present invention relates to compounds that are inhibitors of Flavivirus NS2B-NS3 proteases and inhibit replication of flavivirus in cells. Compounds of this invention are useful alone or in combination with other agents for use in the treatment of infections caused by Flaviviruses, in particular by Dengue, West Nile, Zika, Japan Encephalitis viruses. The present invention also relates to pharmaceutical compositions containing said compounds.

### BACKGROUND

The Flavivirus genus of the Flaviviridae family includes more than 70 related arthropodborne viruses. The most common and representative members are the dengue virus (DENV) with four serotypes (DENV-1, -2, -3, and -4), Zika (ZIKV), West Nile (WNV), Japanese-encephalitis (JEV), Yellow Fever (YFV), and tick-borne encephalitis (TBEV) viruses. These are the causative agents for viral haemorrhagic fever and encephalitis in human beings. These viruses are transmitted primarily by *Aedes* mosquitos. Infections with flaviviruses are a continuing public health threat The mosquito-borne dengue virus serotypes 1-4 (DENV1-4) are included in the National Institute of Allergy and Infectious Disease (NIAID) list of Category A, B, or C emerging human pathogens along with yellow fever virus (YFV), West Nile virus (WNV), and Japanese encephalitis virus (JEV). DENVs cause serious illnesses associated with considerable morbidity and mortality. According to World Health Organization estimates, globally over 50 million people suffer from the symptoms of dengue fever annually, and of these at least 250,000 cases develop into dengue hemorrhagic fever (DHF) or dengue shock syndrome (DSS) resulting in considerable mortality, particularly among children in South Asian Countries. The World Health Organization estimates that 2.5 billion people (~40% of the world's population) live in areas that have increased risk of contracting dengue virus infections. Frequent outbreaks occur in the Americas including the continental U.S., mainly in the Southern states as well as in Puerto Rico and Hawaii.

Zika virus (ZIKV) has caused three major outbreaks in Pacific Ocean islands, Brazil, and other American countries, in which more than 1 million infections were reported and a large number of patients sought medical treatment. More seriously, Zika infection has been correlated with a 20-fold increased incidence of serious neurological disorders, including Guillain-Barre syndrome and more than 4,000 cases of microcephaly in newborns. Zika has quickly spread to 48 Pan-American countries and has recently been found to be transmitted through sex or body fluids.

Inhibitors of two major steps of flaviviral entry have been reported: (i) molecules that block virusreceptor interaction; (ii) compounds that prevent conformational change of viral envelope protein during virus-host membrane fusion (ACS Infect. Dis. 2015, 1, 9, 428-434).

As such, a need exists for more effective compositions and methods for treating viral infections caused by flaviviruses. In addition to vaccine development and vector control, the search for antiviral agents that alleviate symptoms in patients are of considerable interest. Flaviviruses share epidemiological, structural, and ecologic features and often different viruses can co-infect the same host. Therefore, the identification of broad-spectrum inhibitors is highly desirable either for known flaviviruses or for viruses that likely will emerge in the future. Strategies targeting both virus and host factors have been pursued to identify broad-spectrum antiflaviviral agents (https://doi.org/10.1002/cmdc.202000464).

The Flavivirus genome consists of a positive-sense single-stranded RNA which is ~11 kb in length, consisting of a single long open reading frame (ORF). The single ORF encodes a polyprotein that is further processed by proteases from the host and the viral NS2B-NS3 complex. The flavivirus protease is highly conserved and essential for virus replication. The viral polyprotein is processed to three structural proteins (Capsid, pr-Membrane, and Envelope) and seven non-structural (NS) proteins (NS1, NS2A, NS2B, NS3, NS4A, NS4B, and NS5). Three structural proteins form the virus shell, whereas seven NS proteins participate in the membranebound replication complex. Among these NS proteins, only NS3 and NS5 bear enzymatic function. (Pathogens 2022, 11 293). Flaviviral replication depends on the NS3 protease, which is a 69 kDa protein with two domains that have different enzymatic functions: a trypsin-like serine protease domain located within the *N*-terminal region; while the C-terminal region has the activities of an RNA-helicase and an RNA-stimulated NTPase. Moreover, it is only entirely activated when associated with its cofactor, namely NS2B; forming an enzymatic complex, NS2B-NS3. Considering this, NS2B-NS3 proteases represent a valuable target for the development of new antiviral compounds, which act inhibiting their replication complex and leading to the flaviviral death.

Flaviviral proteases share a high degree of structural similarity and substrate-recognition profile, which may facilitate a strategy towards development of pan-flaviviral protease inhibitors. However, the success of various drug discovery attempts during the last decade has been limited by the nature of the viral enzyme as well as a lack of robust structural templates. Small-molecular, structurally diverse protease inhibitors have been reported to reach affinities in the lower micromolar range. Peptide-based, substrate-derived compounds are often nanomolar inhibitors, however, with highly compromised drug-likeness. With some exceptions, the antiviral cellular activity of most of the reported compounds have been patchy and insufficient for further development. Recent progress has been made in the elucidation of inhibitor binding using different structural methods. This will hopefully lead to more rational attempts for the identification of various lead compounds that may be successful in cellular assays, animal models and ultimately translated to patients.

WO2021/175437 and WO2021/175670 provides non-peptidic (4-phenoxy)-phenylglycine and (4-benzyloxy)-phenylglycine derivatives as inhibitors of the NS2B-NS3 serine protease for use in the treatment and prevention of flaviviruses infection.

The present invention yields a novel series of drug-like, broadly active inhibitors of flavivirus proteases.

### SUMMARY OF THE INVENTION

The present invention provides a series of compounds targeting the NS2B-NS3 protease. The compounds of the invention are sydnone imine derivatives thus having a mesoionic heterocyclic core properly substituted with a nitrogen derivative substituent at the imino nitrogen, an alkyl or aromatic substituent at position 1. The chemotype discovered in the present invention results in extremely potent ZIKV NS2B-NS3 protease inhibitors, active also on the JEV, Dengue and WNV proteases. The compounds of the invention are capable of inhibiting replication of the above viruses in cells.

It is therefore an object of the invention a compound of general Formula (I): 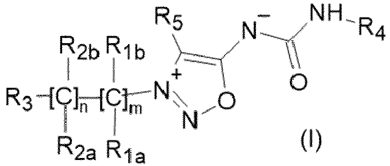 wherein:
m and n are each independently selected from 0, 1 and 2;
R₁ₐ and R_{1b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₁ₐ and R_{1b} are linked together to form a C₃₋₆-cycloalkyl ring or a C₄₋₆- heterocycloalkyl ring;
or R₁ₐ is linked to R₃ to form a partially unsaturated aromatic or heteroaromatic bicycle;
or R_{1b} doesn't exist and C-R₁ₐ is C=CH₂;
R₂ₐ and R_{2b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₂ₐ and R_{2b} are linked together to form a C₃₋₆-cycloalkyl ring or a C₄₋₆-heterocycloalkyl ring; or any two of R₁ₐ and R₂ₐ or of R_{1b} and R_{2b} are linked together to form a C₃₋₆cycloalkyl or a C₃₋₆heterocycloalkyl;
R₃ is heterocycloalkyl, aromatic or heteroaromatic ring, each of said heterocycloalkyl, aromatic or heteroaromatic ring being optionally substituted with one or more substituents independently selected from halogen, OH, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, haloC₁₋₆alkyl, C₁₋₆alkoxy, cyano, B(OH)₂, aryl, heteroaryl wherein said aryl or heteroaryl ring are optionally substituted with one or more substituents each independently selected from halogen, hydroxy, cyano, NH₂, NHCOCH₃, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, N-methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and N-methylpyperazine;
R₄ is a ring of formula (II): 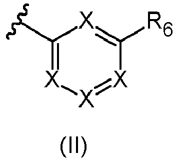 wherein
   - R₆ is CF₃, F, Cl, CN or C₁₋₃alkyl;
   - X is CR₇ or N;
   - R₇ is at each occurrence independently selected from H, halogen, C₁₋₆alkyl, C₁₋₆alkoxy,
      haloC₁₋₆alkyl, hydroxy;
      or R₄ is 4-trifluoromethylphenyl, thiophene, thiazole, imidazole, pyrazole, oxazole, oxadiazole, naphthalene, quinoline, isoquinoline, quinazoline or napthyridine, wherein each of said ring is optionally substituted with one or more substituents selected from halogen, C₁₋₆alkyl, hydroxy, haloC₁₋₆alkyl, C₁₋₆alkoxy, NH₂, NHC(O)C₁₋₆alkyl, CN;
      or R₄ is phenyl and R₃ is a phenyl-heteroaryl system optionally substituted with one or more substituents selected from halogen, C₁₋₆alkyl, C₁₋₆alkoxy;
      R₅ is H, C₁₋₆alkyl, C₁₋₆haloalkyl or halogen;
      or a pharmaceutically acceptable salt, tautomer, solvate, or stereoisomer thereof; provided that compounds indicated below are not included:

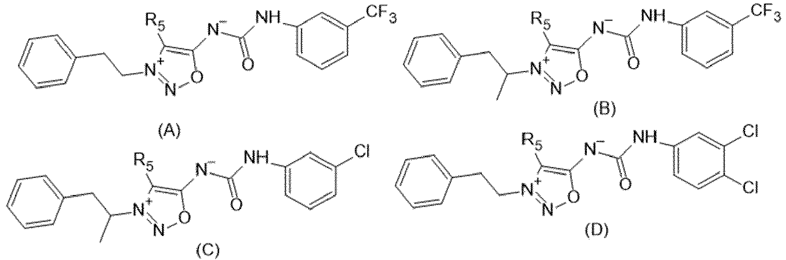

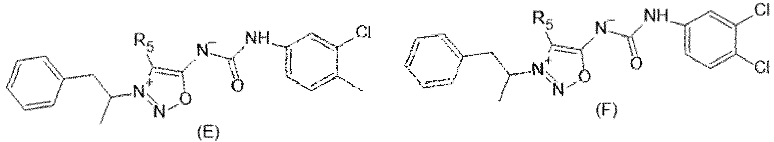

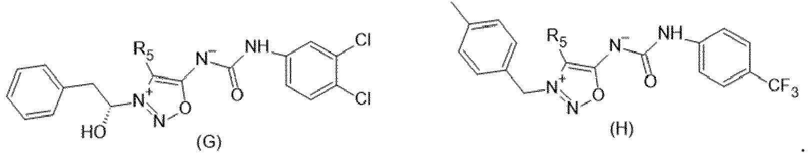

Preferably R_{1b} and/or R_{2b} are H; and/or m and n are each independently selected from 0, 1; still preferably R₃ is phenyl, naphtyl, biphenyl, phenyl-heteroaryl or bis-heteroaryl wherein each of said phenyl, naphtyl or heteroaryl ring is optionally substituted with one or more substituents each independently selected from halogen, hydroxy, cyano, B(OH)₂, NH₂, NHCOCH₃, NHCOOC₁₋₆alkyl, NHC₁₋₃alkyl, N(C₁₋₃alkyl)₂, C₁₋₆alkyl, C₁₋₆alkoxy, pyrrolidine, pyperidine, morpholine, cyclopropyl, cyclobutyl, cyclopentyl, cyclohexyl, wherein said C₁₋₆alkyl, C₁₋₆alkoxy are optionally substituted with one or more halogen, hydroxy, NH₂, N(CH₃)₂, morpholine or pyrrolidine.

Still preferably R₄ is 3-trifluoromethylphenyl, 4-trifluoromethylphenyl, naphthyl, 5-(trifluoromethyl)thiazol-2-yl, 3-chlorophenyl, 3-fluoro-5-trifluoromethylphenyl, quinolinyl, 5-(trifluoromethyl)pyridin-3-yl, 4-(trifluoromethyl)pyridin-2-yl, 3-methyl-5-trifluoromethylphenyl, 3 -methoxy-5 -trifluoromethylphenyl, 3 -cyanophenyl.

Preferably the compound is selected from the following list:
- (3-(1-phenylpropan-2-yl)-1,2,3 -oxadiazol-3 -ium-5 -yl)((4-(trifluoromethyl)phenyl) carbamoyl)amide;
- (naphthalen-2-ylcarbamoyl)(3-(1-phenylpropan-2-yl)-1,2,3-oxadiazol-3-ium-5-yl)amide;
- (3-(1-phenylpropan-2-yl)-1,2,3-oxadiazol-3-ium-5-yl)((5-(trifluoromethyl)thiazol-2-yl)carbamoyl)amide;
- (3-benzyl-1,2,3-oxadiazol-3-ium-5-yl)((3-(trifluoromethyl)phenyl)carbamoyl)amide;
- (3-phenyl-1,2,3-oxadiazol-3-ium-5-yl)((3-(trifluoromethyl)phenyl)carbamoyl)amide;
- ((3-chlorophenyl)carbamoyl)(3-(1-phenylpropan-2-yl)-1,2,3-oxadiazol-3 -ium-5-yl)amide
- (3-(1-(3,4-dichlorophenyl)propan-2-yl)-1,2,3-oxadiazol-3-ium-5-yl)((3-(trifluoromethyl) phenyl)carbamoyl)amide;
- (3 -(2,3 -dihydro- 1*H*-inden-2-yl)-1,2,3 -oxadiazol-3 -ium-5-yl)((3-(trifluoromethyl)phenyl) carbamoyl)amide;
- ((3-fluoro-5-(trifluoromethyl)phenyl)carbamoyl)(3-(1-phenylpropan-2-yl)-1,2,3-oxadiazol-3 -ium-5 -yl)amide;
- (3-(1-phenylpropan-2-yl)-1,2,3-oxadiazol-3-ium-5-yl)(quinolin-6-ylcarbamoyl)amide;
- (R)-(3 -(1-phenylpropan-2-yl)-1,2,3 -oxadiazol -3 -ium-5 -yl)((3 -(trifluoromethyl)phenyl) carbamoyl)amide;
- (*S*)-(3-(1-phenylpropan-2-yl)-1,2,3-oxadiazol-3 -ium-5-yl)((3-(trifluoromethyl)phenyl) carbamoyl)amide;
- (3-(1-phenylpropan-2-yl)-1,2,3-oxadiazol-3-ium-5-yl)((5-(trifluoromethyl)pyridin-3-yl)carbamoyl)amide;
- (3-(1-phenylpropan-2-yl)-1,2,3-oxadiazol-3-ium-5-yl)((4-(trifluoromethyl)pyridin-2-yl)carbamoyl)amide;
- (3-([1,1'-biphenyl]-4-ylmethyl)-1,2,3-oxadiazol-3 -ium-5-yl)((3-(trifluoromethyl)phenyl) carbamoyl)amide;
- (3-(naphthalen-2-ylmethyl)-1,2,3-oxadiazol-3 -ium-5-yl)((3-(trifluoromethyl)phenyl) carbamoyl)amide;
- (3-(4-bromobenzyl)-1,2,3-oxadiazol-3 -ium-5-yl)((3-(trifluoromethyl)phenyl)carbamoyl) amide;
- (3-(4-(thiophen-3-yl)benzyl)-1,2,3-oxadiazol-3 -ium-5-yl)((3-(trifluoromethyl)phenyl) carbamoyl)amide;
- (3-((2'-methyl-[1,1'-biphenyl]-4-yl)methyl)-1,2,3-oxadiazol-3 -ium-5-yl)((3-(trifluoromethyl)phenyl)carbamoyl)amide;
- (3-(4-(pyridin-3-yl)benzyl)-1,2,3-oxadiazol-3-ium-5-yl)((3-(trifluoromethyl)phenyl) carbamoyl)amide;
- (3-(4-(1-methyl-1*H*-pyrazol-4-yl)benzyl)-1,2,3-oxadiazol-3 -ium-5-yl)((3-(trifluoromethyl)phenyl)carbamoyl)amide;
- (3-benzyl-4-chloro-1,2,3-oxadiazol-3-ium-5-yl)((3-(trifluoromethyl)phenyl)carbamoyl) amide;
- (3-(1-phenylcyclopropyl)-1,2,3-oxadiazol-3-ium-5 -yl)((3 -(trifluoromethyl)phenyl) carbamoyl)amide;
- (3-(4-(pyrimidin-5-yl)benzyl)-1,2,3-oxadiazol-3 -ium-5-yl)((3-(trifluoromethyl)phenyl) carbamoyl)amide;
- (3-([1,1'-biphenyl]-3-ylmethyl)-1,2,3-oxadiazol-3 -ium-5-yl)((3-(trifluoromethyl)phenyl) carbamoyl)amide;
- (3-(2-phenylpropyl)-1,2,3-oxadiazol-3-ium-5-yl)((3-(trifluoromethyl)phenyl)carbamoyl) amide;
- (3-(pyridin-3-ylmethyl)-1,2,3-oxadiazol-3 -ium-5-yl)((3-(trifluoromethyl)phenyl) carbamoyl)amide;
- (*R*)-(3-(2-hydroxy-1-phenylethyl)-1,2,3-oxadiazol-3-ium-5-yl)((3-(trifluoromethyl) phenyl)carbamoyl)amide;
- (*S*)-(3-(2-hydroxy-1-phenylethyl)-1,2,3-oxadiazol-3-ium-5-yl)((3-(trifluoromethyl) phenyl)carbamoyl)amide;
- (*R*)-(3-(1-hydroxy-3-phenylpropan-2-yl)-1,2,3-oxadiazol-3-ium-5 -yl)((3 - (trifluoromethyl)phenyl)carbamoyl)amide;
- ((3-methyl-5-(trifluoromethyl)phenyl)carbamoyl)(3-(1-phenylpropan-2-yl)-1,2,3-oxadiazol-3 -ium-5 -yl)amide;
- ((3-methoxy-5-(trifluoromethyl)phenyl)carbamoyl)(3-(1-phenylpropan-2-yl)-1,2,3-oxadiazol-3 -ium-5 -yl)amide;
- (3-(1-phenylvinyl)-1,2,3-oxadiazol-3 -ium-5-yl)((3-(trifluoromethyl)phenyl)carbamoyl) amide;
- (3-(3-phenylprop-1-en-2-yl)-1,2,3 -oxadiazol -3 -ium-5 -yl)((3-(trifluoromethyl)phenyl) carbamoyl)amide;
- (*S*)-(3-(1-fluoro-3 -phenylpropan-2-yl)-1,2,3 -oxadiazol-3 -ium-5 -yl)((3 -(trifluoromethyl) phenyl)carbamoyl)amide;
- (3-(1-phenylethyl)-1,2,3-oxadiazol-3-ium-5-yl)((3-(trifluoromethyl)phenyl)carbamoyl) amide;
- (3-(4-(pyridin-4-yl)benzyl)-1,2,3-oxadiazol-3-ium-5-yl)((3-(trifluoromethyl)phenyl) carbamoyl)amide;
- (*S*)-(3-(1-phenyl-3-(pyrrolidin-1-yl)propan-2-yl)-1,2,3-oxadiazol-3 -ium-5-yl)((3-(trifluoromethyl)phenyl)carbamoyl)amide;
- (3-(4-(2-methoxypyrimidin-5-yl)benzyl)-1,2,3-oxadiazol-3-ium-5-yl)((3-(trifluoromethyl)phenyl)carbamoyl)amide;
- (3-(4-(pyrazin-2-yl)benzyl)-1,2,3-oxadiazol-3 -ium-5-yl)((3-(trifluoromethyl)phenyl) carbamoyl)amide;
- (3-(2,2,2-trifluoro-1-phenylethyl)-1,2,3-oxadiazol-3-ium-5-yl)((3-(trifluoromethyl) phenyl)carbamoyl)amide;
- (R)-(3 -(1-hydroxy-3 -phenylpropan-2-yl)-1,2,3 -oxadiazol-3 -ium-5 -yl)((3 - (trifluoromethyl)phenyl)carbamoyl)amide;
- (3-(3-bromobenzyl)-1,2,3-oxadiazol-3 -ium-5-yl)((3-(trifluoromethyl)phenyl)carbamoyl) amide;
- (3-(4-iodobenzyl)-1,2,3-oxadiazol-3-ium-5-yl)((3-(trifluoromethyl)phenyl)carbamoyl) amide;
- (3-(4-(isoxazol-4-yl)benzyl)-1,2,3-oxadiazol-3-ium-5-yl)((3-(trifluoromethyl)phenyl) carbamoyl)amide;
- (R)-(3 -(1-amino-3 -phenylpropan-2-yl)-1,2,3 -oxadiazol-3 -ium-5 -yl)((3 -(trifluoromethyl) phenyl)carbamoyl)amide;
- (3-(3-(pyrimidin-5-yl)benzyl)-1,2,3-oxadiazol-3 -ium-5-yl)((3-(trifluoromethyl)phenyl) carbamoyl)amide;
- (3-(3-(pyridin-3-yl)benzyl)-1,2,3-oxadiazol-3-ium-5-yl)((3-(trifluoromethyl)phenyl) carbamoyl)amide;
- (3 -(3 -(1-methyl- 1*H*-pyrazol-4-yl)benzyl)-1,2,3 -oxadiazol-3 -ium-5 -yl)((3 - (trifluoromethyl)phenyl)carbamoyl)amide;
- (3-(3-(2-methoxypyrimidin-5-yl)benzyl)-1,2,3-oxadiazol-3-ium-5-yl)((3-(trifluoromethyl)phenyl)carbamoyl)amide;
- (*S*)-(3-(1-amino-3 -phenylpropan-2-yl)-1,2,3-oxadiazol-3-ium-5 -yl)((3 -(trifluoromethyl) phenyl)carbamoyl)amide;
- (3-(4-iodobenzyl)-1,2,3-oxadiazol-3-ium-5-yl)((5-(trifluoromethyl)pyridin-3-yl) carbamoyl)amide;
- (3-((4'-(morpholinomethyl)-[1,1'-biphenyl]-4-yl)methyl)-1,2,3-oxadiazol-3 -ium-5-yl)((3-(trifluoromethyl)phenyl)carbamoyl)amide;
- (3-(4-(pyrimidin-5-yl)benzyl)-1,2,3-oxadiazol-3 -ium-5-yl)((5-(trifluoromethyl)pyridin-3-yl)carbamoyl)amide;
- (3-(4-(2-methoxypyrimidin-5-yl)benzyl)-1,2,3-oxadiazol-3-ium-5-yl)((5-(trifluoromethyl)pyridin-3-yl)carbamoyl)amide;
- (3-((1*R*,2*S*)-2-phenylcyclopentyl)-1,2,3-oxadiazol-3-ium-5-yl)((3-(trifluoromethyl) phenyl)carbamoyl)amide;
- (3-(4-(4-methylpyrimidin-5-yl)benzyl)-1,2,3-oxadiazol-3 -ium-5-yl)((3-(trifluoromethyl) phenyl)carbamoyl)amide;
- (3-(4-(pyridazin-4-yl)benzyl)-1,2,3-oxadiazol-3 -ium-5-yl)((3-(trifluoromethyl)phenyl) carbamoyl)amide;
- (3-(4-(4-methoxypyrimidin-5-yl)benzyl)-1,2,3-oxadiazol-3-ium-5-yl)((3-(trifluoromethyl)phenyl)carbamoyl)amide;
- (3-(4-(2-methoxypyridin-4-yl)benzyl)-1,2,3-oxadiazol-3 -ium-5-yl)((3-(trifluoromethyl) phenyl)carbamoyl)amide;
- (3-(4-(2-hydroxypyridin-4-yl)benzyl)-1,2,3-oxadiazol-3-ium-5-yl)((3-(trifluoromethyl) phenyl)carbamoyl)amide;
- (*S*)-(3-(1-([1,1'-biphenyl]-4-yl)-3 -aminopropan-2-yl)-1,2,3-oxadiazol-3-ium-5-yl)((3-(trifluoromethyl)phenyl)carbamoyl)amide;
- (*R*)-(3-(1-([1,1''-biphenyl]-4-yl)-3-aminopropan-2-yl)-1,2,3-oxadiazol-3-ium-5-yl)((3-(trifluoromethyl)phenyl)carbamoyl)amide;
- (3-(4-(pyridin-4-yl)benzyl)-1,2,3-oxadiazol-3-ium-5-yl)((5-(trifluoromethyl)pyridin-3-yl)carbamoyl)amide;
- (R)-(3 -(1-(4-bromophenyl)ethyl)-1,2,3 -oxadiazol-3 -ium-5 -yl)((3 -(trifluoromethyl) phenyl)carbamoyl)amide;
- (*S*)-(3-(1-(4-bromophenyl)ethyl)-1,2,3-oxadiazol-3-ium-5 -yl)((3 -(trifluoromethyl) phenyl)carbamoyl)amide;
- (*S*)-(3-(1-([1,1''-biphenyl]-4-yl)-3 -acetamidopropan-2-yl)-1,2,3-oxadiazol-3 -ium-5-yl)((3-(trifluoromethyl)phenyl)carbamoyl)amide;
- (*R*)-(3-(1-([1,1'-biphenyl]-4-yl)-3-acetamidopropan-2-yl)-1,2,3-oxadiazol-3 -ium-5-yl)((3-(trifluoromethyl)phenyl)carbamoyl)amide;
- (R)-(3 -(1-(4-(pyrimidin-5-yl)phenyl)ethyl)-1,2,3-oxadiazol-3-ium-5 -yl)((3 - (trifluoromethyl)phenyl)carbamoyl)amide;
- (R)-(3 -(1-(4-bromophenyl)ethyl)-1,2,3 -oxadiazol-3 -ium-5 -yl)((5 -(trifluoromethyl) pyridin-3-yl)carbamoyl)amide;
- (*S*)-(3-(1-(4-(pyrimidin-5-yl)phenyl)ethyl)-1,2,3-oxadiazol-3-ium-5 -yl)((3 - (trifluoromethyl)phenyl)carbamoyl)amide;
- (*R*)-(3-(1-(4-(pyrimidin-5-yl)phenyl)ethyl)-1,2,3-oxadiazol-3 -ium-5-yl)((5-(trifluoromethyl)pyridin-3-yl)carbamoyl)amide;
- (*S*)-(3-(1-(4-bromophenyl)ethyl)-1,2,3-oxadiazol-3-ium-5 -yl)((5 -(trifluoromethyl) pyridin-3-yl)carbamoyl)amide;
- (*S*)-(3-(1-(4-(pyrimidin-5-yl)phenyl)ethyl)-1,2,3-oxadiazol-3 -ium-5-yl)((5-(trifluoromethyl)pyridin-3-yl)carbamoyl)amide;
- (3-(4-(3,5-dimethylisoxazol-4-yl)benzyl)-1,2,3-oxadiazol-3 -ium-5-yl)((3-(trifluoromethyl)phenyl)carbamoyl)amide;
- (*S*)-(3-(1-(4-(4-methylpyrimidin-5-yl)phenyl)ethyl)-1,2,3-oxadiazol-3-ium-5 -yl)((3 - (trifluoromethyl)phenyl)carbamoyl)amide;
- ((3-(trifluoromethyl)phenyl)carbamoyl)(3-(4-(4-(trifluoromethyl)pyrimidin-5-yl)benzyl)-1,2,3-oxadiazol-3-ium-5-yl)amide;
- (3-(4-(4,6-dimethylpyrimidin-5-yl)benzyl)-1,2,3-oxadiazol-3 -ium-5-yl)((3-(trifluoromethyl)phenyl)carbamoyl)amide;
- (R)-(3 -(1-(4-(4-methylpyrimidin-5-yl)phenyl)ethyl)-1,2,3-oxadiazol-3-ium-5 -yl)((3 - (trifluoromethyl)phenyl)carbamoyl)amide;
- (3-(3-(4-methylpyrimidin-5-yl)benzyl)-1,2,3-oxadiazol-3 -ium-5-yl)((3-(trifluoromethyl) phenyl)carbamoyl)amide;
- (3-(3-(4-methoxypyrimidin-5-yl)benzyl)-1,2,3-oxadiazol-3-ium-5-yl)((3-(trifluoromethyl)phenyl)carbamoyl)amide;
- (3-(4-(4-methylpyridin-3-yl)benzyl)-1,2,3-oxadiazol-3-ium-5-yl)((3-(trifluoromethyl) phenyl)carbamoyl)amide;
- (3-(4-(3-methylpyrazin-2-yl)benzyl)-1,2,3-oxadiazol-3-ium-5-yl)((3-(trifluoromethyl) phenyl)carbamoyl)amide;
- (3-(3-methyl-4-(pyrimidin-5-yl)benzyl)-1,2,3-oxadiazol-3-ium-5-yl)((3-(trifluoromethyl) phenyl)carbamoyl)amide;
- (3-(4-(3,5-dimethylisoxazol-4-yl)-3 -methylbenzyl)-1,2,3-oxadiazol-3 -ium-5-yl)((3-(trifluoromethyl)phenyl)carbamoyl)amide;
- (3-(4-(2-(2-hydroxyethoxy)-4-methylpyrimidin-5-yl)benzyl)-1,2,3-oxadiazol-3-ium-5-yl)((3-(trifluoromethyl)phenyl)carbamoyl)amide;
- (3-(4-(2-methoxy-4-methylpyrimidin-5-yl)benzyl)-1,2,3-oxadiazol-3 -ium-5-yl)((3-(trifluoromethyl)phenyl)carbamoyl)amide;
- (3-(4-(4-cyclopropylpyrimidin-5-yl)benzyl)-1,2,3-oxadiazol-3 -ium-5-yl)((3-(trifluoromethyl)phenyl)carbamoyl)amide;
- (3-(4-(2-methylpyridin-3-yl)benzyl)-1,2,3-oxadiazol-3-ium-5-yl)((3-(trifluoromethyl) phenyl)carbamoyl)amide;
- (3-(4-(5-methylpyridazin-4-yl)benzyl)-1,2,3-oxadiazol-3 -ium-5-yl)((3-(trifluoromethyl) phenyl)carbamoyl)amide;
- (3-(3-(3,5-dimethylisoxazol-4-yl)benzyl)-1,2,3-oxadiazol-3 -ium-5-yl)((3-(trifluoromethyl)phenyl)carbamoyl)amide;
- (3-(4-(1,5-dimethyl-1*H*-pyrazol-4-yl)benzyl)-1,2,3-oxadiazol-3-ium-5-yl)((3-(trifluoromethyl)phenyl)carbamoyl)amide;
- (3-(4-(1,3-dimethyl-1*H*-pyrazol-4-yl)benzyl)-1,2,3-oxadiazol-3-ium-5-yl)((3-(trifluoromethyl)phenyl)carbamoyl)amide;
- ((3 -(trifluoromethyl)phenyl)carbamoyl)(3 -(4-(1,3 ,5 -trimethyl-1*H*-pyrazol -4-yl)benzyl)-1,2,3-oxadiazol-3-ium-5-yl)amide;
- (3-(4-(1,4-dimethyl-1*H*-imidazol-5-yl)benzyl)-1,2,3-oxadiazol-3-ium-5-yl)((3-(trifluoromethyl)phenyl)carbamoyl)amide;
- (*R*)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide;
- (3-((5-(pyrimidin-5-yl)thiophen-3-yl)methyl)-1,2,3-oxadiazol-3-ium-5-yl)((3-(trifluoromethyl)phenyl)carbamoyl)amide;
- (3-(3-methyl-4-(4-methylpyrimidin-5-yl)benzyl)-1,2,3-oxadiazol-3-ium-5-yl)((3-(trifluoromethyl)phenyl)carbamoyl)amide;
- (R)-(3 -(2-amino-1-(4-(pyrimidin-5-yl)phenyl)ethyl)-1,2,3 -oxadiazol-3 -ium-5 -yl)((3 - (trifluoromethyl)phenyl)carbamoyl)amide;
- (3-((6-(pyrimidin-5-yl)pyridin-3-yl)methyl)-1,2,3-oxadiazol-3 -ium-5-yl)((3-(trifluoromethyl)phenyl)carbamoyl)amide;
- (3-((6-(3,5-dimethylisoxazol-4-yl)pyridin-3-yl)methyl)-1,2,3-oxadiazol-3-ium-5-yl)((3-(trifluoromethyl)phenyl)carbamoyl)amide;
- (3-(4-(1,2-dimethyl-1*H*-imidazol-5-yl)benzyl)-1,2,3-oxadiazol-3-ium-5-yl)((3-(trifluoromethyl)phenyl)carbamoyl)amide;
- (3-(4-(4-cyanopyrimidin-5-yl)benzyl)-1,2,3-oxadiazol-3-ium-5-yl)((3-(trifluoromethyl)phenyl)carbamoyl)amide;
- (3 -(4-(4-fluoro-6-oxo-1,6-dihydropyrimidin-5-yl)benzyl)-1,2,3-oxadiazol-3-ium-5 - yl)((3-(trifluoromethyl)phenyl)carbamoyl)amide;
- (3-((5-(pyrimidin-5-yl)pyridin-2-yl)methyl)-1,2,3-oxadiazol-3-ium-5-yl)((3-(trifluoromethyl)phenyl)carbamoyl)amide;
- (3-((5-(3,5-dimethylisoxazol-4-yl)pyridin-2-yl)methyl)-1,2,3-oxadiazol-3-ium-5-yl)((3-(trifluoromethyl)phenyl)carbamoyl)amide;
- (3-((4-(4-methylpyrimidin-5-yl)thiophen-2-yl)methyl)-1,2,3-oxadiazol-3-ium-5-yl)((3-(trifluoromethyl)phenyl)carbamoyl)amide;
- (3-(4-(4-methoxy-6-methylpyrimidin-5-yl)benzyl)-1,2,3-oxadiazol-3-ium-5-yl)((3-(trifluoromethyl)phenyl)carbamoyl)amide;
- (3-(4-(4-(2-hydroxyethoxy)-6-methylpyrimidin-5-yl)benzyl)-1,2,3-oxadiazol-3-ium-5-yl)((3-(trifluoromethyl)phenyl)carbamoyl)amide;
- ((3-chlorophenyl)carbamoyl)(3-(4-(2-methoxy-4-methylpyrimidin-5-yl)benzyl)-1,2,3-oxadiazol-3-ium-5-yl)amide;
- ((3-cyanophenyl)carbamoyl)(3-(4-(2-methoxy-4-methylpyrimidin-5-yl)benzyl)-1,2,3-oxadiazol-3-ium-5-yl);
- (3-(4-(2-methoxy-4-methylpyrimidin-5-yl)benzyl)-1,2,3-oxadiazol-3-ium-5-yl)(phenylcarbamoyl)amide amide;
- (*S*)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide;
- (3-((6-(1-(piperidin-4-yl)-1*H*-pyrazol-4-yl)pyridin-3-yl)methyl)-1,2,3-oxadiazol-3-ium-5-yl)((3-(trifluoromethyl)phenyl)carbamoyl)amide;
- (3-((5-(1,4-dimethyl-1*H*-imidazol-5-yl)pyridin-2-yl)methyl)-1,2,3-oxadiazol-3-ium-5-yl)((3-(trifluoromethyl)phenyl)carbamoyl)amide;
- (3-((5-(isoindolin-4-yl)pyridin-2-yl)methyl)-1,2,3-oxadiazol-3-ium-5-yl)((3-(trifluoromethyl)phenyl)carbamoyl)amide;
- (3-((5-(1-(piperidin-4-yl)-1*H*-pyrazol-4-yl)pyridin-2-yl)methyl)-1,2,3-oxadiazol-3-ium-5-yl)((3-(trifluoromethyl)phenyl)carbamoyl)amide;
- (3-((5-(2-(aminomethyl)phenyl)pyridin-2-yl)methyl)-1,2,3-oxadiazol-3-ium-5-yl)((3-(trifluoromethyl)phenyl)carbamoyl)amide;
- (3-((5-boronopyridin-2-yl)methyl)-1,2,3-oxadiazol-3-ium-5-yl)((3-(trifluoromethyl)phenyl)carbamoyl)amide;
- (3-((5-(4-(2-(dimethylamino)ethoxy)-6-methylpyrimidin-5-yl)pyridin-2-yl)methyl)-1,2,3-oxadiazol-3-ium-5-yl)((3-(trifluoromethyl)phenyl)carbamoyl)amide;
- (3-((5-(4,5,6,7-tetrahydropyrazolo[1,5-a]pyrazin-3-yl)pyridin-2-yl)methyl)-1,2,3-oxadiazol-3-ium-5-yl)((3-(trifluoromethyl)phenyl)carbamoyl)amide;
- (3-((5-(2-(2-(dimethylamino)ethoxy)-4-methylpyrimidin-5-yl)pyridin-2-yl)methyl)-1,2,3-oxadiazol-3-ium-5-yl)((3-(trifluoromethyl)phenyl)carbamoyl)amide.

Preferably the compound according is an inhibitor of NS2B-NS3 protease of a Flavivirus, preferably an inhibitor of the NS2B-NS3 protease of Zika and/or Dengue and/or West Nile and/or Japan Encephalitis virus, thus inhibiting replication of the above viruses in cells.

It is a further object if the invention a compound as defined above for medical use, preferably for use 1) in treatment and/or prevention of a Flavivirus infection, preferably wherein the Flavivirus is selected from the group consisting of Dengue, West Nile, Zika and Japan Encephalitis virus.

It is a further object of the invention a compound of general formula (I): 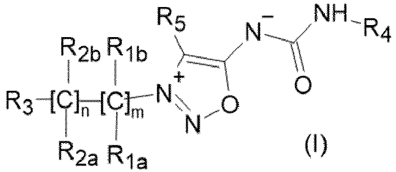 Wherein m and n are each independently selected from 0, 1 and 2;
R₁ₐ and R_{1b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₁ₐ and R_{1b} are linked together to form a C₃₋₆-cycloalkyl ring or a C₄₋₆-heterocycloalkyl ring;
or R₁ₐ is linked to R₃ to form a partially unsaturated aromatic or heteroaromatic bicycle;
or R_{1b} doesn't exist and C-R₁ₐ is C=CH₂;
R₂ₐ and R_{2b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine; or R₂ₐ and R_{2b} are linked together to form a C₃₋₆-cycloalkyl ring or a C₄₋₆- heterocycloalkyl ring; or any two of R₁ₐ and R₂ₐ or of R_{1b} and R_{2b} are linked together to form a C₃₋₆cycloalkyl or a C₃₋₆heterocycloalkyl;
R₃ is heterocycloalkyl, aromatic or heteroaromatic ring, each of said heterocycloalkyl, aromatic or heteroaromatic ring being optionally substituted with one or more substituents independently selected from halogen, OH, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, haloC₁₋₆alkyl, C₁₋₆alkoxy, cyano, B(OH)₂, aryl, heteroaryl wherein said aryl or heteroaryl ring are optionally substituted with one or more substituents each independently selected from halogen, hydroxy, cyano, NH₂, NHCOCH₃, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, N-methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and N-methylpyperazine;
R₄ is an aromatic or heteroaromatic ring optionally substituted with one or more substituents each independently selected from halogen, OH, C₁₋₆alkyl, haloC₁₋₆alkyl, C₁₋₆alkoxy, cyano, NH₂, NHC(O)C₁₋₆alkyl, NHC₁₋₃alkyl, N(C₁₋₃alkyl)₂, aziridine, azetidine, pyrrolidine, pyperidine, morpholine, piperazine and N-methylpyperazine;
R₅ is H, C₁₋₆alkyl, C₁₋₆haloalkyl or halogen;
or a pharmaceutically acceptable salt, tautomer, solvate, or stereoisomer thereof for use in the treatment of Flavivirus infection, preferably wherein the Flavivirus is selected from the group consisting of Dengue, West Nile, Zika and Japan Encephalitis virus.

Preferably:
- R_{1b} and R_{2b} are H; m and n are each independently selected from 0, 1; more preferably m is 1 and n is 1; still preferably m is 1 and n is 0; and/or
- R₃ is a phenyl, naphtyl, biphenyl, phenyl-heteroaryl or bis-heteroaryl wherein each of said phenyl, naphtyl or heteroaryl ring is optionally substituted with one or more substituents each independently selected from halogen, hydroxy, cyano, B(OH)₂, NH₂, NHCOCH₃, NHCOOC₁₋₆alkyl, NHC₁₋₃alkyl, N(C₁₋₃alkyl)₂, C₁₋₆alkyl, C₁₋₆alkoxy, pyrrolidine, pyperidine, morpholine, cyclopropyl, cyclobutyl, cyclopentyl, cyclohexyl, wherein said C₁₋₆alkyl, C₁₋₆alkoxy are optionally substituted with one or more halogen, hydroxy, NH₂, N(CH₃)₂, morpholine or pyrrolidine; and/or
- R₄ is selected from 3-trifluoromethylphenyl, naphthyl, 5-(trifluoromethyl)thiazol-2-yl, 3-chlorophenyl, 3-fluoro-5-trifluoromethylphenyl, quinolinyl, 5-(trifluoromethyl)pyridin-3-yl, 4-(trifluoromethyl)pyridin-2-yl, 3-methyl-5-trifluoromethylphenyl, 3-methoxy-5-trifluoromethylphenyl, 3-cyanophenyl.

Still preferably the compound for use in the treatment of flavivirus infection, preferably wherein the flavivirus is selected from the group consisting of Dengue, West Nile, Zika and Japan Encephalitis virus, is selected from:
- (3 -(1-phenylpropan-2-yl)-1,2,3 -oxadiazol -3 -ium-5 -yl)((3 -(trifluoromethyl) phenyl)carbamoyl)amide;
- (3-phenethyl-1,2,3-oxadiazol-3-ium-5-yl)((3-(trifluoromethyl)phenyl) carbamoyl)amide.

It is a further object of the invention a pharmaceutical composition comprising the compound as defined above and at least one pharmaceutically acceptable excipient.

Preferably the pharmaceutical composition is for use in the treatment and/or prevention of a flavivirus infection, preferably wherein the flavivirus is selected from the group consisting of Dengue, West Nile, Zika and Japan Encephalitis virus.

In a preferred embodiment, said pharmaceutical composition comprises at least one further active compound selected from the group consisting of: antivirals, antibacterials, anti-inflammatory agents, anti-pain agents and antipyretic agents.

It is a further object of the invention a method for the synthesis of the compound of general formula (I) as defined above.

The present invention includes within its scope prodrugs of the compound of formula (I). In general, such prodrugs will be functional derivatives of the compound of formula (I) which are readily convertible *in vivo* into the required compound of formula (I). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compound of formula (I) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and all tautomeric forms are intended to be encompassed by the scope of the invention, even though only one tautomeric structure is depicted. Specific tautomeric forms of compounds of the invention are depicted below:

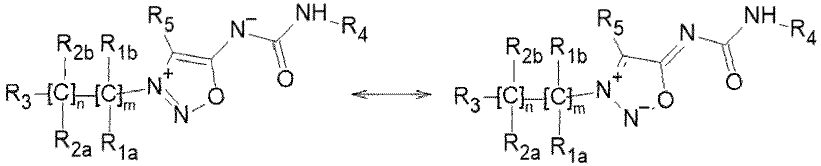

The compounds may exist in different isomeric forms, all of which are encompassed by the present invention.

When any variable occurs more than one time in any constituent, its definition on each occurrence is independent of every other occurrence. Also, combinations of substituents and variables are permissible only if such combinations result in stable compounds.

It is understood that substituents and substitution patterns on the compounds of the instant invention can be selected by one of ordinary skill in the art to provide compounds that are chemically stable and that can be readily synthesized from readily available starting materials by techniques known in the art, as well as those methods set forth below. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, "alkyl" is intended to include both branched and straight-chain saturated aliphatic hydrocarbon groups having the specified number of carbon atoms. For example,"C₁-C₆ alkyl" is defined to include groups having 1, 2, 3, 4, 5 or 6 carbons in a linear or branched arrangement. For example, "C₁-C₆ alkyl" specifically includes methyl, ethyl, *n*-propyl, *i*-propyl, *n*-butyl, *t*-butyl, *i*-butyl, pentyl, hexyl, and so on. The term "cycloalkyl" means a monocyclic, bicyclic or polycyclic saturated aliphatic hydrocarbon group having the specified number of carbon atoms. For example, "C₃₋₁₀cycloalkyl" includes cyclopropyl, methyl-cyclopropyl, 2,2-dimethyl-cyclobutyl, 2-ethyl-cyclopentyl, cyclohexyl, and so on. In an embodiment of the invention the term "cycloalkyl" includes the groups described immediately above and further includes monocyclic unsaturated aliphatic hydrocarbon groups. For example, "cycloalkyl" as defined in this embodiment includes cyclopropyl, methyl-cyclopropyl, 2,2-dimethyl-cyclobutyl, 2-ethyl-cyclopentyl, cyclohexyl, cyclopentenyl, cyclobutenyl, 7,7-dimethylbicyclo[2.2.1]heptyl and so on. Preferred cycloalkyl groups are cyclopropyl, cyclobutyl, cyclopentyl and cyclohexyl.

"Alkoxy" represents an alkyl group of indicated number of carbon atoms attached through an oxygen bridge. "Alkoxy" therefore encompasses the definitions of alkyl above. Examples of suitable alkoxy groups include methoxy, ethoxy, *n*-propoxy, *i*-propoxy, *n*-butoxy, s-butoxy and *t-*butoxy. The preferred alkoxy group is methoxy.

The terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. Preferred are fluoroC₁₋₆alkyl and fluoroC₁₋₆alkoxy groups, in particular fluoroC₁₋₃alkyl and fluoroC₁₋₃alkoxy groups, for example, CF₃, CHF₂, CH₂F, CH₂CH₂F, CH₂CHF₂, CH₂CF₃, OCF₃, OCHF₂, OCH₂F, OCH₂CH₂F, OCH₂CHF₂ or OCH₂CF₃, and most especially CF₃, OCF₃ and OCHF₂.

The term "hydroxyC₁₋₆alkyl" means a C₁₋₆alkyl group in which one or more (in particular, 1 to 3) hydrogen atoms have been replaced by hydroxy groups. Preferred are CH₂OH, CH₂CHOH and CHOHCH₃.

As used herein, "aryl" is intended to mean any stable monocyclic or bicyclic carbon ring of 6 to 10 atoms, wherein at least one ring is aromatic. Examples of such aryl elements include phenyl, naphthyl, tetrahydronaphthyl, indanyl and tetrahydrobenzo[7]annulene. The preferred aryl group is phenyl or naphthyl, especially phenyl.

As used herein, "heteroaryl" is intended to mean any stable monocyclic or bicyclic ring containing 1, 2, 3 or 4 heteroatoms independently selected from N, O and S, wherein at least one ring is aromatic. In particular the term heteroaryl includes 5 membered aromatic heterocycles containing 1, 2, 3 or 4 heteroatoms independently selected from N, O and S , but not more than one of which is O or S; 6 membered aromatic heterocycles containing 1, 2 or 3 nitrogen atoms; or a 7-13 membered aromatic heterocycle containing heteroatoms independently selected from N, O or S of 5 to 10 atoms, wherein at least one ring is aromatic. Heteroaryl ring also includes partially saturated bicyclic systems, such as indoline, isoindoline, 4,5,6,7-tetrahydropyrazolo[1,5-a]pyrazine; further included are tautomeric structures, such as for example hydroxypyrimidine and hydroxypyrimidones.

Examples of particular heteroaryl of this invention are benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, chromenyl, chromanyl, isochromanyl, carbazolyl, carbolinyl, cinnolinyl, epoxidyl, furanyl, furazanyl, imidazolyl, imidazothiazolyl, indolinyl, indolyl, indolizinyl, isoindolinyl, indazolyl, isobenzofuranyl, isoindolyl, isoquinolyl, isothiazolyl, isoxazolyl, naphthpyridinyl, oxadiazolyl, oxazolyl, oxazolinyl, oxoquinazolinyl isoxazolinyl, oxetanyl, purinyl, pyranyl, pyrazinyl, pyrazolyl, pyridazinyl, pyridopyridinyl, pyridazinyl, pyridinyl, pyrimidinyl, triazinyl, tetrazinyl, pyrrolyl, quinazolinyl, quinolyl, quinoxalinyl, quinolizinyl, tetrazolyl, tetrazolopyridyl, thiadiazolyl, thiazolidinyl, thiazolyl, thienyl, triazolyl, imidazopyridinyl, phthalazinyl, naphthyridinyl, quinazolinyl, pteridinyl, pyrimidin-4(3H)-one and N-oxides thereof. Attachment of a heterocycles substituent can occur via a carbon atom or via a heteroatom.

Biphenyl indicates a phenyl ring substituted with a further phenyl ring; phenyl-heteroaryl indicates a phenyl ring substituted with an heteroaromatic ring; bis heteroaryl indicates and heteroaryl substituted with a further heteroaromatic ring.

The term "heterocyclic ring" refers to a non-aromatic ring radical, which consists of carbon atoms and at least one heteroatom of nitrogen, phosphorus, oxygen or sulfur. The heterocyclic ring may be a mono-, bi-, tri-, or tetracyclic ring system, which may include fused, bridged or spiro ring systems, and the nitrogen, phosphorus, carbon, oxygen, or sulfur atoms in the heterocyclic ring radical may be optionally oxidized to various oxidation states. In addition, the nitrogen atom may be optionally quaternized.

Examples of particular heterocyclic rings of the invention are tetrahydrofuranyl, tetrahydropyranyl, tetrahydrothiopyranyl, tetrahydroisoquinolinyl, azetidinyl, 1,4-dioxanyl, hexahydroazepinyl, piperazinyl, piperidyl, pyridin-2-onyl, pyrrolidinyl, imidazolinyl, pyrazolinyl, pyrrolinyl, morpholinyl, thiomorpholinyl, dihydrobenzoimidazolyl, dihydrobenzofuranyl, dihydrobenzothiophenyl, dihydrobenzoxazolyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydroisochromenyl, dihydroimidazolonyl, dihydrotriazolonyl, dihydrobenzodioxinyl, dihydrothiazolopyrimidinyl, methylenedioxybenzoyl, tetrahydrofuranyl, tetrahydrothienyl, tetrahydroquinolinyl, thiazolidinonyl, imidazolonyl, isoindolinonyl, octahydroquinolizinyl, octahydroisoindolyl, azabicycloheptanyl, chromenonyl, triazolopyrimidinyl, dihydrobenzoxazinyl, thiazolotriazolyl, azoniabicycloheptanyl, azoniabicyclooctanyl. Attachment of a heterocyclyl substituent can occur via a carbon atom or via a heteroatom.

A preferred 4 membered saturated heterocycle is azetidine.

A preferred 5 membered saturated heterocycle is tetrahydrofurane.

Preferred 6 membered saturated or partially saturated heterocycles are pyrrolidinyl, piperidyl, piperazinyl, morpholinyl, thiomorpholinyl and thiazolidinyl.

Preferred 5 membered heteroaryls are thienyl, thiazolyl, pyrazolyl, isoxazolyl, imidazolyl, thiadiazolyl, oxazolyl, triazolyl, tetrazolyl, furyl and oxadiazolyl.

Preferred 6 membered heteroaryls are pyridinyl and pyrymidinyl.

Preferred 8-10 membered heteroaryls are benzothienyl, indolyl, benzothiadiazolyl, benzoxadiazolyl, thiazolotriazolyl, dihydrobenzodioxinyl, dihydrothiazolopyrimidinyl, dihydrobenzoxazinyl, dihydrobenzofuranyl, benzothiazolyl, quinolinyl, isoquinolinyl, benzimidazolyl, benzofuranyl, dihydrobenzoxazolyl, dihydroindolyl, dihydroquinazolinyl, dihydrophthalazinyl, indazolyl, benzisoxazolyl, benzotriazolyl, dihydroisoindolyl, tetrahydronaphthyridinyl, triazolopyrimidinyl and tetrahydroquinolinyl.

As used herein, the term 'halogen' refers to fluorine, chlorine, bromine and iodine, of which fluorine, chlorine and bromine are preferred.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reaction of the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reaction of a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I) and 1, 2 or 3 equivalent of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, *N,N*¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the invention find use in a variety of applications for human and animal health. The compounds of the invention are flavivirus inhibitors and can be used in the treatment and/or prevention of flavivirus infections.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants. Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution may be then introduced into a water and glycerol mixture and processed to form a microemulstion. The injectable solutions or microemulsions may be introduced into a patient's blood stream by local bolus injection.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of the invention are employed. The compounds of the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol. When a compound according to this invention is administered into a human subject, the daily dosage regimen will normally be determined by the prescribing physician with the dosage generally varying according to the age, weight, sex and response of the individual patient, as well as the severity of the patient's symptoms.

The instant compounds are also useful in combination with known therapeutic agents for simultaneous, separate or sequential administration.

The term "administration" and variants thereof (e.g., "administering" a compound) in reference to a compound of the invention means introducing the compound or a prodrug of the compound into the system of the subject in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

When a compound of the present invention is administered into a human subject, the daily dosage regimen will normally be determined by the prescribing physician, with the dosage generally varying according to the age, weight, and response of the individual patient, as well as the severity of the patient's symptoms. In one exemplary application, oral dosages of the present invention will range between about 0.01 mg per kg of body weight per day (mg/kg/day) to about 100 mg/kg/day, preferably 0.01 to 10 mg/kg/day, and most preferably 0.1 to 5.0 mg/kg/day.

These and other aspects of the invention will be apparent from the teachings contained herein.

### Chemistry

### General

The compounds, or pharmaceutically acceptable salts thereof, compositions, and methods described herein are further illustrated by the following non-limiting examples.

As used herein, the following abbreviations have the following meanings. If an abbreviation is not defined, it has its generally accepted meaning.

CDI 1,1'-Carbonyldiimidazole; DBU: 1,8-Diazabicyclo[5.4.0]undec-7-ene; DCM: Dichloromethane; DIPEA: *N,N*-Diisopropylethylamine; DMAP: 4-Dimethylaminopyridine; DMF: Dimethylformamide; DMSO: Dimethylsulfoxide; DPPA:
[azido(phenoxy)phosphoryl]oxybenzene; ES⁺: Electrospray Positive Ionisation; EtOAc: Ethyl acetate; h: Hour; HPLC: High Performance Liquid Chromatography; LCMS: Liquid Chromatography Mass Spectrometry; MeCN: Acetonitrile; MeOH: Methanol; min: Minute;
MTBE: Methyl tert-butyl ether; NMP *N*-Methylpyrrolidone; NMR Nuclear Magnetic Resonance; RP: Reverse Phase; *t*_{R}: Retention time; rt: room temperature; TBAB: Tetra-n-butylammonium bromide; TEA: Triethylamine; TFA: Trifluoroacetic acid; THF: Tetrahydrofurane; UPLC: Ultra High Performance Liquid Chromatography.

### General experimental details

Solvents and reagents were obtained from commercial suppliers and were used without further purification. Silica gel chromatography purifications were performed on prepacked cartridges on a Biotage system. UPLC-MS analyses were performed on a Waters Acquity UPLC^{™}, equipped with a diode array and a ZQ mass spectrometer, using an X-Terra C18 column (5 µm, 4.6 x 50 mm) or a BEH C18 column (1.7 µm, 2.1 x 50 mm). Mobile phase comprised a linear gradient of binary mixtures of H₂O containing 0.1% formic acid (A), and MeCN containing 0.1% formic acid (B). The linear gradient used is: (A): 90% (0.1 min), 90%-0% (2.6 min), 0% (0.3 min), 0%-90% (0.1 min) with a 0.5 mL/min flow. ¹H, ¹⁹F, ¹³C NMR spectra were at 400, 377 and 101 MHz on a 400 MHz Bruker spectrometer. Chemical shift (δ) are reported in parts per million downfield to tetramethylsilane using CDCl₃ as a solvent unless otherwise noted. Coupling constants (*J*) are reported in Hertz (Hz). Multiplicities are reported as singlet (s), broad (br), doublet (d), doublet of doublets (dd), doublet of doublets of doublets (ddd), triplet (t), doublet of triplet (dt) or multiplet (m). Unless indicated, spectra were acquired at 300 K. Temperatures are expressed in degrees Celsius (°C) and are uncorrected. The reported yields are the actual isolated yields of purified material and are not optimized. The purity of Intermediates **1-10, 23** and **24** and Examples **1-30, 34, 38-43, 46-48, 50-53, 55-64, 67-69, 71-98, 100, 101, 103-115** and **117-124** was assessed in a Waters Acquity UPLC^{™}, equipped with a diode array and a ZQ mass spectrometer a BEH C18 column (1.7 µm, 2.1 x 50 mm). Mobile phase comprised a linear gradient of binary mixtures of H₂O containing 0.1% formic acid (A), and MeCN containing 0.1% formic acid (B). The linear gradient of B used is: 10% (0.1 min), 10-100% (1.5 min), 100% (0.3 min), 100-10% (0.1 min) with a 0.5 mL/min flow. Run time: 3 min. The purity of Intermediates **11-22, 25-30** and Examples **30-32, 35-37, 44, 45, 49, 54, 65, 66, 70, 99, 102** and **116** was assessed in a Waters Acquity UPLC^{™}, equipped with a diode array and a ZQ mass spectrometer a BEH C18 column (1.7 µm, 2.1 x 50 mm). Mobile phase comprised a linear gradient of binary mixtures of H₂O containing 0.05% TFA (A), and MeCN containing 0.05% TFA (B). The linear gradient of B used is: 5% (0.1 min), 5-100% (2.8 min), 100% (0.2 min), 100-5% (0.8 min) with a 0.5 mL/min flow. Run time: 3 min. Unless otherwise indicated, commercially available reagents and solvents (HPLC grade) were used without further purification. Where the synthesis of intermediates and starting materials is not described, these compounds are commercially available or can be made from commercially available compounds by standard methods or by extension of the Examples herein. During any of the synthetic sequences described herein it may be necessary and/or desirable to protect sensitive or reactive groups or any of the molecules concerned, this may be achieved by means of conventional protecting groups, such as those described in Protecting Groups in Organic Synthesis (3rd Edition, Greene, T.W. and Wuts, P.G.M.; Wiley Interscience, 1999) and Protecting Groups (Kocienski, P.J.; Thieme, 1994).

### Example 1: (3-(1-Phenylpropan-2-yl)-1,2,3-oxadiazol-3-ium-5-yl)((3-(trifluoromethyl) phenyl)carbamoyl)amide

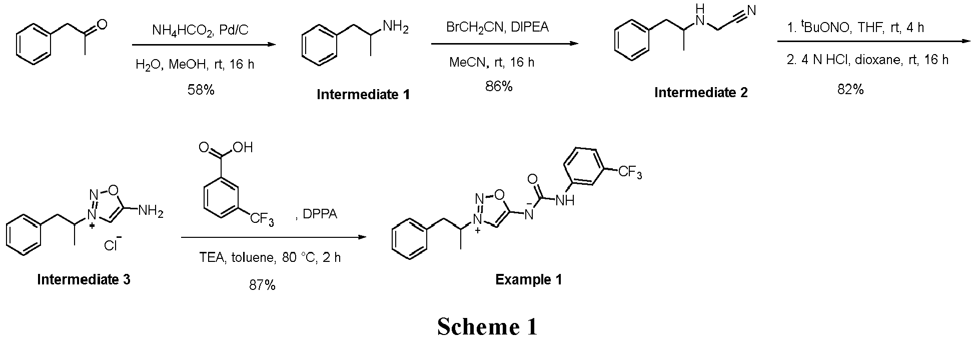

### Step 1: 1-Phenylpropan-2-amine (Intermediate 1).

Ammonium formate (24.44 g, 387.54 mmol) was dissolved in a mixture of water (9 mL) and methanol (80 mL) and the solution was treated with 1-phenylpropan-2-one (4.95 mL, 37.26 mmol) and Pd/C (10% w/w) (1.19 g, 11.18 mmol). The mixture was stirred at rt for 16 h then filtered through a pad of Celite and washed with methanol. The filtrate was evaporated under reduced pressure to get a white powder which was suspended in EtOAc (150 mL) and stirred at rt for 1 h. The solid formed was isolated by filtration to get the desired product as a salt (4.2 g). 500 mg of isolated product were dissolved in methanol (1.5 mL) and charged on a SCX cartridge (5 g). The resin was washed with methanol followed by a 2 M NH₃ solution in methanol. After removal of the solvents under reduced pressure the title compound was obtained as a white powder (350 mg, 58%). ¹H NMR (400 MHz, DMSO-*d*₆) δ 7.30-7.26 (m, 2H), 7.20-7.17 (m, 3H), 3.03-2.96 (m, 1H), 2.55-2.48 (m, 2H), 0.95 (d, *J=* 4.0 Hz, 3H). LCMS (ES⁺) *m*/*z* calculated for C₉H₁₃N 135.10, found 136 (M+H)⁺. HPLC *t*_{R} 0.59 min.

### Step 2: 2-((1-Phenylpropan-2-yl)amino)acetonitrile (Intermediate 2).

A suspension of 1-phenylpropan-2-amine (Intermediate **1**) (1.31 g, 9.71 mmol) and DIPEA (3.44 mL, 19.42 mmol) in MeCN (11.8 mL) was treated with 2-bromoacetonitrile (0.47 mL, 6.8 mmol) and the reaction mixture was stirred at rt for 16 h. Solvent was then removed under reduced pressure and the residue treated with water (10 mL) and extracted with EtOAc (3 x 50 mL). The collected organics were washed with brine, dried over Na₂SO₄, and evaporated under reduced pressure to get the title compound as a colourless oil (1.45 g, 86%). ¹H NMR (400 MHz, DMSO*d₆)* δ 7.32-7.28 (m, 2H), 7.22-7.18 (m, 3H), 3.73-3.61 (m, 2H), 2.95-2.90 (m, 1H), 2.77 (dd, *J₁ =* 4.0 Hz, *J₂* = 12.0 Hz, 1H), 2.46-2.41 (m, 2H), 0.92 (d, *J=* 4.0 Hz, 3H). LCMS (ES⁺) *m*/*z* calculated for C₁₁H₁₄N₂ 174.12, found 175 (M+H)⁺. HPLC *t*_{R} 0.81 min.

### Step 3: 5-Amino-3-(1-phenylpropan-2-yl)-1,2,3-oxadiazol-3-ium chloride (Intermediate 3).

A solution of 2-((1-phenylpropan-2-yl)amino)acetonitrile (Intermediate **2**) (1.4 g, 8.03 mmol) in THF (3.9 mL) was treated with tert-butyl nitrite (3.84 mL, 32.14 mmol). The solution was stirred at rt for 4 h. Then, 4 N HCl in dioxane (13.1 mL, 52.23 mmol) was added and the reaction mixture was stirred at rt for 18 h. Diethyl ether was added to the mixture and the precipitate formed was collected by filtration and washed with diethyl ether to afford the title compound as a white powder (1.39 g, 82%). ¹H NMR (400 MHz, DMSO-*d*₆) δ 9.71 (s, 2H), 8.20 (s, 1H), 7.34-7.21 (m, 5H), 5.33-5.27 (m, 1H), 3.32-3.30 (m, 2H), 1.65 (d, *J=* 4.0 Hz, 3H). LCMS (ES⁺) m/z calculated for C₁₁H₁₄N₃O⁺ 204.11, found 204 (M+H)⁺. HPLC t_{R} 0.84 min.

### Step 4: (3-(1-Phenylpropan-2-yl)-1,2,3-oxadiazol-3-ium-5-yl)((3-(trifluoromethyl)phenyl) carbamoyl)amide (Example 1).

A solution of 3-(trifluoromethyl)benzoic acid (277.6 mg, 1.46 mmol) in toluene (1.7 mL, 0.016 mol) was treated with TEA (0.22 mL, 1.61 mmol) and DPPA (0.35 mL, 1.61 mmol). The mixture was heated at 70 °C for 1 h then Intermediate **3** (175 mg, 0.730 mmol) was added and the mixture stirred at 80 °C for 2 h. Mixture was cooled to rt and quenched with addition of 1 N NaHCO₃ (aq. sol.) and extracted with EtOAc. The combined organic phase was washed with water and brine, dried over Na₂SO₄ and evaporated under reduced pressure. The residue obtained was triturated with petroleum ether to get the title compound as a light-yellow solid (258 mg, 87%). ¹H NMR (400 MHz, DMSO-*d*₆) δ 9.65 (s, 1H), 8.32 (s, 1H), 8.27 (br s, 1H), 7.68 (br d, *J=* 8.0 Hz, 1H), 7.43 (t, *J=* 8.0 Hz, 1H), 7.31-7.27 (m, 2H), 7.24-7.19 (m, 4H), 5.16-5.11 (m, 1H), 3.32 (m, 2H), 1.65 (d, *J=* 8.0 Hz, 3H). ¹⁹F NMR (377 MHz, DMSO-*d*₆) δ -61.28 (s, 3F). LCMS (ES⁺) *m*/*z* calculated for C₁₉H₁₇F₃N₄O₂ 390.13; found 391 (M+H)⁺. HPLC *t*_{R} 2.0 min.

### Examples 5, 12, 18 and33 were synthesized according to the above procedure (Scheme 1) by reacting with the appropriate starting materials.

### Example 5: (3-phenethyl-1,2,3-oxadiazol-3-ium-5-yl)((3-(trifluoromethyl)phenyl) carbamoyl)amide.

The title compound was obtained as a beige solid reacting 2-phenylethan-1-amine in Step 2. ¹H NMR (400 MHz, DMSO-*d*₆) δ 9.69 (s, 1H), 8.25 (br s, 1H), 8.24 (br s, 1H), 7.72 (br d, *J=* 8.3 Hz, 1H), 7.44 (t, *J=* 8.0 Hz, 1H), 7.35-7.22 (m, 6H), 4.82 (t, *J=* 7.1 Hz, 2H), 3.32 (t, *J=* 7.1 Hz, 2H). ¹⁹F NMR (377 MHz, DMSO-*d*₆) δ -61.28 (s, 3F). LCMS (ES⁺) *m*/*z* calculated for C₁₈H₁₅F₃N₄O₂ 376.11; found 377 (M+H)⁺. HPLC *t*_{R} 1.90 min.

### Example 12: (3-(1-phenylpropan-2-yl)-1,2,3-oxadiazol-3-ium-5-yl)(quinolin-6-ylcarbamoyl) amide.

The title compound was obtained as a beige solid reacting quinoline-6-carboxylic acid in Step 4. ¹H NMR (400 MHz, DMSO-*d*₆) δ 9.88 (s, 1H), 8.87 (d, *J=* 3.5 Hz, 1H), 8.53-8.51 (br m, 2H), 8.31 (s, 1H), 7.99 (br s, 2H), 7.68-7.65 (m, 1H), 7.33-7.22 (m, 5H), 5.21-5.16 (m, 1H), 3.34-3.32 (m, 2H), 1.68 (d, *J=* 6.6 Hz, 3H). LCMS (ES⁺) *m*/*z* calculated for C₂₁H₁₉N₅O₂ 373.15; found 374 (M+H)⁺. HPLC *t*_{R} 1.24 min.

### Example 18: (3-(naphthalen-2-ylmethyl)-1,2,3-oxadiazol-3-ium-5-yl)((3-(trifluoromethyl) phenyl)carbamoyl)amide.

The title compound was obtained as a white powder reacting 5-amino-3-(naphthalen-2-ylmethyl)-1,2,3-oxadiazol-3-ium chloride in Step 4. ¹H NMR (400 MHz, DMSO-*d*₆) δ 9.63 (br s, 1H), 8.17 (s, 1H), 8.10 (d, *J=* 15.5 Hz, 2H), 7.96-7.90 (m, 3H), 7.64 (br d, *J=* 8.3 Hz, 1H), 7.58 (dd, *J₁* = 8.6 Hz, *J₂ =* 1.8 Hz, 1H), 7.53-7.50 (m, 2H), 7.37-7.33 (m, 1H), 7.14 (d, *J=* 7.7 Hz, 1H), 5.89 (s, 2H). ¹⁹F NMR (377 MHz, DMSO-*d*₆) δ -61.28 (s, 3F). LCMS (ES⁺) *m*/*z* calculated for C₂₁H₁₅F₃N₄O₂ 412.11; found 411 (M-H)⁻. HPLC *t*_{R} 2.06 min.

### Example 33: ((3-methyl-5-(trifluoromethyl)phenyl)carbamoyl)(3-(1-phenylpropan-2-yl)-1,2,3-oxadiazol-3-ium-5-yl)amide.

The title compound was obtained as a white powder reacting 3-methyl-5-(trifluoromethyl)benzoic acid in Step 4. ¹H NMR (400 MHz, DMSO-*d*₆) δ 9.59 (s, 1H), 8.33 (s, 1H), 8.04 (br s, 1H), 7.56 (br s, 1H), 7.32-7.20 (m, 5H), 7.05 (s, 1H), 5.16-5.11 (m, 1H), 3.35-3.29 (m, 2H), 2.32 (s, 3H), 1.66 (d, *J=* 6.8 Hz, 3H). ¹⁹F NMR (377 MHz, DMSO-*d*₆) δ -61.23 (s, 3F). LCMS (ES⁺) *m*/*z* calculated for C₂₀H₁₉F₃N₄O₂ 404.15; found 405 (M+H)⁺. HPLC *t*_{R} 2.09 min.

### (R)-(3-(1-phenylpropan-2-yl)-1,2,3-oxadiazol-3-ium-5-yl)((3-(trifluoromethyl)phenyl)carbamoyl)amide (Example 13) & (S)-(3-(1-phenylpropan-2-yl)-1,2,3-oxadiazol-3-ium-5-yl)((3-(trifluoromethyl)phenyl)carbamoyl)amide (Example 14)

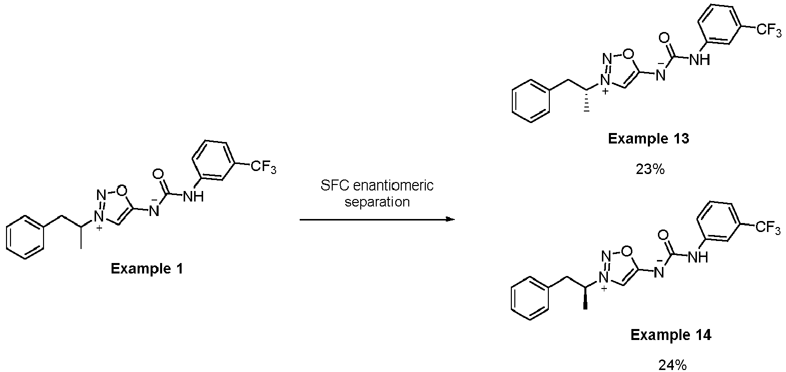

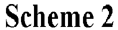

Step 1: (*R*)-(3-(1-Phenylpropan-2-yl)-1,2,3-oxadiazol-3-ium-5-yl)((3-(trifluoromethyl)phenyl)carbamoyl)amide (**Example 13**) & (*S*)-(3-(1-phenylpropan-2-yl)-1,2,3-oxadiazol-3-ium-5-yl)((3-(trifluoromethyl)phenyl)carbamoyl)amide (**Example 14**). (3-(1-phenylpropan-2-yl)-1,2,3-oxadiazol-3-ium-5-yl)((3-(trifluoromethyl)phenyl)carbamoyl) amide (**Example 1**) (45 mg, 0.115 mmol) was subjected to SFC purification (Column: Chiralpak IA (cellulose) - Run time 19 min - Eluent: MeOH - Method: 15% MeOH (2 min), 15-20% MeOH (8 min), 20% MeOH (7 min), 20-15% MeOH (1 min), 15% MeOH (1 min) to get (*R*)-(3-(1-phenylpropan-2-yl)-1,2,3-oxadiazol-3-ium-5-yl)((3-(trifluoromethyl)phenyl)carbamoyl)amide

(**Example 13**) (1^{st} eluted compound) as a white powder (10.2 mg, 23%). ¹³C NMR (101 MHz, DMSO-*d*₆) δ 172.6, 159.0, 141.9, 136.0, 129.4, 128.8, 128.5, 127.0, 125.7, 123.0, 121.3, 117.2, 113.6, 102.8, 62.8, 40.5, 19.3. LCMS (ES⁺) *m*/*z* calculated for C₁₉H₁₇F₃N₄O₂ 390.13; found 391 (M+H)⁺. HPLC *t*_{R} 2.0 min, and (*S*)-(3-(1-phenylpropan-2-yl)-1,2,3-oxadiazol-3-ium-5-yl)((3-(trifluoromethyl)phenyl)carbamoyl)amide (**Example 14**) (2^{nd} eluted compound) as a white powder (10.7 mg, 24%). LCMS (ES⁺) *m*/*z* calculated for C₁₉H₁₇F₃N₄O₂ 390.13; found 391 (M+H)⁺. HPLC *t*_{R} 2.0 min.

### Example 3: (Naphthalen-2-ylcarbamoyl)(3-(1-phenylpropan-2-yl)-1,2,3-oxadiazol-3-ium-5-yl)amide

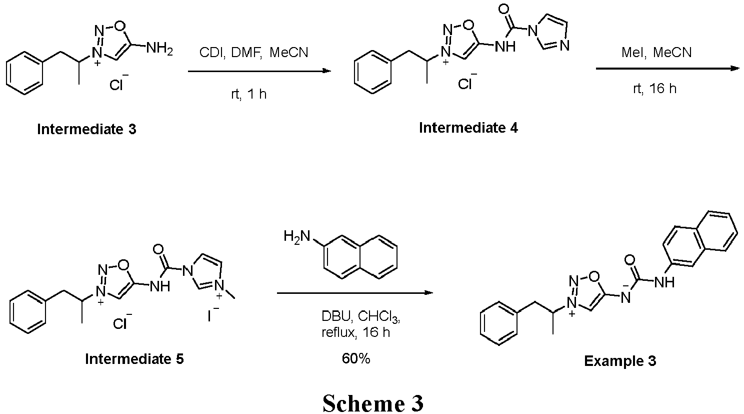

### Step 1: 5-(1H-Imidazole-1-carboxamido)-3-(1-phenylpropan-2-yl)-1,2,3-oxadiazol-3-ium chloride (Intermediate 4)

A solution of CDI (223 mg, 1.38 mmol) in a mixture of DMF/MeCN (3 mL/0.5 mL) was treated with Intermediate **3** (300 mg, 1.25 mmol). The solution was stirred at rt for 1 h. After concentration under reduced pressure the title compound was obtained as a crude, which was used directly in the next reaction LCMS (ES⁺) *m*/*z* calculated for C₁₅H₁₆N₅O₂ 298.13, found 320 (M+Na)⁺. HPLC *t*_{R} 1.06 min.

### Step 2: 5-(3-Methyl-1H-imidazol-3-ium-1-carboxamido)-3-(1-phenylpropan-2-yl)-1,2,3-oxadiazol-3-ium chloride iodide (Intermediate 5).

A solution of crude Intermediate **4** (417 mg, 1.25 mmol) in anhydrous MeCN (19 mL) was treated with iodomethane (0.33 mL, 5.37 mmol). The mixture was stirred at rt for 16 h. After concentration under reduced pressure the title compound was obtained as a yellow oil crude, which was used directly in the next reaction.

### Step 3: (Naphthalen-2-ylcarbamoyl)(3-(1-phenylpropan-2-yl)-1,2, 3-oxadiazol-3-ium-5-yl)amide (Example 3).

A solution of crude Intermediate **5** (60 mg, 0.13 mmol) in chloroform (1 mL) was treated with naphthalen-2-amine (18.1 mg, 0.13 mmol) in chloroform (1 mL) and DBU (0.04 mL, 0.25 mmol). The mixture was stirred at reflux overnight and the solvent was then evaporated under reduced pressure. The title compound was obtained after purification by automated RP-HPLC using water (+ 0.1% TFA) and MeCN (+ 0.1% TFA) as eluents (C18 column). Fractions containing product were lyophilized to give the title compound as a solid (28.4 mg, 60%). ¹H NMR (400 MHz, DMSO-*d*₆) δ 9.56 (br s, 1H), 8.35 (s, 1H), 8.34 (br s, 1H), 7.78-7.75 (m, 2H), 7.70 (d, *J=* 8.0 Hz, 1H), 7.60 (d, *J=* 8.0 Hz, 1H), 7.42 (t, *J=* 8.0 Hz, 1H), 7.34-7.28 (m, 3H), 7.25-7.21 (m, 3H), 5.19-5.14 (m, 1H), 3.33-3.31 (m, 2H), 1.67 (d, *J=* 8.0 Hz, 3H). LCMS (ES⁺) *m*/*z* calculated for C₂₂H₂₀N₄O₂ 372.16; found 373 (M+H)⁺. HPLC *t*_{R} 1.93 min.

**Example 4** was synthesized according to the above procedure (Scheme 3) by reacting with the appropriate starting material.

### Example 4: (3-(1-phenylpropan-2-yl)-1,2,3-oxadiazol-3-ium-5-yl)((5-(trifluoromethyl) thiazol-2-yl)carbamoyl)amide.

The title compound was obtained as a beige solid reacting Intermediate **5** and 5-(trifluoromethyl)thiazol-2-amine in Step 3. ¹H NMR (400 MHz, DMSO-*d*₆) δ 11.78 (br s, 1H), 8.46 (s, 1H), 7.92 (br d, *J*= 1.3 Hz, 1H), 7.32-7.20 (m, 5H), 5.25-5.19 (m, 1H), 3.36-3.30 (m, 2H), 1.67 (d, *J=* 6.8 Hz, 3H). ¹⁹F NMR (377 MHz, DMSO-*d*₆) δ -59.51 (s, 3F). LCMS (ES⁺) *m*/*z* calculated for C₁₆H₁₄F₃N₅O₂S 397.08; found 398 (M+H)⁺. HPLC *t*_{R} 1.91 min.

### Example 6: (3-Benzyl-1,2,3-oxadiazol-3-ium-5-yl)((3-(trifluoromethyl)phenyl)carbamoyl) amide

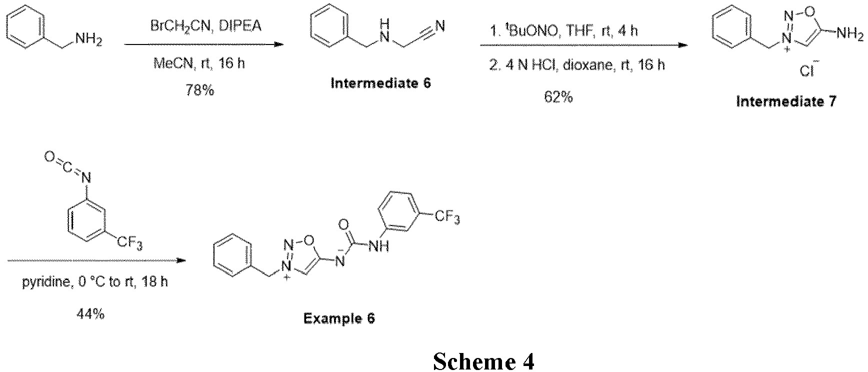

### Step 1: 2-(Benzylamino)acetonitrile (Intermediate 6).

The title compound was prepared using the procedure reported for the synthesis of Intermediate **2** starting from benzylamine (1.01 mL, 9.3 mmol). The crude product was purified by chromatography on silica gel (eluting with 100% DCM) to give the title compound as a colorless oil (1.06 g, 78%). ¹H NMR (400 MHz, DMSO-*d*₆) δ 7.36-7.32 (m, 4H), 7.28-7.23 (m, 1H), 3.76 (d, *J=* 8.0 Hz, 2H), 3.58 (d, *J=* 8.0 Hz, 2H), 3.04-2.98 (m, 1H). LCMS (ES⁺) *m*/*z* calculated for C₉H₁₀N₂ 146.08, found 147 (M+H)⁺. HPLC *t*_{R} 0.98 min.

### Step 2: 5-Amino-3-benzyl-1,2,3-oxadiazol-3-ium chloride (Intermediate 7).

The title compound was prepared using the procedure reported for the synthesis of Intermediate **3** starting from Intermediate **6** (877 mg, 6.0 mmol). After filtration the title compound was obtained as a solid (786 mg, 62%). ¹H NMR (400 MHz, DMSO-*d*₆) δ 9.84 (br s, 2H), 8.15 (br s, 1H), 7.57-7.45 (br m, 5H), 5.91 (br s, 2H).

### Step 3: (3-Benzyl-1,2,3-oxadiazol-3-ium-5-yl)((3-(trifluoromethyl)phenyl)carbamoyl)amide (Example 6).

A solution of 5-amino-3-benzyl-1,2,3-oxadiazol-3-ium chloride (Intermediate **7**) (30 mg, 0.170 mmol) in anhydrous pyridine (1.02 mL) was cooled at 0 °C and 1-isocyanato-3-(trifluoromethyl)benzene (0.05 mL, 0.34 mmol) was added. The reaction mixture was left warming to rt and stirred for 18 h. After evaporation of the solvent under reduced pressure, the crude product was purified by chromatography on silica gel (eluting with 10-50% EtOAc in petroleum ether) to give the title compound as a solid (27.1 mg, 44%). ¹H NMR (400 MHz, DMSO-*d*₆) δ 9.71 (s, 1H), 8.19 (br s, 2H), 7.71 (br d, *J=* 8.0 Hz, 1H), 7.58-7.56 (m, 2H), 7.48-7.41 (m, 4H), 7.21 (br d, *J=* 8.0 Hz, 1H), 5.78 (s, 2H). ¹⁹F NMR (377 MHz, DMSO-*d*₆) δ -61.28 (s, 3F). LCMS (ES⁺) *m*/*z* calculated for C₁₇H₁₃F₃N₄O₂ 362.10; found 363 (M+H)⁺. HPLC *t*_{R} 1.87 min.

**Examples 2, 7, 8, 9, 10, 11, 19, 25, 27, 28, 29, 38, 46, 47, 59, 68, 69, 113, 114 and 115** were synthesized according to the above procedure (Scheme 4) by reacting with the appropriate starting materials.

### Example 2: (3-(1-phenylpropan-2-yl)-1,2,3-oxadiazol-3-ium-5-yl)((4-(trifluoromethyl) phenyl)carbamoyl)amide.

The title compound was obtained as a beige solid reacting Intermediate 3 and 1-isocyanato-4-(trifluoromethyl)benzene in Step 3. ¹H NMR (400 MHz, DMSO-*d*₆) δ 9.73 (s, 1H), 8.29 (s, 1H), 7.82 (d, *J*= 8.8 Hz, 2H), 7.57 (d, *J=* 8.6 Hz, 2H), 7.32-7.21 (m, 5H), 5.20-5.14 (m, 1H), 3.32-3.29 (m, 2H), 1.66 (d, *J=* 6.8 Hz, 3H). ¹⁹F NMR (377 MHz, DMSO-*d*₆) δ -59.88 (s, 3F). LCMS (ES⁺) *m*/*z* calculated for C₁₉H₁₇F₃N₄O₂ 390.13; found 391 (M+H)⁺. HPLC *t*_{R} 2.01 min.

### Example 7: (3-phenyl-1,2,3-oxadiazol-3-ium-5-yl)((3-(trifluoromethyl)phenyl)carbamoyl) amide.

The title compound was obtained as a beige solid reacting 5-amino-3-phenyl-1,2,3-oxadiazol-3-ium chloride in Step 3. ¹H NMR (400 MHz, DMSO-*d*₆) δ 9.82 (s, 1H), 8.68 (s, 1H), 8.29 (s, 1H), 8.10 (br *d, J=* 8.1 Hz, 2H), 7.80-7.71 (m, 4H), 7.47 (t, *J=* 7.9 Hz, 1H), 7.25 (br d, *J=* 7.5 Hz, 1H). ¹⁹F NMR (377 MHz, DMSO-*d*₆) δ -61.25 (s, 3F). LCMS (ES⁺) *m*/*z* calculated for C₁₆H₁₁F₃N₄O₂ 348.08; found 349 (M+H)⁺. HPLC *t*_{R} 1.85 min.

### Example 8: ((3-chlorophenyl)carbamoyl)(3-(1-phenylpropan-2-yl)-1,2,3-oxadiazol-3-ium-5-yl)amide.

The title compound was obtained as a white solid reacting 1-chloro-3-isocyanatobenzene in Step 3. ¹H NMR (400 MHz, DMSO-*d*₆) δ 9.54 (s, 1H), 8.30 (s, 1H), 7.88 (t, *J*= 2.0 Hz, 1H), 7.45 (dd, *J₁* = 8.3 Hz, *J₂* = 1.1 Hz, 1H), 7.32-7.21 (m, 6H), 6.94 (dd, *J₁* = 7.9 Hz, *J₂* = 1.1 Hz, 1H), 5.20-5.12 (m, 1H), 3.33-3.28 (m, 2H), 1.66 (d, *J=* 6.6 Hz, 3H). LCMS (ES⁺) *m*/*z* calculated for C₁₈H₁₇ClN₄O₂ 356.10; found 357 (M+H)⁺. HPLC *t*_{R} 1.92 min.

### Example 9: (3-(1-(3,4-dichlorophenyl)propan-2-yl)-1,2,3-oxadiazol-3-ium-5-yl)((3-(trifluoromethyl)phenyl)carbamoyl)amide.

The title compound was obtained as a white solid reacting 5-amino-3-(1-(3,4-dichlorophenyl)propan-2-yl)-1,2,3-oxadiazol-3-ium chloride in Step 3. ¹H NMR (400 MHz, DMSO-*d*₆) δ 9.71 (s, 1H), 8.37 (s, 1H), 8.28 (br s, 1H), 7.70 (br d, *J=* 8.1 Hz, 1H), 7.60-7.57 (m, 2H), 7.45 (t, *J=* 8.0 Hz, 1H), 7.24-7.20 (m, 2H), 5.23-5.15 (m, 1H), 3.39-3.28 (m, 2H), 1.64 (d, *J* = 6.6 Hz, 3H). ¹⁹F NMR (377 MHz, DMSO-*d*₆) δ -61.29 (s, 3F). LCMS (ES⁺) *m*/*z* calculated for C₁₉H₁₅Cl₂F₃N₄O₂ 458.05; found 459 (M+H)⁺. HPLC *t*_{R} 2.22 min.

### Example 10: (3-(2,3-dihydro-1H-inden-2-yl)-1,2,3-oxadiazol-3-ium-5-yl)((3-(trifluoromethyl)phenyl)carbamoyl)amide.

The title compound was obtained as a beige solid reacting 5-amino-3-(2,3-dihydro-1H-inden-2-yl)-1,2,3-oxadiazol-3-ium chloride in Step 3. ¹H NMR (400 MHz, DMSO-*d*₆) δ 9.69 (s, 1H), 8.18 (s, 1H), 8.07 (s, 1H), 7.72 (br d, *J=* 8.1 Hz, 1H), 7.44 (t, *J=* 7.9 Hz, 1H), 7.35-7.32 (m, 2H), 7.28-7.21 (m, 3H), 5.71-5.65 (m, 1H), 3.67 (dd, *J₁* = 16.9 Hz, *J₂* = 7.7 Hz, 2H), 3.52 (dd, *J₁* = 16.9 Hz, *J₂* = 4.4 Hz, 2H). ¹⁹F NMR (377 MHz, DMSO-*d*₆) δ -61.26 (s, 3F). LCMS (ES⁺) *m*/*z* calculated for C₁₉H₁₅F₃N₄O₂ 388.11; found 389 (M+H)⁺. HPLC *t*_{R} 1.96 min.

### Example 11: ((3-fluoro-5-(trifluoromethyl)phenyl)carbamoyl)(3-(1-phenylpropan-2-yl)-1,2,3-oxadiazol-3-ium-5-yl)amide.

The title compound was obtained as a beige solid reacting Intermediate 3 and 1-fluoro-3-isocyanato-5-(trifluoromethyl)benzene in Step 3. ¹H NMR (400 MHz, DMSO-*d*₆) δ 9.87 (s, 1H), 8.36 (s, 1H), 7.96 (s, 1H), 7.71 (br d, *J*= 11.6 Hz, 1H), 7.32-7.20 (m, 5H), 7.12 (br d, *J*= 8.3 Hz, 1H), 5.19-5.14 (m, 1H), 3.33-3.29 (m, 2H), 1.66 (d, *J=* 6.8 Hz, 3H). LCMS (ES⁺) *m*/*z* calculated for C₁₉H₁₆F₄N₄O₂ 408.12; found 409 (M+H)⁺. HPLC *t*_{R} 2.11 min.

### Example 19: (3-(4-bromobenzyl)-1,2,3-oxadiazol-3-ium-5-yl)((3-(trifluoromethyl)phenyl) carbamoyl)amide.

The title compound was obtained as a yellow solid reacting 5-amino-3-(4-bromobenzyl)-1,2,3-oxadiazol-3-ium chloride in Step 3. ¹H NMR (400 MHz, DMSO-*d*₆) δ 9.71 (s, 1H), 8.23 (s, 1H), 8.21 (s, 1H), 7.72 (br d, *J=* 8.3 Hz, 1H), 7.68 (br d, *J=* 7.7 Hz, 2H), 7.54 (br d, *J=* 8.1 Hz, 2H), 7.44 (br t, *J=* 7.9 Hz, 1H), 7.22 (br d, *J=* 7.7 Hz, 1H), 5.78 (s, 2H). ¹⁹F NMR (377 MHz, DMSO-*d*₆) δ -61.29 (s, 3F). LCMS (ES⁺) *m*/*z* calculated for C₁₇H₁₂BrF₃N₄O₂ 440.01; found 441 (M+H)⁺. HPLC *t*_{R} 2.03 min.

### Example 25: (3-(1-phenylcyclopropyl)-1,2,3-oxadiazol-3-ium-5-yl)((3-(trifluoromethyl) phenyl)carbamoyl)amide.

The title compound was obtained as a yellow solid reacting 5-amino-3-(1-phenylcyclopropyl)-1,2,3-oxadiazol-3-ium chloride in Step 3. ¹H NMR (400 MHz, DMSO-*d*₆) δ 9.72 (s, 1H), 8.24 (br s, 2H), 7.69 (br d, *J=* 8.1 Hz, 1H), 7.54-7.52 (m, 2H), 7.48-7.41 (m, 4H), 7.22 (br d*, J=* 7.7 Hz, 1H), 2.02-1.99 (m, 2H), 1.72-1.69 (m, 2H). ¹⁹F NMR (377 MHz, DMSO-*d*₆) δ -61.27 (s, 3F). LCMS (ES⁺) *m*/*z* calculated for C₁₉H₁₅F₃N₄O₂ 388.11; found 389 (M+H)⁺. HPLC *t*_{R} 2.03 min.

### Example 27: (3-([1,1'-biphenyl]-3-ylmethyl)-1,2,3-oxadiazol-3-ium-5-yl)((3-(trifluoromethyl)phenyl)carbamoyl)amide.

The title compound was obtained as a white solid reacting 3-([1,1'-biphenyl]-3-ylmethyl)-5-amino-1,2,3-oxadiazol-3-ium chloride in Step 3. ¹H NMR (400 MHz, DMSO-*d*₆) δ 9.63 (s, 1H), 8.19 (s, 1H), 8.12 (br s, 1H), 7.86 (s, 1H), 7.69-7.62 (m, 4H), 7.49 (br d, *J* = 4.8 Hz, 2H), 7.43 (t, *J* = 7.6 Hz, 2H), 7.37-7.31 (m, 2H), 7.14 (br d, *J* = 7.7 Hz, 1H), 5.77 (s, 2H). ¹⁹F-NMR (377 MHz, DMSO-*d*₆) δ -61.28 (s, 3F). LCMS (ES⁺) *m*/*z* calculated for C₂₃H₁₇F₃N₄O₂ 438.13; found 439 (M+H)⁺. HPLC *t*_{R} 2.20 min.

### Example 28: (3-(2-phenylpropyl)-1,2,3-oxadiazol-3-ium-5-yl)((3-(trifluoromethyl)phenyl) carbamoyl)amide.

The title compound was obtained as a white solid reacting 5-amino-3-(2-phenylpropyl)-1,2,3-oxadiazol-3-ium chloride in Step 3. ¹H NMR (400 MHz, DMSO-*d*₆) δ 9.66 (s, 1H), 8.23 (s, 1H), 8.17 (s, 1H), 7.71 (br d, *J* = 8.3 Hz, 1H), 7.44 (t, *J* = 8.0 Hz, 1H), 7.34-7.31 (m, 4H), 7.26-7.21 (m, 2H), 4.76 (br d, *J* = 8.1 Hz, 2H), 3.58-3.53 (m, 1H), 1.30 (d, *J* = 7.0 Hz, 3H). ¹⁹F NMR (377 MHz, DMSO-*d*₆) δ -61.28 (s, 3F). LCMS (ES⁺) *m*/*z* calculated for C₁₉H₁₇F₃N₄O₂ 390.13; found 391 (M+H)⁺. HPLC *t*_{R} 2.01 min.

### Example 29: (3-(pyridin-3-ylmethyl)-1,2,3-oxadiazol-3-ium-5-yl)((3-(trifluoromethyl) phenyl)carbamoyl)amide.

The title compound was obtained as a white solid reacting 5-amino-3-(pyridin-3-ylmethyl)-1,2,3-oxadiazol-3-ium chloride in Step 3. ¹H NMR (400 MHz, DMSO-*d*₆) δ 9.71 (s, 1H), 8.80 (s, 1H), 8.64 (d, *J* = 4.8 Hz, 1H), 8.28 (s, 1H), 8.20 (s, 1H), 8.00 (br d, *J* = 7.9 Hz, 1H), 7.71 (br d, *J* = 8.3 Hz, 1H), 7.51-7.41 (m, 2H), 7.21 (br d, *J* = 7.7 Hz, 1H), 5.84 (s, 2H). ¹⁹F NMR (377 MHz, DMSO-*d*₆) δ -61.87 (s, 3F). LCMS (ES⁺) *m*/*z* calculated for C₁₆H₁₂F₃N₅O₂ 363.09; found 364 (M+H)⁺. HPLC *t*_{R} 1.42 min.

### Example 38: (3-(1-phenylethyl)-1,2,3-oxadiazol-3-ium-5-yl)((3-(trifluoromethyl)phenyl) carbamoyl)amide.

The title compound was obtained as a white solid reacting 5-amino-3-(1-phenylethyl)-1,2,3-oxadiazol-3-ium chloride in Step 3. ¹H NMR (400 MHz, DMSO-*d*₆) δ 9.69 (s, 1H), 8.23 (br s, 1H), 8.19 (s, 1H), 7.69 (br d, *J* = 8.3 Hz, 1H), 7.61 (br d, *J* = 6.2 Hz, 2H), 7.50-7.41 (m, 4H), 7.22 (br d, *J* = 7.5 Hz, 1H), 6.16 (q, *J* = 6.0 Hz, 1H), 2.01 (d, *J* = 7.2 Hz, 3H). ¹⁹F NMR (377 MHz, DMSO-*d*₆) δ -61.28 (s, 3F). LCMS (ES⁺) *m*/*z* calculated for C₁₈H₁₅F₃N₄O₂ 376.11; found 375 (M-H)⁻. HPLC *t*_{R} 1.95 min.

### Example 46: (3-(3-bromobenzyl)-1,2,3-oxadiazol-3-ium-5-yl)((3-(trifluoromethyl)phenyl) carbamoyl)amide.

The title compound was obtained as a light orange solid reacting 5-amino-3-(3-bromobenzyl)-1,2,3-oxadiazol-3-ium chloride in Step 3. ¹H NMR (400 MHz, DMSO-*d*₆) δ 9.74 (s, 1H), 8.27 (s, 1H), 8.21 (br s, 1H), 7.86 (br s, 1H), 7.72 (br d, *J* = 8.3 Hz, 1H), 7.67 (br d, *J* = 7.9 Hz, 1H), 7.59 (br d, *J* = 7.9 Hz, 1H), 7.43 (br t, *J* = 7.8 Hz, 2H), 7.22 (br d, *J* = 7.7 Hz, 1H), 5.78 (s, 2H). ¹⁹F NMR (377 MHz, DMSO-*d*₆) δ -61.28 (s, 3F). LCMS (ES⁺) *m*/*z* calculated for C₁₇H₁₂BrF₃N₄O₂ 440.01; found 441 (M+H)⁺. HPLC *t*_{R} 2.07 min.

### Example 47: (3-(4-iodobenzyl)-1,2,3-oxadiazol-3-ium-5-yl)((3-(trifluoromethyl)phenyl) carbamoyl)amide.

The title compound was obtained as a light-yellow solid reacting 5-amino-3-(4-iodobenzyl)-1,2,3-oxadiazol-3-ium chloride in Step 3. ¹H NMR (400 MHz, DMSO-*d*₆) δ 9.73 (s, 1H), 8.22 (s, 1H), 8.21 (br s, 1H), 7.84 (br d, *J* = 8.6 Hz, 2H), 7.72 (br d, *J* = 8.8 Hz, 1H), 7.44 (t, *J* = 8.0 Hz, 1H), 7.38 (d, *J* = 8.3 Hz, 2H), 7.22 (br d, *J* = 7.7 Hz, 1H), 5.75 (s, 2H). ¹⁹F NMR (377 MHz, DMSO-*d*₆) δ -61.28 (s, 3F). LCMS (ES⁺) *m*/*z* calculated for C₁₇H₁₂F₃IN₄O₂ 488.00; found 489 (M+H)⁺. HPLC *t*_{R} 2.12 min.

### Example 59: (3-((1R,2S)-2-phenylcyclopentyl)-1,2,3-oxadiazol-3-ium-5-yl)((3-(trifluoromethyl)phenyl)carbamoyl)amide.

The title compound was obtained as a white solid reacting 5-amino-3-((1*R*,2*S*)-2-phenylcyclopentyl)-1,2,3-oxadiazol-3-ium chloride in Step 3. ¹H NMR (400 MHz, DMSO-*d*₆) δ 9.70 (s, 1H), 8.26 (s, 1H), 8.25 (br s, 1H), 7.69 (br d, *J* = 8.1 Hz, 1H), 7.43 (t, *J* = 8.0 Hz, 1H), 7.35-7.30 (m, 4H), 7.27-7.21 (m, 2H), 5.29-5.22 (m, 1H), 3.76-3.69 (m, 1H), 2.56-2.50 (m, 1H), 2.37-2.22 (m, 2H), 2.05-1.86 (m, 3H). ¹⁹F NMR (377 MHz, DMSO-*d*₆) δ -61.27 (s, 3F). LCMS (ES⁺) *m*/*z* calculated for C₂₁H₁₉F₃N₄O₂ 416.15; found 417 (M+H)⁺. HPLC *t*_{R} 2.15 min.

### Example 68: (R)-(3-(1-(4-bromophenyl)ethyl)-1,2,3-oxadiazol-3-ium-5-yl)((3-(trifluoromethyl)phenyl)carbamoyl)amide.

The title compound was obtained as a white solid reacting (*R*)-5-amino-3-(1-(4-bromophenyl)ethyl)-1,2,3-oxadiazol-3-ium chloride in Step 3. ¹H NMR (400 MHz, DMSO-*d*₆) δ 9.72 (s, 1H), 8.24 (s, 2H), 7.70-7.67 (m, 3H), 7.57 (d, *J* = 8.6 Hz, 2H), 7.43 (t, *J* = 8.0 Hz, 1H), 7.22 (d, *J* = 7.7 Hz, 1H), 6.16 (q, *J* = 7.0 Hz, 1H), 2.00-1.99 (m, 3H). ¹⁹F NMR (377 MHz, DMSO-*d*₆) δ -61.27 (s, 3F). LCMS (ES⁺) *m*/*z* calculated for C₁₈H₁₄BrF₃N₄O₂ 454.03; found 453 (M-H)⁻. HPLC *t*_{R} 2.13 min.

### Example 69: (S)-(3-(1-(4-bromophenyl)ethyl)-1,2,3-oxadiazol-3-ium-5-yl)((3-(trifluoromethyl)phenyl)carbamoyl)amide.

The title compound was obtained as a white solid reacting (*S*)-5-amino-3-(1-(4-bromophenyl)ethyl)-1,2,3-oxadiazol-3-ium chloride in Step 3. ¹H NMR (400 MHz, DMSO-*d*₆) δ 9.72 (s, 1H), 8.24 (s, 2H), 7.70-7.67 (m, 3H), 7.57 (d, *J* = 8.6 Hz, 2H), 7.43 (t, *J* = 7.9 Hz, 1H), 7.22 (d, *J* = 7.7 Hz, 1H), 6.16 (q, *J* = 7.0 Hz, 1H), 2.00-1.99 (m, 3H). ¹⁹F NMR (377 MHz, DMSO-*d*₆) δ -61.27 (s, 3F). LCMS (ES⁺) *m*/*z* calculated for C₁₈H₁₄BrF₃N₄O₂ 454.03; found 453 (M-H)⁻. HPLC *t*_{R} 2.13 min.

### Example 113: ((3-chlorophenyl)carbamoyl)(3-(4-(2-methoxy-4-methylpyrimidin-5-yl)benzyl)-1,2,3-oxadiazol-3-ium-5-yl)amide.

The title compound was obtained as a white solid reacting 1-chloro-3-isocyanatobenzene and 5-amino-3-(4-(2-methoxy-4-methylpyrimidin-5-yl)benzyl)-1,2,3-oxadiazol-3-ium 2,2,2-trifluoroacetate in Step 3. ¹H NMR (400 MHz, DMSO-*d*₆) δ 9.58 (s, 1H), 8.43 (s, 1H), 8.21 (s, 1H), 7.84 (br t, *J* = 2.0 Hz, 1H), 7.69 (d, *J* = 8.1 Hz, 2H), 7.53 (d, *J* = 8.3 Hz, 2H), 7.46 (br d, *J* = 9.2 Hz, 1H), 7.23 (t, *J* = 8.1 Hz, 1H), 6.93 (dd, *J₁* = 7.9 Hz, *J₂* = 1.3 Hz, 1H), 5.85 (s, 2H), 3.94 (s, 3H), 2.39 (s, 3H). LCMS (ES⁺) *m*/*z* calculated for C₂₂H₁₉ClN₆O₃ 450.12; found 449 (M-H)⁻. HPLC *t*_{R} 1.85 min.

### Example 114: ((3-cyanophenyl)carbamoyl)(3-(4-(2-methoxy-4-methylpyrimidin-5-yl)benzyl)-1,2,3-oxadiazol-3-ium-5-yl)amide.

The title compound was obtained as a beige solid reacting 3-isocyanatobenzonitrile and 5-amino-3-(4-(2-methoxy-4-methylpyrimidin-5-yl)benzyl)-1,2,3-oxadiazol-3-ium 2,2,2-trifluoroacetate in Step 3. ¹H NMR (400 MHz, DMSO-*d*₆) δ 9.74 (s, 1H), 8.43 (s, 1H), 8.23 (s, 1H), 8.12 (br s, 1H), 7.81 (br d, *J* = 8.1 Hz, 1H), 7.69 (d, *J* = 8.1 Hz, 2H), 7.53 (d, *J* = 8.1 Hz, 2H), 7.43 (br t, *J* = 8.0 Hz, 1H), 7.34 (d, *J* = 7.7 Hz, 1H), 5.87 (s, 2H), 3.94 (s, 3H), 2.39 (s, 3H). LCMS (ES⁺) *m*/*z* calculated for C₂₃H₁₉N₇O₃ 441.15; found 442 (M+H)⁺. HPLC *t*_{R} 1.65 min.

### Example 115: (3-(4-(2-methoxy-4-methylpyrimidin-5-yl)benzyl)-1,2,3-oxadiazol-3-ium-5-yl)(phenylcarbamoyl)amide.

The title compound was obtained as a beige solid reacting isocyanatobenzene and 5-amino-3-(4-(2-methoxy-4-methylpyrimidin-5-yl)benzyl)-1,2,3-oxadiazol-3-ium 2,2,2-trifluoroacetate in Step 3. ¹H NMR (400 MHz, DMSO-*d*₆) δ 9.35 (br s, 1H), 8.44 (s, 1H), 8.17 (s, 1H), 7.69 (d, *J* = 8.3 Hz, 2H), 7.61 (d, *J* = 7.7 Hz, 2H), 7.54 (d, *J* = 8.3 Hz, 2H), 7.21 (br t, *J* = 7.9 Hz, 2H), 6.90 (t, *J* = 7.3 Hz, 1H), 5.85 (s, 2H), 3.95 (s, 3H), 2.40 (s, 3H). LCMS (ES⁺) *m*/*z* calculated for C₂₂H₂₀N₆O₃ 416.16; found 415 (M-H)⁻. HPLC *t*_{R} 1.61 min.

### Example 15: (3-(1-Phenylpropan-2-yl)-1,2,3-oxadiazol-3-ium-5-yl)((5-(trifluoromethyl)pyridin-3-yl)carbamoyl)amide

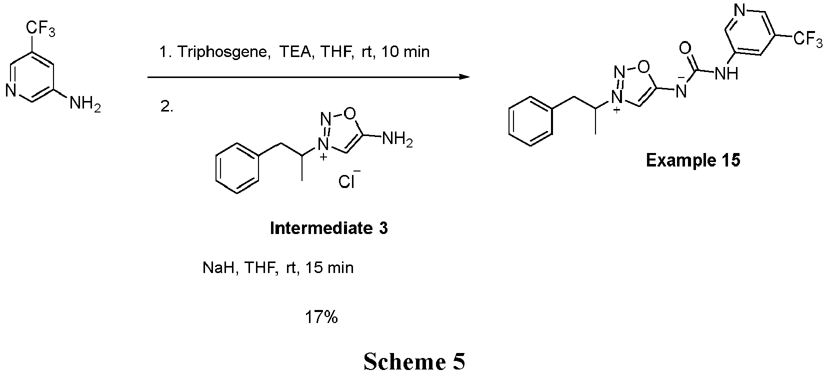

A suspension of 5-(trifluoromethyl)pyridin-3-amine (135.3 mg, 0.83 mmol) and TEA (0.23 mL, 1.67 mmol) in THF (5.4 mL) was treated with triphosgene (123.8 mg, 0.42 mmol). The reaction mixture was stirred for 10 min at rt. Diethyl ether (2 mL) was added to the mixture and the salt formed was filtered off to afford a clear solution of isocyanate in THF/ethyl ether that was stored under N₂. A solution of Intermediate **3** (100 mg, 0.420 mmol) in THF was treated with sodium hydride (35 mg, 1.04 mmol, mineral oil 60%) and the mixture was stirred at rt for 15 min. After addition of the solution of isocyanate in THF/ethyl ether, the reaction mixture was stirred for 6 h. After evaporation of the solvent under reduced pressure, the residue was purified by chromatography on silica gel (eluting with 1% of MeOH in DCM) to afford the title compound as a light beige solid (28 mg, 17%). ¹H NMR (400 MHz, DMSO-*d*₆) δ 9.90 (br s, 1H), 8.89 (s, 1H), 8.60 (s, 1H), 8.46 (s, 1H), 8.37 (s, 1H), 7.31-7.19 (m, 5H), 5.20-5.11 (m, 1H), 3.31 (m, 2H), 1.66 (d, *J* = 4.0 Hz, 3H). ¹⁹F NMR (377 MHz, DMSO-*d*₆) δ -61.21 (s, 3F). LCMS (ES⁺) *m*/*z* calculated for C₁₈H₁₆F₃N₅O₂ 391.13; found 392 (M+H)⁺. HPLC *t*_{R} 1.59 min.

**Examples 16, 34, 55, 73 and 76** were synthesized according to the above procedure (Scheme 5) by reacting with the appropriate starting materials.

### Example 16: (3-(1-phenylpropan-2-yl)-1,2,3-oxadiazol-3-ium-5-yl)((4-(trifluoromethyl) pyridin-2-yl)carbamoyl)amide.

The title compound was obtained as a yellow solid reacting 4-(trifluoromethyl)pyridin-2-amine in Step 1. LCMS (ES⁺) *m*/*z* calculated for C₁₈H₁₆F₃N₅O₂ 391.13; found 392 (M+H)⁺. HPLC *t*_{R} 1.89 min.

### Example 34: ((3-methoxy-5-(trifluoromethyl)phenyl)carbamoyl)(3-(1-phenylpropan-2-yl)-1,2,3-oxadiazol-3-ium-5-yl)amide.

The title compound was obtained as a white solid reacting 3-methoxy-5-(trifluoromethyl)aniline in Step 1. ¹H NMR (400 MHz, DMSO-*d*₆) δ 9.62 (s, 1H), 8.34 (s, 1H), 7.74 (s, 1H), 7.48 (s, 1H), 7.32-7.20 (m, 5H), 6.76 (s, 1H), 5.17-5.10 (m, 1H), 3.78 (s, 3H), 3.32-3.29 (m, 2H), 1.66 (d, *J* = 6.6 Hz, 3H). ¹⁹F NMR (377 MHz, DMSO-*d*₆) δ -61.36 (s, 3F). LCMS (ES⁺) *m*/*z* calculated for C₂₀H₁₉F₃N₄O₃ 420.14; found 421 (M+H)⁺. HPLC *t*_{R} 2.04 min.

### Example 55: (3-(4-iodobenzyl)-1,2,3-oxadiazol-3-ium-5-yl)((5-(trifluoromethyl)pyridin-3-yl)carbamoyl)amide.

The title compound was obtained as a white solid reacting 5-amino-3-(4-iodobenzyl)-1,2,3-oxadiazol-3-ium chloride in Step 1. ¹H NMR (400 MHz, DMSO-*d*₆) δ 9.97 (br s, 1H), 8.91 (d, *J* = 2.0 Hz, 1H), 8.55 (s, 1H), 8.48 (s, 1H), 8.27 (s, 1H), 7.84 (d, *J* = 8.3 Hz, 2H), 7.38 (d, *J* = 8.6 Hz, 2H), 5.77 (s, 2H). ¹⁹F NMR (377 MHz, DMSO-*d*₆) δ -61.21 (s, 3F). LCMS (ES⁺) *m*/*z* calculated for C₁₆H₁₁F₃IN₅O₂ 488.99; found 490 (M+H)⁺. HPLC *t*_{R} 2.00 min.

### Example 73: (R)-(3-(1-(4-bromophenyl)ethyl)-1,2,3-oxadiazol-3-ium-5-yl)((5-(trifluoromethyl)pyridin-3-yl)carbamoyl)amide.

The title compound was obtained as a white solid reacting (*R*)-5-amino-3-(1-(4-bromophenyl)ethyl)-1,2,3-oxadiazol-3-ium chloride in Step 1. ¹H NMR (400 MHz, DMSO-*d*₆) δ 9.96 (br s, 1H), 8.89 (d, *J* = 2.0 Hz, 1H), 8.58 (s, 1H), 8.47 (s, 1H), 8.29 (s, 1H), 7.68 (d, *J* = 8.6 Hz, 2H), 7.58 (d, *J* = 8.6 Hz, 2H), 6.18 (q, *J* = 7.0 Hz, 1H), 2.00 (d, *J* = 7.0 Hz, 3H). ¹⁹F NMR (377 MHz, DMSO-*d*₆) δ -61.22 (s, 3F). LCMS (ES⁺) *m*/*z* calculated for C₁₇H₁₃BrF₃N₅O₂ 455.02; found 455-457 (M+H)⁺. HPLC *t*_{R} 1.91 min.

### Example 76: (S)-(3-(1-(4-bromophenyl)ethyl)-1,2,3-oxadiazol-3-ium-5-yl)((5-(trifluoromethyl)pyridin-3-yl)carbamoyl)amide.

The title compound was obtained as a light orange solid reacting (*S*)-5-amino-3-(1-(4-bromophenyl)ethyl)-1,2,3-oxadiazol-3-ium chloride in Step 1. ¹H NMR (400 MHz, DMSO-*d*₆) δ 9.96 (br s, 1H), 8.89 (d, *J* = 2.2 Hz, 1H), 8.58 (s, 1H), 8.47 (s, 1H), 8.29 (s, 1H), 7.68 (d, *J* = 8.6 Hz, 2H), 7.58 (d, *J* = 8.6 Hz, 2H), 6.18 (q, *J* = 7.0 Hz, 1H), 2.00 (d, *J* = 7.0 Hz, 3H). ¹⁹F NMR (377 MHz, DMSO-*d*₆) δ -61.21 (s, 3F). LCMS (ES⁺) *m*/*z* calculated for C₁₇H₁₃BrF₃N₅O₂ 455.02; found 454-456 (M-H)⁻. HPLC *t*_{R} 1.93 min.

### Example 17: (3-([1,1'-Biphenyl]-4-ylmethyl)-1,2,3-oxadiazol-3-ium-5-yl)((3-(trifluoromethyl)phenyl)carbamoyl)amide

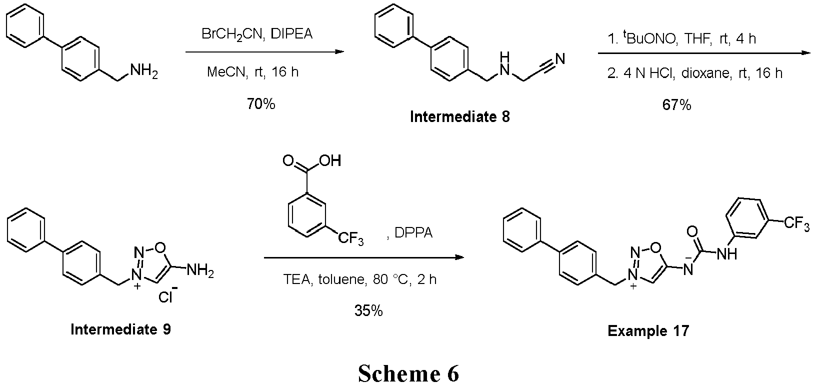

### Step 1: 2-(([1,1'-Biphenyl]-4-ylmethyl)amino)acetonitrile (Intermediate 8).

The title compound was prepared using the procedure reported for the synthesis of Intermediate **2** starting from (4-phenylphenyl)methanamine (400 mg, 2.18 mmol). The crude product was purified by chromatography on silica gel (eluting with 0-50% EtOAc in diethyl ether) to afford the title compound as a white solid (339 mg, 70%). ¹H NMR (400 MHz, DMSO-*d*₆) δ 7.67-7.62 (m, 4H), 7.48-7.41 (m, 4H), 7.36 (t, *J* = 8.0 Hz, 1H), 3.80 (br s, 2H), 3.62 (br d, *J* = 4.0 Hz, 2H), 3.08 (br s, 1H).

### Step 2: 3-([1,1'-biphenyl]-4-ylmethyl)-5-amino-1,2,3-oxadiazol-3-ium chloride (Intermediate 9).

The title compound was prepared using the procedure reported for the synthesis of Intermediate **3** starting from Intermediate **8** (339 mg, 1.53 mmol). After filtration the title compound was obtained as a white powder (258 mg, 67%). ¹H NMR (400 MHz, DMSO-*d*₆) δ 9.73 (br s, 2H), 8.16 (s, 1H), 7.76 (d, *J* = 8.0 Hz, 2H), 7.69-7.66 (m, 4H), 7.48 (t, *J* = 8.0 Hz, 2H), 7.41 (t, *J* = 8.0 Hz, 1H), 5.96 (s, 2H).

### Step 3: (3-([1,1'-biphenyl]-4-ylmethyl)-1,2,3-oxadiazol-3-ium-5-yl)((3-(trifluoromethyl)phenyl) carbamoyl)amide (Example 17).

The title compound was prepared using the procedure reported for the synthesis of **Example 1** starting from Intermediate **9** (50 mg, 0.200 mmol). After evaporation of the solvent under reduced pressure, the residue was purified by RP-HPLC using water (+ 0.1% TFA) and MeCN (+ 0.1% TFA) as eluents (C18 column). Fractions containing product were lyophilized to give the title compound as a white solid (30 mg, 35%). ¹H NMR (400 MHz, DMSO-*d*₆) δ 9.71 (s, 1H), 8.24 (s, 1H), 8.21 (br s, 1H), 7.77-7.66 (m, 7H), 7.50-7.37 (m, 4H), 7.22 (d, *J* = 8 Hz, 1H), 5.83 (s, 2H). ¹⁹F-NMR (377 MHz, DMSO-*d*₆) δ -61.29 (s, 3F). LCMS (ES⁺) *m*/*z* calculated for C₂₃H₁₇F₃N₄O₂ 438.13; found 437 (M-H)⁻. HPLC *t*_{R} 2.2 min.

### Example 24: (3-Benzyl-4-chloro-1,2,3-oxadiazol-3-ium-5-yl)((3-(trifluoromethyl)phenyl) carbamoyl)amide

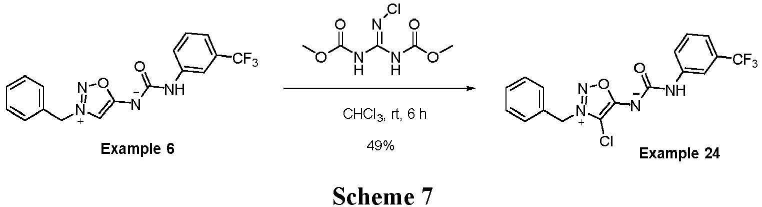

A solution of **Example 6** (30 mg, 0.08 mmol) in chloroform (0.8 mL) was treated with Palau'Chlor (17.4 mg, 0.08 mmol) and the reaction mixture was stirred at rt for 6 h. After evaporation of the solvent under reduced pressure, the residue was purified by RP-HPLC using water (+ 0.1% TFA) and MeCN (+ 0.1% TFA) as eluents (C18 column). Fractions containing product were lyophilized to give the title compound as a light-yellow solid (16 mg, 49%). ¹H NMR (400 MHz, DMSO-*d*₆) δ 9.78 (s, 1H), 8.15 (s, 1H), 7.80 (d, *J* = 8 Hz, 1H), 7.48-7.43 (m, 6H), 7.24 (d, *J* = 8 Hz, 1H), 5.83 (s, 2H). ¹⁹F NMR (377 MHz, DMSO-*d*₆) δ -61.24 (s, 3F). LCMS (ES⁺) *m*/*z* calculated for C₁₇H₁₂ClF₃N₄O₂ 396.06; found 397 (M+H)⁺. HPLC *t*_{R} 1.91 min.

### Example 26: (3-(4-(Pyrimidin-5-yl)benzyl)-1,2,3-oxadiazol-3-ium-5-yl)((3-(trifluoromethyl)phenyl)carbamoyl)amide.

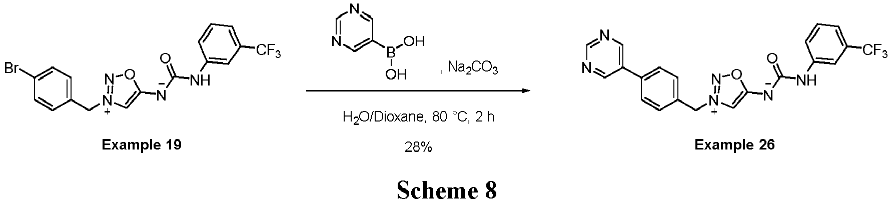

A solution of **Example 19** (50 mg, 0.110 mmol), tetrakis(triphenylphosphine)palladium(0) (5.9 mg, 0.01 mmol), disodium carbonate (36.0 mg, 0.34 mmol) and pyrimidin-5-ylboronic acid (18.3 mg, 0.15 mmol) in dioxane (2 mL) and water (0.4 mL) in a sealed tube was heated at 80 °C for 2 h. After cooling the reaction mixture was filtered on a pad of solka floc and the filtrate was evaporated under reduced pressure. The residue was purified by RP-HPLC using water (+ 0.1% TFA) and MeCN (+ 0.1% TFA) as eluents (C18 column). Fractions containing product were lyophilized to give the title compound as a white solid (14 mg, 28%). ¹H NMR (400 MHz, DMSO-*d*₆) δ 9.71 (br s, 1H), 9.21 (s, 1H), 9.17 (s, 2H), 8.24 (s, 1H), 8.20 (s, 1H), 7.91 (d, *J* = 8 Hz, 2H), 7.75 (d, *J* = 8 Hz, 2H), 7.72-7.69 (m, 1H), 7.43 (t, *J* = 8 Hz, 1H), 7.21 (d, *J* = 8 Hz, 1H), 5.86 (s, 2H). ¹³C NMR (101 MHz, DMSO-*d*₆) δ 172.6, 159.0, 157.5, 154.9, 141.8, 134.9, 132.8, 132.5, 130.1, 129.5, 129.1, 127.7, 125.7, 121.3, 117.3, 113.7, 104.5, 55.5. ¹⁹F NMR (377 MHz, DMSO-*d*₆) δ -61.29 (s, 3F). LCMS (ES⁺) *m*/*z* calculated for C₂₁H₁₅F₃N₆O₂ 440.12; found 441 (M+H)⁺. HPLC *t*_{R} 1.62 min.

**Examples 20, 21, 22, 23, 41, 48, 72, 74, 75, 77, 78, 93, 95, 96 and 97** were synthesized according to the above procedure (Scheme 8) by reacting with the appropriate starting materials.

### Example 20: (3-(4-(thiophen-3-yl)benzyl)-1,2,3-oxadiazol-3-ium-5-yl)((3(trifluoromethyl) phenyl)carbamoyl)amide.

The title compound was obtained as a white solid reacting thiophen-3-ylboronic acid in Step 1. ¹H NMR (400 MHz, DMSO-*d*₆) δ 9.71 (s, 1H), 8.21 (br s, 2H), 7.95 (br s, 1H), 7.81 (br d, *J* = 7.7 Hz, 2H), 7.72 (br d, *J* = 8.3 Hz, 1H), 7.67-7.59 (m, 4H), 7.43 (t, *J* = 7.9 Hz, 1H), 7.22 (br d, *J* = 7.9 Hz, 1H), 5.80 (s, 2H). ¹⁹F NMR (377 MHz, DMSO-*d*₆) δ -61.29 (s, 3F). LCMS (ES⁺) *m*/*z* calculated for C₂₁H₁₅F₃N₄O₂S 444.09; found 443 (M-H)⁻. HPLC *t*_{R} 2.12 min.

### Example 21: (3-((2'-methyl-[1,1'-biphenyl]-4-yl)methyl)-1,2,3-oxadiazol-3-ium-5-yl)((3-(trifluoromethyl)phenyl)carbamoyl)amide.

The title compound was obtained as a white solid reacting o-tolylboronic acid in Step 1. ¹H NMR (400 MHz, DMSO-*d*₆) δ 9.72 (s, 1H), 8.28 (s, 1H), 8.22 (br s, 1H), 7.73 (br d, *J* = 8.1 Hz, 1H), 7.64 (br d, *J* = 7.7 Hz, 2H), 7.44 (br d, *J* = 7.2 Hz, 3H), 7.30-7.20 (m, 5H), 5.84 (s, 2H), 2.23 (s, 3H). ¹⁹F NMR (377 MHz, DMSO-*d*₆) δ -61.28 (s, 3F). LCMS (ES⁺) *m*/*z* calculated for C₂₄H₁₉F₃N₄O₂ 452.15; found 451 (M-H)⁻. HPLC *t*_{R} 2.29 min.

### Example 22: (3-(4-(pyridin-3-yl)benzyl)-1,2,3-oxadiazol-3-ium-5-yl)((3-(trifluoromethyl) phenyl)carbamoyl)amide.

The title compound was obtained as a white solid reacting pyridin-3-ylboronic acid in Step 1. ¹H NMR (400 MHz, DMSO-*d*₆) δ 9.72 (s, 1H), 8.92 (s, 1H), 8.60 (br d, *J* = 4.6 Hz, 1H), 8.25 (s, 1H), 8.21 (br s, 1H), 8.11 (br d, *J* = 7.9 Hz, 1H), 7.84 (br d, *J* = 7.7 Hz, 2H), 7.72 (br d, *J* = 7.9 Hz, 3H), 7.53-7.49 (m, 1H), 7.44 (br t, *J* = 8.0 Hz, 1H), 7.22 (br d, *J* = 7.7 Hz, 1H), 5.85 (s, 2H). ¹⁹F NMR (377 MHz, DMSO-*d*₆) δ -61.28 (s, 3F). LCMS (ES⁺) *m*/*z* calculated for C₂₂H₁₆F₃N₅O₂ 439.13; found 438 (M-H)⁻. HPLC *t*_{R} 1.45 min.

### Example 23: (3-(4-(1-methyl-1H-pyrazol-4-yl)benzyl)-1,2,3-oxadiazol-3-ium-5-yl)((3-(trifluoromethyl)phenyl)carbamoyl)amide.

The title compound was obtained as a white solid reacting 1-methyl-4-(4,4,5,5-tetramethyl-1,3,2-dioxaborolan-2-yl)-1H-pyrazole in Step 1. ¹H NMR (400 MHz, DMSO-*d*₆) δ 9.69 (s, 1H), 8.19 (br s, 1H), 8.17 (br s, 2H), 7.89 (br s, 1H), 7.71 (br d, *J* = 8.1 Hz, 1H), 7.65-7.63 (m, 2H), 7.56-7.54 (m, 2H), 7.42 (br t, *J* = 8.0 Hz, 1H), 7.21 (br d, *J* = 7.5 Hz, 1H), 5.74 (s, 2H), 3.86 (s, 3H).

¹⁹F NMR (377 MHz, DMSO-*d*₆) δ -61.28 (s, 3F). LCMS (ES⁺) *m*/*z* calculated for C₂₁H₁₇F₃N₆O₂ 442.14; found 441 (M-H)⁻. HPLC *t*_{R} 1.71 min.

### Example 41: (3-(4-(2-methoxypyrimidin-5-yl)benzyl)-1,2,3-oxadiazol-3-ium-5-yl)((3-(trifluoromethyl)phenyl)carbamoyl)amide.

The title compound was obtained as a white solid reacting (2-methoxypyrimidin-5-yl)boronic acid in Step 1. ¹H NMR (400 MHz, DMSO-*d*₆) δ 9.71 (s, 1H), 8.97 (s, 2H), 8.23 (s, 1H), 8.21 (br s, 1H), 7.83 (br d, *J* = 8.1 Hz, 2H), 7.71 (br d, *J=* 8.1 Hz, 3H), 7.44 (br t, *J* = 8.0 Hz, 1H), 7.22 (br d, *J* = 7.7 Hz, 1H), 5.84 (s, 2H), 3.98 (s, 3H). ¹⁹F NMR (377 MHz, DMSO-*d*₆) δ -61.29 (s, 3F). LCMS (ES⁺) *m*/*z* calculated for C₂₂H₁₇F₃N₆O₃ 470.13; found 469 (M-H)⁻. HPLC *t*_{R} 1.82 min.

### Example 48: (3-(4-(isoxazol-4-yl)benzyl)-1,2,3-oxadiazol-3-ium-5-yl)((3-(trifluoromethyl) phenyl)carbamoyl)amide.

The title compound was obtained as a white solid reacting isoxazol-4-ylboronic acid in Step 1. ¹H NMR (400 MHz, DMSO-*d*₆) δ 9.73 (s, 1H), 9.51 (s, 1H), 9.21 (s, 1H), 8.21 (br s, 2H), 7.80 (br d, *J* = 8.3 Hz, 2H), 7.71 (br d, *J* = 9.2 Hz, 1H), 7.66 (br d, *J* = 8.3 Hz, 2H), 7.43 (br t, *J* = 7.9 Hz, 1H), 7.22 (br d, *J* = 7.7 Hz, 1H), 5.80 (s, 2H). ¹⁹F NMR (377 MHz, DMSO-*d*₆) δ -61.28 (s, 3F). LCMS (ES⁺) *m*/*z* calculated for C₂₀H₁₄F₃N₅O₃ 429.10; found 428 (M-H)⁻. HPLC *t*_{R} 1.86 min.

### Example 72: (R)-(3-(1-(4-(pyrimidin-5-yl)phenyl)ethyl)-1,2,3-oxadiazol-3-ium-5-yl)((3-(trifluoromethyl)phenyl)carbamoyl)amide.

The title compound was obtained as a white solid reacting pyrimidin-5-ylboronic acid and (*R*)-(3-(1-(4-bromophenyl)ethyl)-1,2,3-oxadiazol-3-ium-5-yl)((3-(trifluoromethyl)phenyl)carbamoyl)amide (prepared as reported in Scheme 1) in Step 1. ¹H NMR (400 MHz, DMSO-*d*₆) δ 9.65 (s, 1H), 9.14 (s, 1H), 9.10 (s, 2H), 8.20 (s, 1H), 8.17 (br s, 1H), 7.85 (br d, *J* = 8.1 Hz, 2H), 7.72 (br d, *J* = 8.1 Hz, 2H), 7.61 (br d, *J* = 8.6 Hz, 1H), 7.36 (br t, *J* = 7.9 Hz, 1H), 7.15 (br d, *J* = 7.5 Hz, 1H), 6.17 (q, *J* = 7.0 Hz, 1H), 1.99 (br d, *J* = 7.0 Hz, 3H). ¹⁹F NMR (377 MHz, DMSO-*d*₆) δ -61.27 (s, 3F). LCMS (ES⁺) *m*/*z* calculated for C₂₂H₁₇F₃N₆O₂ 454.14; found 453 (M-H)⁻. HPLC *t*_{R} 1.71 min.

### Example 74: (S)-(3-(1-(4-(pyrimidin-5-yl)phenyl)ethyl)-1,2,3-oxadiazol-3-ium-5-yl)((3-(trifluoromethyl)phenyl)carbamoyl)amide.

The title compound was obtained as a white solid reacting pyrimidin-5-ylboronic acid and (*S*)-(3-(1-(4-bromophenyl)ethyl)-1,2,3-oxadiazol-3-ium-5-yl)((3-(trifluoromethyl)phenyl)carbamoyl)amide (prepared as reported in Scheme 1) in Step 1. ¹H NMR (400 MHz, DMSO-*d*₆) δ 9.65 (s, 1H), 9.14 (s, 1H), 9.10 (s, 2H), 8.20 (s, 1H), 8.17 (br s, 1H), 7.85 (br d, *J* = 8.6 Hz, 2H), 7.72 (br d, *J* = 8.3 Hz, 2H), 7.61 (br d, *J* = 8.3 Hz, 1H), 7.36 (br t, *J* = 7.4 Hz, 1H), 7.14 (br d, *J* = 8.3 Hz, 1H), 6.17 (q, *J* = 7.2 Hz, 1H), 1.99 (br d, *J* = 7.0 Hz, 3H). ¹⁹F NMR (377 MHz, DMSO-*d*₆) δ -61.27 (s, 3F). LCMS (ES⁺) *m*/*z* calculated for C₂₂H₁₇F₃N₆O₂ 454.14; found 453 (M-H)⁻. HPLC *t*_{R} 1.71 min.

### Example 75: (R)-(3-(1-(4-(pyrimidin-5-yl)phenyl)ethyl)-1,2,3-oxadiazol-3-ium-5-yl)((5-(trifluoromethyl)pyridin-3-yl)carbamoyl)amide.

The title compound was obtained as a white solid reacting pyrimidin-5-ylboronic acid and (*R*)-(3-(1-(4-bromophenyl)ethyl)-1,2,3-oxadiazol-3-ium-5-yl)((5-(trifluoromethyl)pyridin-3-yl)carbamoyl)amide (prepared as reported in Scheme 1) in Step 1. ¹H NMR (400 MHz, DMSO-*d*₆) δ 9.96 (br s, 1H), 9.22 (s, 1H), 9.18 (s, 2H), 8.89 (br d, *J* = 2.0 Hz, 1H), 8.58 (br s, 1H), 8.47 (br s, 1H), 8.32 (s, 1H), 7.92 (br d, *J* = 8.3 Hz, 2H), 7.79 (br d, *J* = 8.3 Hz, 2H), 6.27 (q, *J* = 7.0 Hz, 1H), 2.07 (br d, *J* = 7.0 Hz, 3H). ¹⁹F NMR (377 MHz, DMSO-*d*₆) δ -61.21 (s, 3F). LCMS (ES⁺) *m*/*z* calculated for C₂₁H₁₆F₃N₇O₂ 455.13; found 456 (M+H)⁺. HPLC *t*_{R} 1.49 min.

### Example 77: (S)-(3-(1-(4-(pyrimidin-5-yl)phenyl)ethyl)-1,2,3-oxadiazol-3-ium-5-yl)((5-(trifluoromethyl)pyridin-3-yl)carbamoyl)amide.

The title compound was obtained as a white solid reacting pyrimidin-5-ylboronic acid and (*S*)-(3-(1-(4-bromophenyl)ethyl)-1,2,3-oxadiazol-3-ium-5-yl)((5-(trifluoromethyl)pyridin-3-yl)carbamoyl)amide (prepared as reported in Scheme 1) in Step 1. ¹H NMR (400 MHz, DMSO-*d*₆) δ 9.89 (br s, 1H), 9.14 (s, 1H), 9.10 (s, 2H), 8.82 (br d, *J* = 2.0 Hz, 1H), 8.50 (br s, 1H), 8.40 (br s, 1H), 8.25 (s, 1H), 7.85 (br d, *J* = 8.3 Hz, 2H), 7.72 (br d, *J* = 8.3 Hz, 2H), 6.19 (q, *J* = 7.0 Hz, 1H), 1.99 (br d, *J* = 7.0 Hz, 3H). ¹⁹F NMR (377 MHz, DMSO-*d*₆) δ -61.22 (s, 3F). LCMS (ES⁺) *m*/*z* calculated for C₂₁H₁₆F₃N₇O₂ 455.13; found 456 (M+H)⁺. HPLC *t*_{R} 1.49 min.

### Example 78: (3-(4-(3,5-dimethylisoxazol-4-yl)benzyl)-1,2,3-oxadiazol-3-ium-5-yl)((3-(trifluoromethyl)phenyl)carbamoyl)amide.

The title compound was obtained as a white solid reacting (3,5-dimethylisoxazol-4-yl)boronic acid in Step 1. ¹H NMR (400 MHz, DMSO-*d*₆) δ 9.73 (s, 1H), 8.28 (s, 1H), 8.21 (s, 1H), 7.72 (br d, *J* = 8.1 Hz, 1H), 7.68 (br d, *J* = 8.1 Hz, 2H), 7.50 (br d, *J* = 8.1 Hz, 2H), 7.44 (br t, *J* = 8.0 Hz, 1H), 7.22 (d, *J* = 7.9 Hz, 1H), 5.83 (s, 2H), 2.41 (s, 3H), 2.24 (s, 3H). ¹⁹F NMR (377 MHz, DMSO-*d*₆) δ -61.29 (s, 3F). LCMS (ES⁺) *m*/*z* calculated for C₂₂H₁₈F₃N₅O₃ 457.14; found 458 (M+H)⁺. HPLC *t*_{R} 1.92 min.

### Example 93: (3-(4-(5-methylpyridazin-4-yl)benzyl)-1,2,3-oxadiazol-3-ium-5-yl)((3-(trifluoromethyl)phenyl)carbamoyl)amide.

The title compound was obtained as a beige solid reacting 4-methyl-5-(4,4,5,5-tetramethyl-1,3,2-dioxaborolan-2-yl)pyridazine in Step 1. ¹H NMR (400 MHz, DMSO-*d*₆) δ 9.74 (s, 1H), 9.19 (s, 1H), 9.07 (s, 1H), 8.30 (s, 1H), 8.22 (s, 1H), 7.76-7.71 (m, 3H), 7.63 (br d, *J* = 8.1 Hz, 2H), 7.44 (br t, *J* = 7.5 Hz, 1H), 7.23 (d, *J* = 7.5 Hz, 1H), 5.88 (s, 2H), 2.33 (s, 3H). ¹⁹F NMR (377 MHz, DMSO-*d*₆) δ -61.29 (s, 3F). LCMS (ES⁺) *m*/*z* calculated for C₂₂H₁₇F₃N₆O₂ 454.14; found 455 (M+H)⁺. HPLC *t*_{R} 1.61 min.

### Example 95: (3-(4-(1,5-dimethyl-1H-pyrazol-4-yl)benzyl)-1,2,3-oxadiazol-3-ium-5-yl)((3-(trifluoromethyl)phenyl)carbamoyl)amide.

The title compound was obtained as a white solid reacting (1,5-dimethyl-1*H*-pyrazol-4-yl)boronic acid in Step 1. ¹H NMR (400 MHz, DMSO-*d*₆) δ 9.81 (s, 1H), 8.31 (br s, 2H), 7.81 (br d, *J* = 8.6 Hz, 1H), 7.69 (br d, *J* = 6.4 Hz, 3H), 7.58 (d, *J* = 8.1 Hz, 2H), 7.53 (t, *J* = 7.9 Hz, 1H), 7.31 (br d, *J* = 7.2 Hz, 1H), 5.88 (s, 2H), 3.88 (s, 3H), 2.47 (s, 3H). ¹⁹F NMR (377 MHz, DMSO-*d*₆) δ -61.28 (s, 3F). LCMS (ES⁺) *m*/*z* calculated for C₂₂H₉F₃N₆O₂ 456.15; found 455 (M-H)⁻. HPLC *t*_{R} 1.81 min.

### Example 96: (3-(4-(1,3-dimethyl-1H-pyrazol-4-yl)benzyl)-1,2,3-oxadiazol-3-ium-5-yl)((3-(trifluoromethyl)phenyl)carbamoyl)amide.

The title compound was obtained as a white solid reacting (1,3-dimethyl-1*H*-pyrazol-4-yl)boronic acid in Step 1. ¹H NMR (400 MHz, DMSO-*d*₆) δ 9.71 (s, 1H), 8.21 (br s, 2H), 7.94 (s, 1H), 7.72 (br d, *J* = 7.9 Hz, 1H), 7.58 (br d, *J* = 8.1 Hz, 2H), 7.51 (br d, *J* = 8.1 Hz, 2H), 7.44 (br t, *J* = 8.1 Hz, 1H), 7.22 (d, *J* = 7.5 Hz, 1H), 5.77 (s, 2H), 3.79 (s, 3H), 2.30 (s, 3H). ¹⁹F NMR (377 MHz, DMSO-*d*₆) δ -61.28 (s, 3F). LCMS (ES⁺) *m*/*z* calculated for C₂₂H₁₉F₃N₆O₂ 456.15; found 455 (M+H)⁺. HPLC *t*_{R} 1.79 min.

### Example 97: (3-(trifluoromethyl)phenyl)carbamoyl)(3-(4-(1,3,5-trimethyl-1H-pyrazol-4-yl)benzyl)-1,2,3-oxadiazol-3-ium-5-yl)amide.

The title compound was obtained as a white solid reacting (1,3,5-trimethyl-1*H*-pyrazol-4-yl)boronic acid in Step 1. ¹H NMR (400 MHz, DMSO-*d*₆) δ 9.73 (s, 1H), 8.26 (s, 1H), 8.21 (br s, 1H), 7.72 (br d, *J* = 8.6 Hz, 1H), 7.61 (d, *J* = 8.1 Hz, 2H), 7.44 (br t, *J* = 7.9 Hz, 1H), 7.35 (d, *J* = 7.9 Hz, 2H), 7.22 (d, *J* = 7.7 Hz, 1H), 5.80 (s, 2H), 3.70 (s, 3H), 2.22 (s, 3H), 2.13 (s, 3H). ¹⁹F NMR (377 MHz, DMSO-*d*₆) δ -61.29 (s, 3F). LCMS (ES⁺) *m*/*z* calculated for C₂₃H₂₁F₃N₆O₂ 470.17; found 469 (M-H)⁻. HPLC *t*_{R} 1.81 min.

### Example 50: (3-(3-(Pyrimidin-5-yl)benzyl)-1,2,3-oxadiazol-3-ium-5-yl)((3-(trifluoromethyl) phenyl)carbamoyl)amide.

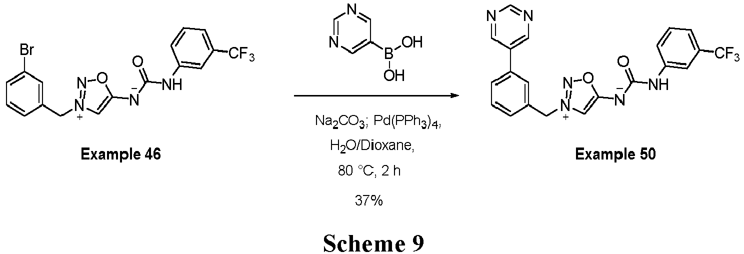

The title compound was prepared using procedure reported for the synthesis of compound **26** starting from **Example 46.** The crude product was purified by RP-HPLC using water (+ 0.1% TFA) and MeCN (+ 0.1% TFA) as eluents (C18 column). Fractions containing product were lyophilized to give the title compound as a white solid (18.4 mg, 37%). ¹H NMR (400 MHz, DMSO-*d*₆) δ 9.73 (br s, 1H), 9.23 (s, 1H), 9.19 (s, 2H), 8.28 (s, 1H), 8.21 (br s, 1H), 8.09 (br s, 1H), 7.92 (d, *J* = 7.7 Hz, 1H), 7.72-7.62 (m, 3H), 7.43 (t, *J* = 8.0 Hz, 1H), 7.22 (d, *J* = 7.7 Hz, 1H), 5.85 (s, 2H). ¹⁹F NMR (377 MHz, DMSO-*d*₆) δ -61.28 (s, 3F). LCMS (ES⁺) *m*/*z* calculated for C₂₁H₁₅F₃N₆O₂ 440.12; found 439 (M-H)⁻. HPLC *t*_{R} 1.68 min.

### Examples 51, 52, 53 and 94 were synthesized according to the above procedure (Scheme 9) by reacting with the appropriate starting materials.

### Example 51: (3-(3-(pyridin-3-yl)benzyl)-1,2,3-oxadiazol-3-ium-5-yl)((3-(trifluoromethyl) phenyl)carbamoyl)amide

The title compound was obtained as a white solid reacting pyridin-3-ylboronic acid in Step 1. ¹H NMR (400 MHz, DMSO-*d*₆) δ 9.72 (s, 1H), 8.96 (br d, *J* = 1.8 Hz, 1H), 8.63 (dd, *J₁* = 4.8 Hz, *J₂* = 1.3 Hz, 1H), 8.28 (s, 1H), 8.21 (br s, 1H), 8.17 (br d, *J* = 7.9 Hz, 1H), 8.02 (br s, 1H), 7.84 (br d, *J* = 7.0 Hz, 1H), 7.71 (br d, *J* = 7.9 Hz, 1H), 7.65-7.56 (m, 3H), 7.43 (t, *J* = 7.9 Hz, 1H), 7.22 (br d, *J* = 7.7 Hz, 1H), 5.85 (s, 2H). ¹⁹F NMR (377 MHz, DMSO-*d*₆) δ -61.28 (s, 3F). LCMS (ES⁺) *m*/*z* calculated for C₂₂H₁₆F₃N₅O₂ 439.13; found 438 (M-H)⁻. HPLC *t*_{R} 1.54 min.

### Example 52: (3-(3-(1-methyl-1H-pyrazol-4-yl)benzyl)-1,2,3-oxadiazol-3-ium-5-yl)((3-(trifluoromethyl)phenyl)carbamoyl)amide.

The title compound was obtained as a white solid reacting 1-methyl-4-(4,4,5,5-tetramethyl-1,3,2-dioxaborolan-2-yl)-1*H*-pyrazole in Step 1. ¹H NMR (400 MHz, DMSO-*d*₆) δ 9.72 (s, 1H), 8.21 (s, 1H), 8.20 (br s, 1H), 8.17 (s, 1H), 7.89 (s, 1H), 7.78 (br s, 1H), 7.71 (br d, *J* = 8.3 Hz, 1H), 7.64 (d, *J* = 7.7 Hz, 1H), 7.46-7.37 (m, 3H), 7.22 (br d, *J* = 7.7 Hz, 1H), 5.76 (s, 2H), 3.87 (s, 3H). ¹⁹F NMR (377 MHz, DMSO-*d*₆) δ -61.27 (s, 3F). LCMS (ES⁺) *m*/*z* calculated for C₂₁H₁₇F₃N₆O₂ 442.14; found 441 (M-H)⁻. HPLC *t*_{R} 1.77 min.

### Example 53: (3-(3-(2-methoxypyrimidin-5-yl)benzyl)-1,2,3-oxadiazol-3-ium-5-yl)((3-(trifluoromethyl)phenyl)carbamoyl)amide.

The title compound was obtained as a white solid reacting (3,5-dimethylisoxazol-4-yl)boronic acid in Step 1. ¹H NMR (400 MHz, DMSO-*d*₆) δ 9.72 (s, 1H), 8.97 (s, 2H), 8.27 (s, 1H), 8.21 (br s, 1H), 8.00 (s, 1H), 7.83 (br d, *J* = 6.4 Hz, 1H), 7.71 (d, *J* = 8.1 Hz, 1H), 7.62-7.60 (m, 2H), 7.43 (br t, *J* = 7.9 Hz, 1H), 7.22 (br d, *J* = 7.5 Hz, 1H), 5.83 (s, 2H), 3.98 (s, 3H). ¹⁹F NMR (377 MHz, DMSO-*d*₆) δ -61.28 (s, 3F). LCMS (ES⁺) *m*/*z* calculated for C₂₂H₁₇F₃N₆O₃ 470.13; found 469 (M-H)⁻. HPLC *t*_{R} 1.86 min.

### Example 94: (3-(3-(3,5-dimethylisoxazol-4-yl)benzyl)-1,2,3-oxadiazol-3-ium-5-yl)((3-(trifluoromethyl)phenyl)carbamoyl)amide.

The title compound was obtained as a white solid reacting (3,5-dimethylisoxazol-4-yl)boronic acid in Step 1 (K₂CO₃ in a mixture of MeOH/dioxane was used). ¹H NMR (400 MHz, DMSO-*d*₆) δ 9.64 (s, 1H), 8.21 (s, 1H), 8.13 (br s, 1H), 7.64 (br d, *J* = 8.1 Hz, 1H), 7.57 (s, 1H), 7.51-7.49 (m, 2H), 7.43-7.40 (m, 1H), 7.36 (br t, *J* = 7.9 Hz, 1H), 7.14 (br d, *J* = 7.7 Hz, 1H), 5.76 (s, 2H), 2.35 (s, 3H), 2.18 (s, 3H). ¹⁹F NMR (377 MHz, DMSO-*d*₆) δ -61.29 (s, 3F). LCMS (ES⁺) *m*/*z* calculated for C₂₂H₁₈F₃N₅O₃ 457.14; found 456 (M-H)⁻. HPLC *t*_{R} 1.96 min.

### Example 57: (3-(4-(pyrimidin-5-yl)benzyl)-1,2,3-oxadiazol-3-ium-5-yl)((5-(trifluoromethyl) pyridin-3-yl)carbamoyl)amide

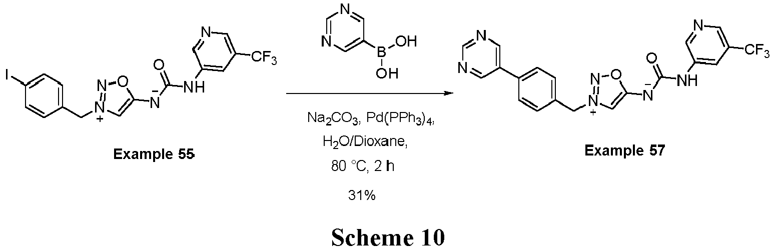

The title compound was prepared using procedure reported for the synthesis of **Example 26** starting from **Example 55.** The title compound was obtained after purification by RP-HPLC using water (+ 0.1% TFA) and MeCN (+ 0.1% TFA) as eluents (C18 column). Fractions containing product were lyophilized to give the title compound as a white solid (13 mg, 31%). ¹H NMR (400 MHz, DMSO-*d*₆) δ 9.90 (br s, 1H), 9.15 (s, 1H), 9.11 (s, 2H), 8.83 (d, *J* = 2.2 Hz, 1H), 8.48 (s, 1H), 8.40 (s, 1H), 8.24 (s, 1H), 7.85 (d, *J* = 8.1 Hz, 2H), 7.68 (d, *J* = 8.3 Hz, 2H), 5.81 (s, 2H). ¹⁹F NMR (377 MHz, DMSO-*d*₆) δ -61.22 (s, 3F). LCMS (ES⁺) *m*/*z* calculated for C₂₀H₁₄F₃N₇O₂ 441.12; found 442 (M+H)⁺. HPLC *t*_{R} 1.41 min.

**Examples 56, 58 and 67** were synthesized according to the above procedure (Scheme 10) by reacting with the appropriate starting materials.

### Example 56: (3-((4'-(morpholinomethyl)-[1,1'-biphenyl]-4-yl)methyl)-1,2,3-oxadiazol-3-ium-5-yl)((3-(trifluoromethyl)phenyl)carbamoyl)amide.

The title compound was obtained as a white solid reacting 4-(4-(4,4,5,5-tetramethyl-1,3,2-dioxaborolan-2-yl)benzyl)morpholine in Step 1. ¹H NMR (400 MHz, DMSO-*d*₆) δ 9.73 (s, 1H), 8.24 (s, 1H), 8.21 (br s, 1H), 7.77-7.65 (m, 7H), 7.46-7.40 (m, 3H), 7.22 (br d, *J* = 7.9 Hz, 1H), 5.83 (s, 2H), 3.58 (br s, 4H), 3.50 (br s, 2H), 2.37 (br s, 4H). ¹⁹F NMR (377 MHz, DMSO-*d*₆) δ - 61.27 (s, 3F). LCMS (ES⁺) *m*/*z* calculated for C₂₈H₂₆F₃N₅O₃ 537.20; found 536 (M-H)⁻. HPLC *t*_{R} 1.48 min.

### Example 58: (3-(4-(2-methoxypyrimidin-5-yl)benzyl)-1,2,3-oxadiazol-3-ium-5-yl)((5-(trifluoromethyl)pyridin-3-yl)carbamoyl)amide.

The title compound was obtained as a white solid reacting (2-methoxypyrimidin-5-yl)boronic acid and (3-(4-iodobenzyl)-1,2,3-oxadiazol-3-ium-5-yl)((5-(trifluoromethyl)pyridin-3-yl)carbamoyl)amide (prepared as reported for the synthesis of Example **15**) in Step 1. ¹H NMR (400 MHz, DMSO-*d*₆) δ 9.89 (br s, 1H), 8.90 (s, 2H), 8.83 (br d, *J* = 1.8 Hz, 1H), 8.48 (br s, 1H), 8.40 (br s, 1H), 8.22 (s, 1H), 7.76 (br d, *J* = 8.3 Hz, 2H), 7.64 (br d, *J* = 8.3 Hz, 2H), 5.79 (s, 2H), 3.90 (s, 3H). ¹⁹F NMR (377 MHz, DMSO-*d*₆) δ -61.22 (s, 3F). LCMS (ES⁺) *m*/*z* calculated for C₂₁H₁₆F₃N₇O₃ 471.13; found 472 (M+H)⁺. HPLC *t*_{R} 1.58 min.

### Example 67: (3-(4-(pyridin-4-yl)benzyl)-1,2,3-oxadiazol-3-ium-5-yl)((5-(trifluoromethyl)pyridin-3-yl)carbamoyl)amide.

The title compound was obtained as a white solid reacting pyridin-4-ylboronic acid and (3-(4-iodobenzyl)-1,2,3-oxadiazol-3-ium-5-yl)((5-(trifluoromethyl)pyridin-3-yl)carbamoyl)amide (prepared as reported for the synthesis of Example **15**) in Step 1. LCMS (ES⁺) *m*/*z* calculated for C₂₁H₁₅F₃N₆O₂ 440.12; found 439 (M-H)⁻. HPLC *t*_{R} 1.07 min.

### Example 60: (3-(4-(4-methylpyrimidin-5-yl)benzyl)-1,2,3-oxadiazol-3-ium-5-yl)((3-(trifluoromethyl)phenyl)carbamoyl)amide

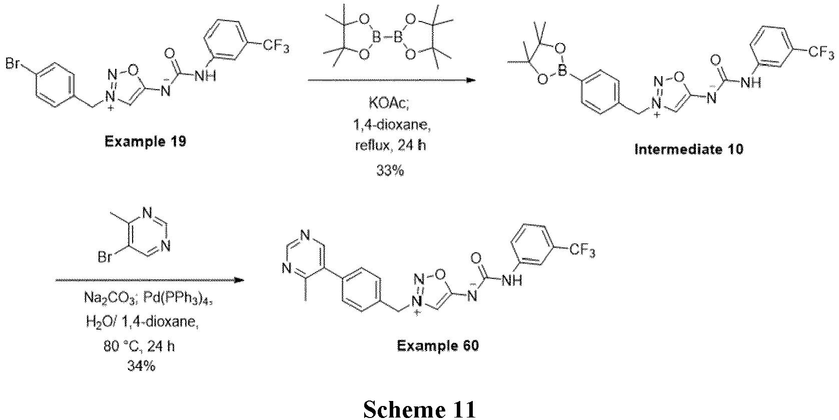

### Step 1: (3-(4-(4,4,5,5-tetramethyl-1,3,2-dioxaborolan-2-yl) benzyl)-1,2,3-oxadiazol-3-ium-5-yl)((3-(trifluoromethyl)phenyl)carbamoyl)amide (Intermediate 10).

A solution of **Example 19** (390 mg, 0.88 mmol) in dioxane (25 mL) was treated with bis(chloranyl)palladium cyclopentyl(diphenyl)phosphane ferrocene (32.8 mg, 0.04 mmol), 4,4,5,5-tetramethyl-2-(4,4,5,5-tetramethyl-1,3,2-dioxaborolan-2-yl)-1,3,2-dioxaborolane (246.9 mg, 0.97 mmol) and potassium acetate (260.3 mg, 2.65 mmol). The reaction mixture was refluxed for 18 h. After cooling down, it was quenched with water and extracted with EtOAc. The combined organic phase was washed with brine and dried over Na₂SO₄ and evaporated under reduced pressure. The title compound was obtained after purification on RP (C18) silica gel (eluting with 10-40% MeCN in H₂O) as a yellow solid (361 mg, 33%). ¹H NMR (400 MHz, DMSO-*d*₆) δ 9.70 (br s, 1H), 8.18 (br s, 2H), 7.75 (d, *J* = 7.7 Hz, 2H), 7.72 (br d, *J* = 9.2 Hz, 1H), 7.56 (d, *J* = 7.9 Hz, 2H), 7.43 (t, *J* = 8.1 Hz, 1H), 7.21 (d, *J* = 7.9 Hz, 1H), 5.81 (s, 2H), 1.29 (s, 12H). LCMS (ES⁺) *m*/*z* calculated for C₂₃H₂₄BF₃N₄O₄ 488.18; found 489 (M+H)⁺. HPLC *t*_{R} 2.24 min.

### Step 2: (3-(4-(4-methylpyrimidin-5-yl)benzyl)-1,2,3-oxadiazol-3-ium-5-yl)((3-(trifluoromethyl)phenyl)carbamoyl)amide (Example 60).

The title compound was prepared using conditions reported for the synthesis of **Example 26** using Intermediate **10** and 5-bromo-4-methylpyrimidine. The title compound was obtained after purification by RP (C18) chromatography (eluting with 10-45% MeCN in H₂O). Fractions containing product were lyophilized to give the title compound as a white solid (16 mg, 34%). ¹H NMR (400 MHz, DMSO-*d*₆) δ 9.73 (br s, 1H), 9.05 (s, 1H), 8.62 (s, 1H), 8.29 (s, 1H), 8.21 (br s, 1H), 7.72 (d, *J* = 8.3 Hz, 3H), 7.58 (d, *J* = 8.1 Hz, 2H), 7.43 (t, *J* = 7.9 Hz, 1H), 7.22 (d, *J* = 7.5 Hz, 1H), 5.86 (s, 2H), 2.45 (s, 3H). ¹³C NMR (101 MHz, DMSO-*d*₆) δ 171.5, 162.7, 157.9, 155.9, 155.0, 140.7, 135.5, 132.3, 131.1, 128.7, 128.4, 128.0, 124.6, 121.9, 120.2, 116.2, 112.6, 103.5, 54.4, 21.5. ¹⁹F NMR (377 MHz, DMSO-*d*₆) δ -61.28 (s, 3F). LCMS (ES⁺) *m*/*z* calculated for C₂₂H₁₇F₃N₆O₂ 454.14; found 455 (M+H)⁺. HPLC *t*_{R} 1.66 min.

**Examples 39, 42, 61, 62, 63, 64, 79, 105 and 106** were synthesized according to the above procedure (Scheme 11) by reacting with the appropriate starting materials.

### Example 39: (3-(4-(pyridin-4-yl)benzyl)-1,2,3-oxadiazol-3-ium-5-yl)((3-(trifluoromethyl) phenyl)carbamoyl)amide.

The title compound was obtained as a white solid reacting 4-bromopyridine in Step 2. ¹H NMR (400 MHz, CDCl₃) δ 8.74 (br s, 2H), 7.92 (br s, 1H), 7.80 (br s, 1H), 7.76 (br d, *J* = 8.1 Hz, 2H), 7.59-7.52 (m, 5H), 7.47 (br s, 1H), 7.39 (t, *J* = 7.9 Hz, 1H), 7.29 (br d, *J* = 8.1 Hz, 1H), 5.57 (s, 2H). ¹⁹F NMR (377 MHz, CDCl₃) δ -62.71 (s, 3F). LCMS (ES⁺) *m*/*z* calculated for C₂₂H₁₆F₃N₅O₂ 439.13; found 438 (M-H)⁻. HPLC *t*_{R} 1.35 min.

### Example 42: (3-(4-(pyrazin-2-yl)benzyl)-1,2,3-oxadiazol-3-ium-5-yl)((3-(trifluoromethyl) phenyl)carbamoyl)amide.

The title compound was obtained as a white solid reacting 2-iodopyrazine in Step 2. ¹H NMR (400 MHz, DMSO-*d*₆) δ 9.72 (s, 1H), 9.30 (s, 1H), 8.75 (s, 1H), 8.65 (br d, *J* = 2.4 Hz, 1H), 8.26-8.21 (m, 4H), 7.76-7.71 (m, 3H), 7.44 (t, *J* = 7.9 Hz, 1H), 7.22 (br d, *J* = 7.5 Hz, 1H), 5.88 (s, 2H). ¹⁹F NMR (377 MHz, DMSO-*d*₆) δ -61.28 (s, 3F). LCMS (ES⁺) *m*/*z* calculated for C₂₁H₁₅F₃N₆O₂ 440.12; found 439 (M-H)⁻. HPLC *t*_{R} 1.76 min.

### Example 61: (3-(4-(pyridazin-4-yl)benzyl)-1,2,3-oxadiazol-3-ium-5-yl)((3-(trifluoromethyl) phenyl)carbamoyl)amide.

The title compound was obtained as a white solid reacting 4-bromopyridazine in Step 2. ¹H NMR (400 MHz, DMSO-*d*₆) δ 9.73 (s, 1H), 9.67 (s, 1H), 9.31 (br d, *J* = 5.5 Hz, 1H), 8.27 (s, 1H), 8.21 (s, 1H), 8.06-8.02 (m, 3H), 7.78 (br d, *J* = 8.1 Hz, 2H), 7.72 (br d, *J* = 8.1 Hz, 1H), 7.44 (t, *J* = 7.9 Hz, 1H), 7.22 (br d, *J* = 7.9 Hz, 1H), 5.88 (s, 2H). ¹⁹F NMR (377 MHz, DMSO-*d*₆) δ -61.28 (s, 3F). LCMS (ES⁺) *m*/*z* calculated for C₂₁H₁₅F₃N₆O₂ 440.12; found 439 (M-H)⁻. HPLC *t*_{R} 1.54 min.

### Example 62: (3-(4-(4-methoxypyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-methoxypyrimidine in Step 2. ¹H NMR (400 MHz, DMSO-*d*₆) δ 9.66 (s, 1H), 8.74 (s, 1H), 8.55 (s, 1H), 8.18 (s, 1H), 8.14 (br s, 1H), 7.66-7.59 (m, 5H), 7.37 (br t, *J* = 8.1 Hz, 1H), 7.15 (br d, *J* = 7.7 Hz, 1H), 5.77 (s, 2H), 3.90 (s, 3H). ¹⁹F NMR (377 MHz, DMSO-*d*₆) δ -61.28 (s, 3F). LCMS (ES⁺) *m*/*z* calculated for C₂₂H₁₇F₃N₆O₃ 470.13; found 469 (M-H)⁻. HPLC *t*_{R} 1.79 min.

### Example 63: (3-(4-(2-methoxypyridin-4-yl)benzyl)-1,2,3-oxadiazol-3-ium-5-yl)((3-(trifluoromethyl)phenyl)carbamoyl)amide.

The title compound was obtained as a light-yellow solid reacting 4-bromo-2-methoxypyridine in Step 2. ¹H NMR (400 MHz, DMSO-*d*₆) δ 9.65 (s, 1H), 8.18 (s, 1H), 8.17 (s, 1H), 8.14 (br s, 1H), 7.80 (br d, *J* = 8.3 Hz, 2H), 7.65-7.62 (m, 3H), 7.36 (br t, *J* = 8.0 Hz, 1H), 7.26 (dd, *J₁* = 5.5 Hz, *J₂* = 1.5 Hz, 1H), 7.15 (br d, *J* = 7.7 Hz, 1H), 7.07 (s, 1H), 5.78 (s, 2H), 3.83 (s, 3H). ¹⁹F NMR (377 MHz, DMSO-*d*₆) δ -61.28 (s, 3F). LCMS (ES⁺) *m*/*z* calculated for C₂₃H₁₈F₃N₅O₃ 469.14; found 468 (M-H)⁻. HPLC *t*_{R} 1.98 min.

### Example 64: (3-(4-(2-hydroxypyridin-4-yl)benzyl)-1,2,3-oxadiazol-3-ium-5-yl)((3-(trifluoromethyl)phenyl)carbamoyl)amide.

The title compound was obtained as a white solid reacting 4-bromopyridin-2-ol in Step 2. ¹H NMR (400 MHz, DMSO-*d*₆) δ 11.71 (br s, 1H), 9.80 (s, 1H), 8.33 (s, 1H), 8.28 (br s, 1H), 7.86 (br d, *J* = 7.9 Hz, 2H), 7.82-7.74 (m, 3H), 7.55-7.49 (m, 2H), 7.30 (br d, *J* = 7.5 Hz, 1H), 6.69 (br s, 1H), 6.59 (d, *J* = 6.8 Hz, 1H), 5.92 (s, 2H). ¹⁹F NMR (377 MHz, DMSO-*d*₆) δ -61.28 (s, 3F). LCMS (ES⁺) *m*/*z* calculated for C₂₂H₁₆F₃N₅O₃ 455.12; found 454 (M-H)⁻. HPLC *t*_{R} 1.47 min.

### Example 79: (S)-(3-(1-(4-(4-methylpyrimidin-5-yl)phenyl)ethyl)-1,2,3-oxadiazol-3-ium-5-yl)((3-(trifluoromethyl)phenyl)carbamoyl)amide.

The title compound was obtained as a white solid reacting (*S*)-(3-(1-(4-(4,4,5,5-tetramethyl-1,3,2-dioxaborolan-2-yl)phenyl)ethyl)-1,2,3-oxadiazol-3-ium-5-yl)((3-(trifluoromethyl)phenyl)carbamoyl)amide and 5-bromo-4-methylpyrimidine in Step 2. LCMS (ES⁺) *m*/*z* calculated for C₂₃H₁₉F₃N₆O₂ 468.15; found 467 (M-H)⁻. HPLC *t*_{R} 1.75 min.

### Example 105: (3-(4-(1,2-dimethyl-1H-imidazol-5-yl)benzyl)-1,2,3-oxadiazol-3-ium-5-yl)((3-(trifluoromethyl)phenyl)carbamoyl)amide.

The title compound was obtained as a beige solid reacting 5-bromo-1,2-dimethyl-1*H*-imidazole in Step 2. ¹H NMR (400 MHz, DMSO-*d*₆) δ 9.73 (s, 1H), 8.25 (s, 1H), 8.21 (br s, 1H), 7.73-7.64 (m, 3H), 7.52 (br d, *J* = 7.9 Hz, 2H), 7.46-7.42 (m, 1H), 7.23-7.21 (m, 1H), 6.93 (s, 1H), 5.82 (s, 2H), 3.54 (s, 3H), 2.35 (s, 3H). ¹⁹F NMR (377 MHz, DMSO-*d*₆) δ -61.28 (s, 3F). LCMS (ES⁺) *m*/*z* calculated for C₂₂H₉F₃N₆O₂ 456.15; found 455 (M-H)⁻. HPLC *t*_{R} 1.31 min.

### Example 106: (3-(4-(4-cyanopyrimidin-5-yl)benzyl)-1,2,3-oxadiazol-3-ium-5-yl)((3-(trifluoromethyl)phenyl)carbamoyl)amide.

The title compound was obtained as a beige solid reacting 5-bromopyrimidine-4-carbonitrile in Step 2. ¹⁹F NMR (377 MHz, DMSO-*d*₆) δ -61.28 (s, 3F). LCMS (ES⁺) *m*/*z* calculated for C₂₂H₁₄F₃N₇O₂ 465.12; found 466 (M+H)⁺. HPLC *t*_{R} 1.84 min.

### Example 99: (R)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide formate

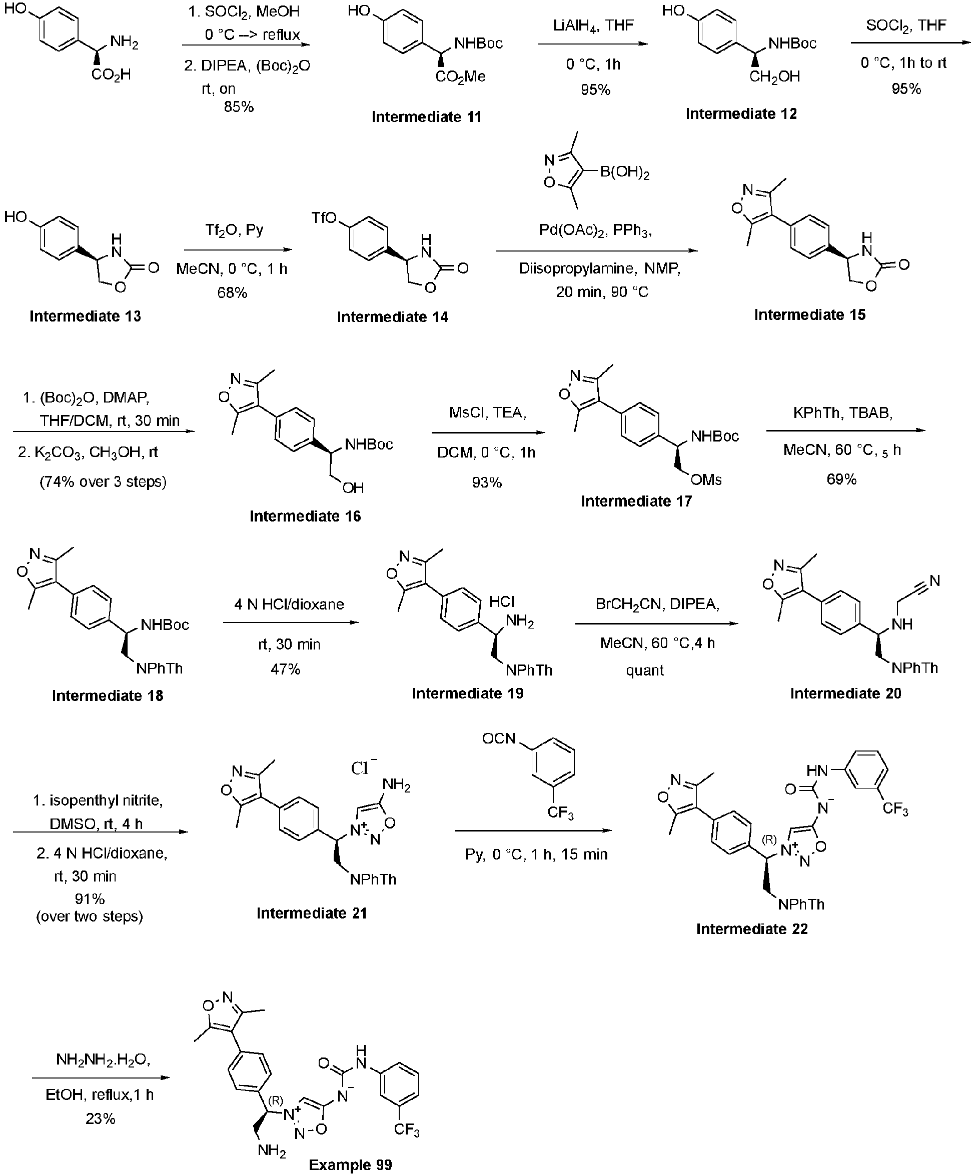

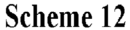

### Step 1: Methyl (R)-2-((tert-butoxycarbonyl)amino)-2-(4-hydroxyphenyl)acetate (Intermediate 11).

To a suspension of (-)-4-hydroxy-*D*-phenylglycine (5 g, 29.91 mmol) in methanol (30 mL) at 0 °C, thionyl chloride (2.17 mL, 29.91 mmol) was added dropwise. The reaction mixture was stirred at reflux for 18 h, then cooled to rt and used as such. DIPEA (13 mL, 59.8 mmol) was then added dropwise followed by a solution of di-*tert*-butyl dicarbonate (7.18 g, 32.9 mmol) in methanol (12 mL) added dropwise over 40 min. The reaction mixture was stirred at rt overnight. Solvent was reduced to half the original volume and the resulting solution was poured into water (100 mL), forming a white precipitate that was collected by filtration. The crude was dried under vacuum pump overnight to afford the title compound as a white solid (7.15 g, 85%). ¹H NMR (400 MHz, DMSO-*d*₆) δ 9.49 (s, 1H), 7.82 (d, *J* = 8.5 Hz, 2H), 7.61 (d, *J* = 7.8 Hz, 1H), 6.71 (d, *J* = 8.4 Hz, 2H), 5.06 (d, *J* = 7.8 Hz, 1H), 3.59 (s, 3H), 1.38 (s, 9H). LCMS (ES⁺) *m*/*z* calculated for C₁₄H₁₉NO₅ 281.13; found 282 (M+H)⁺. HPLC *t*_{R} 1.62 min.

### Step 2: tert-Butyl (R)-(2-hydroxy-1-(4-hydroxyphenyl)ethyl)carbamate (Intermediate 12).

A solution of Intermediate **11** (1.2 g, 4.27 mmol) in anhydrous THF (30 mL) was cooled to 5 °C. 1 M Lithium aluminium hydride in THF (12.8 mL, 12.8 mmol) was added by dropwise slowly over 40 min, maintaining an internal reaction temperature of less than 15 °C. The stirring was continued for 30 min, and then the reaction was carefully quenched with NH₄Cl sat. sol. (2 mL) over 45 min, followed by 0.3 N HCl (2 mL). The resulting suspension was then filtered and rinsed with ethyl acetate, 1 N HCl was added to the filtrate to adjust the aqueous layer to pH = 1. The organic extract was rinsed with water, brine, and dried over MgSO₄. The aqueous phase was extracted additionally with EtOAc and the organic phase was treated as previously. The organic layers were combined and concentrated to give the title compound as a yellowish solid (927 mg, 95%). ¹H NMR (300 MHz, DMSO-*d*₆) δ 9.21 (s, 1H), 7.06 (m, 3H), 6.67 (d, *J* = 8.4 Hz, 2H), 4.68 (t, *J* = 5.7 Hz, 1H), 4.41 (q, *J* = 7.2 Hz, 1H), 3.42 (t, *J* = 6.3 Hz, 2H), 1.36 (s, 9H). LCMS (ES⁺) *m*/*z* calculated for C₁₃H₁₉NO₄ 253.13; found 254 (M+H)⁺. HPLC *t*_{R} 1.26 min.

### Step 3: (R)-4-(4-Hydroxyphenyl)oxazolidin-2-one (Intermediate 13).

A solution Intermediate **12** (927 mg, 3.66 mmol) in THF (11 mL) was cooled to 0 °C, thionyl chloride (0.29 mL, 4.03 mmol) was added dropwise. After the addition the reaction mixture was stirred at rt overnight. Then, the reaction mixture was partially concentrated under reduced pressure. The residual slurry was stirred at rt and MTBE (10 mL) was added. The product started to crystallize out. The slurry was stirred for 15 min and filtered. The filter cake was washed with MTBE (5 × 5 mL). The solid was dried to afford the title compound as a red-brown powder (0.63 g, 95%). ¹H NMR (400 MHz, DMSO-*d*₆) δ 9.48 (s, 1H), 8.05 (s, 1H), 7.12 (d, *J* = 8.4 Hz, 2H), 6.76 (d, *J* = 8.8 Hz, 2H), 4.81 (t, *J* = 8 Hz, 1H), 4.59 (t, *J* = 8 Hz, 1H), 3.93 (dd, *J*₁ = 8 Hz, *J*₂ = 12 Hz, 1H). LCMS (ES⁺) *m*/*z* calculated for C₉H₉NO₃ 179.06; found 180 (M+H)⁺. HPLC *t*_{R} 0.81 min.

### Step 4: (R)-4-(2-Oxooxazolidin-4-yl)phenyl trifluoromethanesulfonate (Intermediate 14).

To a slurry of Intermediate **13** (676 mg, 3.77 mmol) in acetonitrile (7.5 mL) was added dry pyridine (0.85 mL, 11.32 mmol). The mixture was stirred under a nitrogen atmosphere and trifluoromethanesulfonic anhydride (0.76 mL, 4.53 mmol) was added slowly maintaining the reaction temperature at 20 °C. The reaction mixture was stirred at the same temperature for 30 min, followed by 30 min at rt. The solvent was removed under reduced pressure and the resulting oil was taken with DCM. The organic phase was washed with sat. sol. NaHCO₃, brine and dried over Na₂SO₄, then concentrated under reduced pressure to give a dark red oil. The crude product was purified by chromatography on silica gel (eluent gradient from 100% DCM to 15% DCM/EtOAc) to yield the title compound (0.741 mg, 68%). ¹H NMR (300 MHz, DMSO-*d*₆) δ 8.24 (s, 1H), 7.56 (s, 4H), 5.03 (dd, *J* = 8.2, 6.9 Hz, 1H), 4.69 (t, *J* = 8.7 Hz, 1H), 4.04 (dd, *J* = 8.6, 6.3 Hz, 1H). LCMS (ES⁺) *m*/*z* calculated for C₁₀H₈F₃NO₅S 311.01; found 312 (M+H)⁺. HPLC *t*_{R} 1.70 min.

### Step 5: (R)-4-(4-(3,5-Dimethylisoxazol-4-yl)phenyl)oxazolidin-2-one (Intermediate 15).

A pressure vessel was charged with Intermediate **14** (220.5 mg, 0.71 mmol) and (3,5-dimethyl-1,2-oxazol-4-yl)boronic acid (149.8 mg, 1.06 mmol) in NMP (1 mL). Then, triphenylphosphane (29.7 mg, 0.11 mmol), palladium(II) acetate (0.69 mL, 0.02 mmol) and DIPEA (0.1 mL, 0.74 mmol) were added. The reaction mixture was refluxed in a closed vessel at 90 °C for 30 min. The cooled reaction mixture was diluted with DCM and the organic layer was washed with water, 5% citric acid, 3 N HCl and brine. The organic phase was dried over Na₂SO₄, filtered and concentrated. The crude was purified by chromatography on silica gel (eluent DCM/MeOH from 100% DCM to 9/1 DCM/MeOH) to give the title compound as a yellow solid (278 mg) that was used as such in the next synthetic step. ¹H NMR (300 MHz, DMSO-*d*₆) δ 8.21 (s, 1H), 7.49-7.41 (m, 4H), 5.00 (dd, *J* = 8.4, 6.6 Hz, 1H), 4.70 (t, *J* = 8.6 Hz, 1H), 4.08 (dd, *J* = 8.5, 6.4 Hz, 1H), 2.40 (s, 3H), 2.23 (s, 3H). LCMS (ES⁺) *m*/*z* calculated for C₁₄H₁₄N₂O₃ 258.10; found 259 (M+H)⁺. HPLC *t*_{R} 1.41 min.

### Step 6: tert-butyl (R)-(1-(4-(3,5-dimethylisoxazol-4 yl)phenyl)-2-hydrozyethyl)carbamate (Intermediate 16).

A mixture of Intermediate **15** (278 mg, 1.08 mmol), di-*tert*-butyl dicarbonate (470 mg, 2.15 mmol) and DMAP (39.5 mg, 0.32 mmol) in DCM/THF (1:1) (4 mL) was stirred at rt for 30 min. The reaction mixture was diluted with DCM (30 mL) and the organic phase was washed with water, brine and dried over Na₂SO₄. The organic phase was concentrated under reduced pressure to yield crude *tert*-butyl (*R*)-4-(4-(3,5-dimethylisoxazol-4-yl)phenyl)-2-oxooxazolidine-3-carboxylate as a light-yellow foam (357 mg). LCMS (ES⁺) *m*/*z* calculated for C₁₉H₂₂N₂O₅ 358.15, found 359 (M+H)⁺. HPLC *t*_{R} 1.86 min. A mixture of this crude and potassium carbonate (298 mg, 2.16 mmol) in MeOH (3 mL) was sonicated for 5 min and then stirred at rt for 1 h. The reaction mixture was diluted with EtOAc and washed with small amounts of water. The combined aqueous layer was extracted with EtOAc. The organic layers were combined and washed with brine and evaporated to give the title compound (327 mg, 74% over three steps). ¹H NMR (300 MHz, DMSO-*d*₆) δ 7.50-7.18 (m, 5H), 4.83 (br s, 1H), 4.58 (m, 1H), 3.53 (br d, *J* = 3.4 Hz, 2H), 2.40 (s, 3H), 2.23 (s, 3H), 1.46-1.30 (m, 9H). LCMS (ES⁺) *m*/*z* calculated for C₁₈H₂₄N₂O₄ 332.17; found 333 (M+H)⁺. HPLC *t*_{R} 1.72 min.

### Step 7: (R)-2-((tert-Butoxycarbonyl)amino)-2-(4-(3,5-dimethylisoxazol-4-yl)phenyl)ethyl methanesulfonate (Intermediate 17).

To a solution of Intermediate **16** (327 mg, 0.89 mmol) and TEA (0.18 mL, 1.33 mmol) in DCM (3 mL) at 0 °C was added dropwise a solution of methanesulfonyl chloride (0.082 mL. 1.07 mmol) in DCM (1 mL) over 5 min and then the mixture was stirred for 30 min at 0 °C. The reaction mixture was washed with water, sat. sol. KHSO₄ and brine. The organic layer was dried over Na₂SO₄ and concentrated under reduced pressure to the title compound (341 mg, 93%) that was used as such in the next step. LCMS (ES⁺) *m*/*z* calculated for C₁₉H₂₆N₂O₆S 410.15; found 411 (M+H)⁺. HPLC *t*_{R} 1.93 min.

### Step 8: tert-butyl (R)-(1-(4-(3,5-dimethylisoxazol-4 yl)phenyl)-2-(1,3-dioxoisoindolin-2-yl)ethyl)carbamate (Intermediate 18).

In a flask were added Intermediate **17** (340 mg, 0.828 mmol) in DMF (0.25 M), potassium 1,3-dioxoisoindolin-2-ide (153 mg, 0.83 mmol) and TBAB (5.3 mg, 0.017 mmol) were added. The reaction was stirred at 60 °C for 4 h. Then water was added (10 mL) and the precipitated product was filtered, washed additionally with water and dried under vacuum to give the title compound as a light orange powder (264 mg, 69%). The crude was used as such in the next synthetic step. ¹H NMR (300 MHz, DMSO-*d*₆) δ 7.98-7.83 (m, 5H), 7.56-7.37 (m, 4H), 5.16-4.97 (m, 1H), 4.07-3.77 (m, 2H), 2.43 (s, 3H), 2.25 (s, 3H), 1.27 (s, 9H). LCMS (ES⁺) *m*/*z* calculated for C₂₆H₂₇N₃O₅ 461.20; found 462 (M+H)⁺. HPLC *t*_{R} 2.16 min.

### Step 9: (R)-2-(2-amino-2-(4-(3,5-dimethylisoxazol-4-yl)phenyl)ethyl)isoindoline-1,3-dione hydrochloride (Intermediate 19).

Intermediate **18** (260 mg, 0.572 mmol) was treated directly at rt with 4 N HCl in dioxane (1.2 mL). The reaction mixture was stirred at rt overnight. The resulting suspension was diluted with DCM (10 mL), sonicated, and filtered to give the title compound as light pink powder (108 mg, 47%). The crude was used as such in the next step. ¹H NMR (300 MHz, DMSO-*d*₆) δ 8.69 (br s, 3 H), 8.02-7.80 (m, 4H), 7.65 (d, *J* = 8.3 Hz, 2H), 7.48 (d, *J* = 8.3 Hz, 2H), 4.67 (br dd, *J* = 7.9, 5.7 Hz, 1H), 4.23-4.04 (m, 1 H), 4.02-3.87 (m, 1H), 2.39 (s, 3H), 2.21 (s, 3H). LCMS (ES⁺) *m*/*z* calculated for C₂₁H₁₉N₃O₃ 361.14; found 362 (M+H)⁺. HPLC *t*_{R} 1.26 min.

### Step 10: (R)-2-((1-(4-(3,5-dimethylisoxazol-4 yl)phenyl)-2-(1,3-dioxoisoindolin-2-yl)ethyl)amino)acetonitrile (Intermediate 20).

To a suspension of Intermediate **19** (108 mg, 1.08 mmol) and DIPEA (0.18 mL, 1.03 mmol) in MeCN (2 mL), 2-bromoacetonitrile (0.038 mL, 0.54 mmol) was added. The reaction mixture was heated at 60 °C for 3 h and then it was concentrated under reduced pressure. The obtained oil was treated with small amount of water and extracted with EtOAc. The combined organic phase was washed additionally with water (3 × 3 mL) and brine, then dried over Na₂SO₄ and concentrated under reduced pressure to obtain the title compound (117 mg, 100%). ¹H NMR (300 MHz, DMSO-*d*₆) δ 7.91-7.80 (m, 4H), 7.47-7.38 (m, 2H), 7.38-7.26 (m, 2H), 4.27-4.11 (m, 1H), 3.94-3.78 (m, 1H), 3.75-3.55 (m, 2H), 3.45-3.36 (m, 1H), 3.27-3.17 (m, 1H), 2.37 (s, 3H), 2.19 (s, 3H). LCMS (ES⁺) *m*/*z* calculated for C₂₃H₂₀N₄O₃ 400.15; found 401 (M+H)⁺. HPLC *t*_{R} 1.85 min.

### Step 11: (R)-5-amino-3-(1-(4-(3,5-dimethylisoxazol-4-yl)phenyl)-2-(1,3-dioxoisoindolin-2-yl)ethyl)-1,2,3-oxadiazol-3-ium chloride (Intermediate 21)

Intermediate **20** (0.12 g, 0.29 mmol) was dissolved in DMSO (0.2 mL) and isopentyl nitrite (0.12 mL, 0.88 mmol) as added at rt. The reaction mixture was stirred at rt for 4 h. The reaction mixture was diluted with water and extracted with DCM. The organic layer was washed with brine, dried over Na₂SO₄ and concentrated under reduced pressure to give (*R*)-*N*-(cyanomethyl)-*N*-(1-(4-(3,5-dimethylisoxazol-4-yl)phenyl)-2-(1,3-dioxoisoindolin-2-yl)ethyl)nitrous amide as a yellow oil that was used directly in the next synthetic step. LCMS (ES⁺) *m*/*z* calculated for C₂₃H₁₉N₅O 429.14, found 430 (M+H)⁺. HPLC *t*_{R} 1.99 min. The crude was treated directly with 4 N HCl (1.2 mL), allowed without stirring at rt for 4 h. The mixture was concentrated under reduced pressure and stripped several times with DCM and diethyl ether until a solid product was obtained. The crude was triturated with diethyl ether to give the title compound as a beige powder (125 mg, 91%) that was used as such in the next step. LCMS (ES⁺) *m*/*z* calculated for C₂₃H₂₀N₅O₄ 430.15; found 430 (M+H)⁺. HPLC *t*_{R} 1.41 min.

### Step 12: (R)-(3-(1-(4-(3,5-dimethylisoxazol-4-yl)phenyl)-2-(1,3-dioxoisoindolin-2-yl)ethyl)-1,2,3-oxadiazol-3-ium-5-yl)((3-(trifluoromethyl)phenyl)carbamoyl)amide (Intermediate 22).

A cold suspension (ice bath) of Intermediate **21** (40.99 mg, 0.08 mmol) in pyridine (1.5 mL) was treated directly with 1-isocyanato-3-(trifluoromethyl)benzene (0.05 mL, 0.39 mmol). After 10 min the reaction was quenched at 0 °C with water and the suspension was extracted with diethyl ether. The organic phase was washed additionally with water, sat. sol. KHSO₄ and brine, then was dried over Na₂SO₄, concentrated and stripped with DCM under reduced pressure to give the title compound as a solid (138 mg, 0.22 mmol) that was used as such in the next synthetic step. LCMS (ES⁺) *m*/*z* calculated for C₃₁H₂₃F₃N₆O₅ 616.17; found 617 (M+H)⁺. HPLC *t*_{R} 2.21 min.

*Step 13: (R)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide formate (**Example 99**)*

A solution of Intermediate **22** (0.086 g, 0.14 mmol) in ethanol (6 mL) was treated with hydrazine hydrate (0.14 mL, 2.8 mmol) at rt. The reaction was stirred at 65 °C for 1 h. The reaction was cooled to rt and a white precipitate was formed. The reaction was diluted with ethanol (5 mL) and the precipitate was filtered. The solution was concentrated under reduced pressure to give a light-yellow solid. The title compound was obtained after purification by chromatography on silica gel column using water DCM/MeOH 1% eluents. Fractions containing product were dried under reduced pressure and then lyophilized to give the title compound as a white solid (17.4 mg, 23%). ¹H NMR (300 MHz, DMSO-*d*₆) δ 9.77 (s, 1H), 8.43 (s, 1H), 8.36-8.25 (m, 1H), 7.81-7.70 (m, 3H), 7.61-7.44 (m, 3H), 7.27 (d, *J* = 7.7 Hz, 1H), 5.94 (dd, *J* = 9.9, 4.6 Hz, 1H), 3.79 (dd, *J* = 13.8, 10.0 Hz, 1H), 3.46-3.39 (m, 1H), 2.46 (s, 3H), 2.29 (s, 3H), 1.96 (br s, 2H). LCMS (ES⁺) *m*/*z* calculated for C₂₃H₂₁F₃N₆O₃ 486.17; found 487 (M+H)⁺. HPLC *t*_{R} 2.73 min.

**Examples 49, 54, 65, 66, 102 and 116** were synthesized according to the above procedure (Scheme 12) by reacting with the appropriate starting materials.

### Example 49: (R)-(3-(1-amino-3-phenylpropan-2-yl)-1,2,3-oxadiazol-3-ium-5-yl)((3-(trifluoromethyl)phenyl)carbamoyl)amide formate.

The title compound was obtained as a white solid starting from Boc-D-Phenylalaninol following Scheme 12 (step 7-13). ¹H NMR (300 MHz, DMSO-*d*₆) δ 9.76 (s, 1H), 8.58 (s, 1H), 8.16 (br s, 3H), 7.69 (br d, *J* = 8.4 Hz, 1H), 7.46 (t, *J* = 7.9 Hz, 1H), 7.39-7.12 (m, 7H), 5.27-5.06 (m, 1H), 3.82-3.64 (m, 1H), 3.58-3.31 (m, 3H). LCMS (ES⁺) *m*/*z* calculated for C₁₉H₁₈F₃N₅O₂ 405.14; found 406 (M+H)⁺. HPLC *t*_{R} 2.66 min.

### Example 54: (S)-(3-(1-amino-3-phenylpropan-2-yl)-1,2,3-oxadiazol-3-ium-5-yl)((3-(trifluoromethyl)phenyl)carbamoyl)amide formate.

The title compound was obtained as a white solid starting from Boc-*L*-Phenylalaninol following Scheme 12 (step 7-13). ¹H NMR (300 MHz, DMSO-*d*₆) δ 9.76 (s, 1H), 8.58 (s, 1H), 8.16 (br s, 3H), 7.69 (br d, *J* = 8.4 Hz, 1H), 7.46 (t, *J* = 7.9 Hz, 1H), 7.37-7.10 (m, 7H), 5.27-5.06 (m, 1H), 3.82-3.64 (m, 1H), 3.56-3.28 (m, 3H). LCMS (ES⁺) *m*/*z* calculated for C₁₉H₁₈F₃N₅O₂ 405.14; found 406 (M+H)⁺. HPLC *t*_{R} 2.67 min.

### Example 65: (S)-(3-(1-([1,1'-biphenyl]-4-yl)-3-aminopropan-2-yl)-1,2,3-oxadiazol-3-ium-5-yl)((3-(trifluoromethyl)phenyl)carbamoyl)amide formate.

The title compound was obtained as a white solid starting from (*S*)-*tert*-butyl (1-([1,1'-biphenyl]-4-yl)-3-hydroxypropan-2-yl)carbamate following Scheme 12 (step 7-13). ¹H NMR (300 MHz, DMSO-*d*₆) δ 10.21 (br s, 1H), 8.92 (br s, 1H), 8.26 (s, 1H), 8.21-8.05 (m, 4H), 7.76-7.57 (m, 5H), 7.53-7.40 (m, 3H), 7.38-7.25 (m, 4H), 5.37 (br d, *J* = 6.6 Hz, 1H), 3.92-3.63 (m, 1H), 3.63-3.35 (m, 3H). LCMS (ES⁺) *m*/*z* calculated for C₂₅H₂₂F₃N₅O₂ 481.17. found 482 (M+H)⁺. HPLC *t*_{R} 3.07 min.

### Example 66: (R)-(3-(1-([1,1'-biphenyl]-4-yl)-3-aminopropan-2-yl)-1,2,3-oxadiazol-3-ium-5-yl)((3-(trifluoromethyl)phenyl)carbamoyl)amide.

The title compound was obtained as a white solid starting from (*R*)-tert-butyl (1-([1,1'-biphenyl]-4-yl)-3-hydroxypropan-2-yl)carbamate following Scheme 12 (step 7-13). ¹H NMR (300 MHz, DMSO-*d*₆) δ 10.23 (s, 1 H), 8.94 (s, 1H), 8.26 (s, 1H), 8.20-8.04 (m, 3H), 7.74-7.58 (m, 5H), 7.55-7.39 (m, 3 H), 7.37-7.26 (m, 4H), 5.38 (q, *J* = 6.9 Hz, 1H), 3.91-3.66 (m, 1H), 3.61-3.32 (m, 3H). LCMS (ES⁺) *m*/*z* calculated for C₂₅H₂₂F₃N₅O₂ 481.17; found 482 (M+H)⁺. HPLC *t*_{R} 3.08 min.

### Example 102: (R)-(3-(2-amino-1-(4-(pyrimidin-5-yl)phenyl)ethyl)-1,2,3-oxadiazol-3-ium-5-yl)((3-(trifluoromethyl)phenyl)carbamoyl)amide formate.

The title compound was obtained as a white solid following Scheme 12 and using pyrimidin-5-ylboronic acid in step 5. ¹H NMR (300 MHz, DMSO-*d*₆) δ 9.71 (s, 1H), 9.22 (s, 1H), 9.16 (s, 2H), 8.38 (s, 1H), 8.30-8.19 (m, 1H), 7.91 (d, *J* = 8.3 Hz, 2H), 7.77 (d, *J* = 8.3 Hz, 2H), 7.72-7.63 (m, 1H), 7.43 (br t, *J* = 7.9 Hz, 1H), 7.22 (br d, *J* = 8.2 Hz, 1H), 5.92 (dd, *J* = 9.72, 4.58 Hz, 1H), 3.83-3.66 (m, 1H), 3.42-3.36 (m, 1H). LCMS (ES⁺) *m*/*z* calculated for C₂₂H₁₈F₃N₇O₂ 469.43; found 470 (M+H)⁺. HPLC *t*_{R} 3.08 min.

### Example 116: (S)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide formate.

The title compound was obtained as a white solid starting from 4-hydroxy-*L*-phenylglycine and following Scheme 12. ¹H NMR (300 MHz, DMSO-*d*₆) δ 9.77 (s, 1H), 8.43 (s, 1H), 8.36-8.25 (m, 1H), 7.81-7.70 (m, 3H), 7.61-7.44 (m, 3H), 7.27 (d, *J* = 7.7 Hz, 1H), 5.94 (dd, *J* = 9.9, 4.6 Hz, 1H), 3.79 (dd, *J* = 13.8, 10.0 Hz, 1H), 3.46-3.39 (m, 1H), 2.46 (s, 3H), 2.29 (s, 3H), 1.96 (br s, 2H). LCMS (ES⁺) *m*/*z* calculated for C₂₃H₂₁F₃N₆O₃ 486.16; found 487 (M+H)⁺. HPLC *t*_{R} 2.73 min.

### Example 88: (3-(4-(3,5-Dimethylisoxazol-4-yl)-3-methylbenzyl)-1,2,3-oxadiazol-3-ium-5-yl)((3-(trifluoromethyl)phenyl)carbamoyl)amide

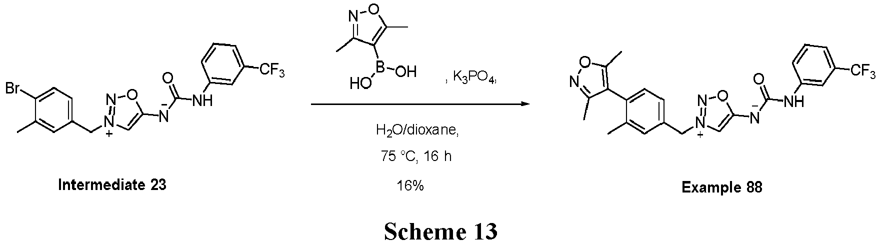

A solution of (3-(4-bromo-3-methylbenzyl)-1,2,3-oxadiazol-3-ium-5-yl)((3-(trifluoromethyl)phenyl)carbamoyl)amide (Intermediate **23**) (prepared as described in the synthesis of **Example 6**) (30.0 mg, 0.07 mmol), tripotassium phosphate (42.0 mg, 0.20 mmol), bis(chloranyl)palladium cyclopentyl(diphenyl)phosphane ferrocene (5.5 mg, 0.01 mmol) and (3,5-dimethylisoxazol-4-yl)boronic acid (13.0 mg, 0.09 mmol) in a mixture of H₂O/dioxane (1:10)

(1.1 mL) was heated at 75 °C for 16 h. The reaction mixture was cooled at rt and filtered on a pad of solca flock. The excess of solvent was removed under reduced pressure to get a residue which was purified by chromatography on silica gel (eluting with 0-100% EtOAc in petroleum ether) to give the title compound (5 mg, 16%) as a white solid. ¹H NMR (400 MHz, DMSO-*d*₆) δ 9.72 (br s, 1H), 8.27 (s, 1H), 8.21 (s, 1H), 7.71 (br d, *J* = 8.3 Hz, 1H), 7.54 (s, 1H), 7.47-7.42 (m, 2H), 7.27-7.21 (m, 2H), 5.79 (s, 2H), 2.20 (s, 3H), 2.12 (s, 3H), 2.01 (s, 3H). ¹⁹F NMR (377 MHz, DMSO-*d*₆) δ -61.28 (s, 3F). LCMS (ES⁺) *m*/*z* calculated for C₂₃H₂₀F₃N₅O₃ 471.15; found 470 (M-H)⁻. HPLC *t*_{R} 2.03 min.

**Examples 87, 100, 103, 104, 108, 109, 110, 117, 119, 120, 121 and 124** were synthesized according to the above procedure (Scheme 13) by reacting with the appropriate starting materials.

### Example 87: (3-(3-methyl-4-(pyrimidin-5-yl)benzyl)-1,2,3-oxadiazol-3-ium-5-yl)((3-(trifluoromethyl)phenyl)carbamoyl)amide.

The title compound was obtained as a white solid reacting pyrimidin-5-ylboronic acid in Step 1. ¹H NMR (400 MHz, DMSO-*d*₆) δ 9.73 (s, 1H), 9.23 (s, 1H), 8.89 (s, 2H), 8.24 (s, 1H), 8.21 (br s, 1H), 7.72 (br d, *J* = 8.6 Hz, 1H), 7.59 (s, 1H), 7.55 (br d, *J=* 7.7 Hz, 1H), 7.46-7.42 (m, 2H), 7.23 (br d, *J* = 7.7 Hz, 1H), 5.82 (s, 2H), 2.30 (s, 3H). ¹⁹F NMR (377 MHz, DMSO-*d*₆) δ -61.28 (s, 3F). LCMS (ES⁺) *m*/*z* calculated for C₂₂H₁₇F₃N₆O₂ 454.14; found 453 (M-H)⁻. HPLC *t*_{R} 1.78 min.

### Example 100: (3-((5-(pyrimidin-5-yl)thiophen-3-yl)methyl)-1,2,3-oxadiazol-3-ium-5-yl)((3-(trifluoromethyl)phenyl)carbamoyl)amide.

The title compound was obtained as a white solid reacting pyrimidin-5-ylboronic acid and (3-((5-bromothiophen-3-yl)methyl)-1,2,3-oxadiazol-3-ium-5-yl)((3-(trifluoromethyl)phenyl)carbamoyl)amide (prepared as reported in the synthesis of Example **6**) in Step 1. ¹H NMR (400 MHz, DMSO-*d*₆) δ 9.49 (br s, 1H), 8.91 (s, 1H), 8.89 (s, 2H), 7.98 (s, 2H), 7.82 (br s, 1H), 7.66 (s, 1H), 7.49 (br d, *J* = 7.9 Hz, 1H), 7.20 (br t, *J* = 7.7 Hz, 1H), 6.99 (br d, *J* = 7.2 Hz, 1H), 5.61 (s, 2H). ¹⁹F NMR (377 MHz, DMSO-*d*₆) δ -61.28 (s, 3F). LCMS (ES⁺) *m*/*z* calculated for C₁₉H₁₃F₃N₆O₂S 446.08; found 445 (M-H)⁻. HPLC *t*_{R} 1.65 min.

### Example 103: (3-((6-(pyrimidin-5-yl)pyridin-3-yl)methyl)-1,2,3-oxadiazol-3-ium-5-yl)((3-(trifluoromethyl)phenyl)carbamoyl)amide.

The title compound was obtained as a pale yellow solid reacting pyrimidin-5-ylboronic acid and (3-((6-bromopyridin-3-yl)methyl)-1,2,3-oxadiazol-3-ium-5-yl)((3-(trifluoromethyl)phenyl)carbamoyl)amide (prepared as reported in the synthesis of Example **6**) in Step 1. ¹H NMR (400 MHz, DMSO-*d*₆) δ 9.73 (br s, 1H), 9.47 (s, 2H), 9.28 (s, 1H), 8.97 (br s, 1H), 8.33 (br s, 1H), 8.26-8.19 (m, 3H), 7.72 (br d, *J* = 8.3 Hz, 1H), 7.44 (br t, *J* = 7.9 Hz, 1H), 7.22 (br d, *J* = 7.7 Hz, 1H), 5.93 (s, 2H). ¹⁹F NMR (377 MHz, DMSO-*d*₆) δ -61.29 (s, 3F). LCMS (ES⁺) *m*/*z* calculated for C₂₀H₁₄F₃N₇O₂ 441.12; found 440 (M-H)⁻. HPLC *t*_{R} 1.51 min.

### Example 104: (3-((6-(3,5-dimethylisoxazol-4-yl)pyridin-3-yl)methyl)-1,2,3-oxadiazol-3-ium-5-yl)((3-(trifluoromethyl)phenyl)carbamoyl)amide.

The title compound was obtained as a white solid reacting (3,5-dimethylisoxazol-4-yl)boronic acid and (3-((6-bromopyridin-3-yl)methyl)-1,2,3-oxadiazol-3-ium-5-yl)((3-(trifluoromethyl)phenyl) carbamoyl)amide (prepared as reported in the synthesis of Example **6**) in Step 1. ¹H NMR (400 MHz, DMSO-*d*₆) δ 9.73 (s, 1H), 8.89 (s, 1H), 8.34 (s, 1H), 8.22 (br s, 1H), 8.10 (br d, *J* = 8.3 Hz, 1H), 7.72 (br d, *J* = 8.6 Hz, 1H), 7.66 (br d, *J* = 8.1 Hz, 1H), 7.44 (br t, *J* = 7.9 Hz, 1H), 7.23 (br d, *J* = 7.9 Hz, 1H), 5.88 (s, 2H), 2.58 (s, 3H), 2.39 (s, 3H). ¹⁹F NMR (377 MHz, DMSO-*d*₆) δ - 61.29 (s, 3F). LCMS (ES⁺) *m*/*z* calculated for C₂₁H₁₇F₃N₆O₃ 458.13; found 457 (M-H)⁻. HPLC *t*_{R} 1.76 min.

### Example 108: (3-((5-(pyrimidin-5-yl)pyridin-2-yl)methyl)-1,2,3-oxadiazol-3-ium-5-yl)((3-(trifluoromethyl)phenyl)carbamoyl)amide.

The title compound was obtained as a white solid reacting pyrimidin-5-ylboronic acid and (3-((5-bromopyridin-2-yl)methyl)-1,2,3-oxadiazol-3-ium-5-yl)((3-(trifluoromethyl)phenyl)carbamoyl) amide (prepared as reported in the synthesis of Example **6**) in Step 1. ¹H NMR (400 MHz, DMSO-*d*₆) δ 9.74 (br s, 1H), 9.26 (br s, 1H), 9.24 (br s, 2H), 9.06 (br s, 1H), 8.39 (dd, *J₁* = 8.0 Hz, *J₂* = 2.1 Hz, 1H), 8.24 (s, 1H), 8.20 (br s, 1H), 7.84 (br d, *J* = 8.1 Hz, 1H), 7.74 (br d, *J* = 7.9 Hz, 1H), 7.44 (br t, *J* = 8.0 Hz, 1H), 7.22 (br d, *J* = 7.5 Hz, 1H), 6.03 (s, 2H). ¹⁹F NMR (377 MHz, DMSO-*d*₆) δ -61.28 (s, 3F). LCMS (ES⁺) *m*/*z* calculated for C₂₀H₁₄F₃N₇O₂ 441.12; found 440 (M-H)⁻. HPLC *t*_{R} 1.49 min.

### Example 109: (3-((5-(3,5-dimethylisoxazol-4-yl)pyridin-2-yl)methyl)-1,2,3-oxadiazol-3-ium-5-yl)((3-(trifluoromethyl)phenyl)carbamoyl)amide.

The title compound was obtained as a white solid reacting (3,5-dimethylisoxazol-4-yl)boronic acid and (3-((5-bromopyridin-2-yl)methyl)-1,2,3-oxadiazol-3-ium-5-yl)((3-(trifluoromethyl)phenyl) carbamoyl)amide (prepared as reported in the synthesis of Example **6**) in Step 1. ¹H NMR (400 MHz, DMSO-*d*₆) δ 9.74 (br s, 1H), 8.67 (br s, 1H), 8.25 (s, 1H), 8.21 (br s, 1H), 8.00 (dd, *J₁* = 8.0 Hz, *J₂* = 2.1 Hz, 1H), 7.78-7.73 (m, 2H), 7.44 (br t, *J* = 8.0 Hz, 1H), 7.22 (br d, *J* = 7.7 Hz, 1H), 6.00 (s, 2H), 2.44 (s, 3H), 2.26 (s, 3H). ¹⁹F NMR (377 MHz, DMSO-*d*₆) δ -61.29 (s, 3F). LCMS (ES⁺) *m*/*z* calculated for C₂₁H₁₇F₃N₆O₃ 458.13; found 457 (M-H)⁻. HPLC *t*_{R} 1.76 min.

### Example 110: (3-((4-(4-methylpyrimidin-5-yl)thiophen-2-yl)methyl)-1,2,3-oxadiazol-3-ium-5-yl)((3-(trifluoromethyl)phenyl)carbamoyl)amide.

The title compound was obtained as a white solid reacting 4-methyl-5-(4,4,5,5-tetramethyl-1,3,2-dioxaborolan-2-yl)pyrimidine and (3-((4-bromothiophen-2-yl)methyl)-1,2,3-oxadiazol-3-ium-5-yl)((3-(trifluoromethyl)phenyl)carbamoyl)amide (prepared as reported in the synthesis of Example **6**) in Step 1. ¹H NMR (400 MHz, DMSO-*d*₆) δ 9.66 (s, 1H), 8.95 (s, 1H), 8.65 (s, 1H), 8.19 (s, 1H), 8.13 (br s, 1H), 7.89 (d, *J* = 1.3 Hz, 1H), 7.68 (s, 1H), 7.65 (br s, 1H), 7.37 (br t, *J* = 7.9 Hz, 1H), 7.16 (br d, *J* = 7.7 Hz, 1H), 6.04 (s, 2H), 2.48 (s, 3H). ¹⁹F NMR (377 MHz, DMSO-*d*₆) δ -61.28 (s, 3F). LCMS (ES⁺) *m*/*z* calculated for C₂₀H₁₅F₃N₆O₂S 460.09; found 459 (M-H)⁻. HPLC *t*_{R} 1.66 min.

### Example 117: (3-((6-(1-(piperidin-4-yl)-1H-pyrazol-4-yl)pyridin-3-yl)methyl)-1,2,3-oxadiazol-3-ium-5-yl)((3-(trifluoromethyl)phenyl)carbamoyl)amide.

The title compound was obtained as a white solid reacting tert-butyl 4-(4-(4,4,5,5-tetramethyl-1,3,2-dioxaborolan-2-yl)-1H-pyrazol-1-yl)piperidine-1-carboxylate and (3-((6-bromopyridin-3-yl)methyl)-l,2,3-oxadiazol-3-ium-5-yl)((3-(trifluoromethyl)phenyl)carbamoyl)amide (prepared as reported in the synthesis of Example **6**) in Step 1, crude material from Step 1 was treated with HCl (4.0 M in dioxane) to get the title compound. ¹H NMR (400 MHz, DMSO-*d*₆) δ 9.73 (s, 1H), 8.74 (d, *J* = 1.8 Hz, 1H), 8.64 (br s, 1H), 8.43 (br s, 2H), 8.27 (s, 1H), 8.22 (br s, 1H), 8.13 (s, 1H), 8.01-7.99 (m, 1H), 7.79 (br d, *J* = 8.1 Hz, 1H), 7.71 (br d, *J* = 8.6 Hz, 1H), 7.44 (br t, *J* = 8.2 Hz, 1H), 7.22 (br d, *J* = 8.1 Hz, 1H), 5.82 (s, 2H), 4.59-4.52 (m, 1H), 3.46-3.40 (m, 2H), 3.14-3.06 (m, 2H), 2.29-2.22 (m, 2H), 2.19-2.11 (m, 2H). ¹⁹F NMR (377 MHz, DMSO-*d*₆) δ -61.28 (s, 3F). LCMS (ES⁺) *m*/*z* calculated for C₂₄H₂₃F₃N₈O₂ 512.19; found 511 (M-H)⁻. HPLC *t*_{R} 1.28 min.

### Example 119: (3-((5-(isoindolin-4-yl)pyridin-2-yl)methyl)-1,2,3-oxadiazol-3-ium-5-yl)((3-(trifluoromethyl)phenyl)carbamoyl)amide.

The title compound was obtained as a white solid reacting *tert*-butyl 4-(4,4,5,5-tetramethyl-1,3,2-dioxaborolan-2-yl)isoindoline-2-carboxylate and (3-((5-bromopyridin-2-yl)methyl)-1,2,3-oxadiazol-3-ium-5-yl)((3-(trifluoromethyl)phenyl)carbamoyl)amide (prepared as reported in the synthesis of Example **6**) in Step 1; crude material from Step 1 was treated with HCl (4 N in dioxane) to get the title compound. ¹H NMR (400 MHz, DMSO-*d*₆) δ 9.75 (br s, 1H), 9.47 (br s, 2H), 8.76 (br d, *J* = 1.8 Hz, 1H), 8.26 (s, 1H), 8.22 (br s, 1H), 8.09 (dd, *J₁* = 8.1 Hz, *J₂* = 2.4 Hz, 1H), 7.81 (br d, *J* = 7.9 Hz, 1H), 7.73 (br d, *J* = 7.7 Hz, 1H), 7.56-7.50 (br m, 3H), 7.45 (br t, *J* = 8.0 Hz, 1H), 7.23 (br d, *J* = 7.5 Hz, 1H), 6.03 (s, 2H), 4.68-4.63 (m, 2H), 4.61-4.58 (m, 2H). ¹⁹F NMR (377 MHz, DMSO-*d*₆) δ -61.29 (s, 3F). LCMS (ES⁺) *m*/*z* calculated for C₂₄H₁₉F₃N₆O₂ 480.15; found 479 (M-H)⁻. HPLC *t*_{R} 1.28 min.

### Example 120: (3-((5-(1-(piperidin-4-yl)-1H-pyrazol-4-yl)pyridin-2-yl)methyl)-1,2,3-oxadiazol-3-ium-5-yl)((3-(trifluoromethyl)phenyl)carbamoyl)amide.

The title compound was obtained as a white solid reacting *tert*-butyl 4-(4-(4,4,5,5-tetramethyl-1,3,2-dioxaborolan-2-yl)-1*H*-pyrazol-1-yl)piperidine-1-carboxylate and (3-((5-bromopyridin-2-yl)methyl)-1,2,3-oxadiazol-3-ium-5-yl)((3-(trifluoromethyl)phenyl)carbamoyl)amide (prepared as reported in the synthesis of Example **6**) in Step 1, crude material from Step 1 was treated with HCl (4.0 N in dioxane) to get the title compound. ¹H NMR (400 MHz, DMSO-*d*₆) δ 9.72 (s, 1H), 8.90 (br d, *J* = 1.8 Hz, 1H), 8.64 (br s, 1H), 8.39 (br s, 2H), 8.20 (br s, 1H), 8.17 (s, 1H), 8.12 (dd, *J₁* = 8.1 Hz, *J₂* = 2.2 Hz, 1H), 8.09 (s, 1H), 7.72 (br d, *J* = 8.1 Hz, 1H), 7.67 (br d, *J* = 8.1 Hz, 1H), 7.44 (br t, *J* = 8.0 Hz, 1H), 7.22 (br d, *J* = 7.5 Hz, 1H), 5.91 (s, 2H), 4.57-4.50 (m, 1H), 3.75-3.69 (m, 1H), 3.46-3.39 (m, 2H), 3.15-3.06 (m, 2H), 2.27-2.22 (m, 2H), 2.17-2.10 (m, 2H). ¹⁹F NMR (377 MHz, DMSO-*d*₆) δ -61.27 (s, 3F). LCMS (ES⁺) *m*/*z* calculated for C₂₄H₂₃F₃N₈O₂ 512.19; found 511 (M-H)⁻. HPLC *t*_{R} 1.20 min.

### Example 121: (3-((5-(2-(aminomethyl)phenyl)pyridin-2-yl)methyl)-1,2,3-oxadiazol-3-ium-5-yl)((3-(trifluoromethyl)phenyl)carbamoyl)amide.

The title compound was obtained as a white solid reacting *tert*-butyl (2-(4,4,5,5-tetramethyl-1,3,2-dioxaborolan-2-yl)benzyl)carbamate and (3-((5-bromopyridin-2-yl)methyl)-1,2,3-oxadiazol-3-ium-5-yl)((3-(trifluoromethyl)phenyl)carbamoyl)amide (prepared as reported in the synthesis of Example **6**) in Step 1. crude material from Step 1 was treated with HCl (4.0 M in dioxane) to get the title compound. ¹H NMR (400 MHz, DMSO-*d*₆) δ 9.75 (s, 1H), 8.63 (br d, *J =* 1.8 Hz, 1H), 8.26 (s, 1H), 8.22 (br s, 1H), 8.12 (br s, 3H), 7.98 (dd, *J₁* = 8.0 Hz, *J₂* = 2.3 Hz, 1H), 7.81-7.72 (m, 2H), 7.66 (br d, *J=* 7.5 Hz, 1H), 7.59-7.50 (m, 2H), 7.45 (br t, *J=* 8.0 Hz, 1H), 7.40 (br d, *J=* 7.7 Hz, 1H), 7.23 (br d*, J =* 7.2 Hz, 1H), 6.03 (s, 2H), 4.00-3.96 (m, 2H). ¹⁹F NMR(377 MHz, DMSO-*d*₆) δ -61.28 (s, 3F). LCMS (ES⁺) *m*/*z* calculated for C₂₃H₁₉F₃N₆O₂ 468.15; found 467 (M-H)⁻. HPLC *t*_{R} 1.30 min.

### Example 124: (3-((5-(4,5,6,7-tetrahydropyrazolo[1,5-a]pyrazin-3-yl)pyridin-2-yl)methyl)-1,2,3-oxadiazol-3-ium-5-yl)((3-(trifluoromethyl)phenyl)carbamoyl)amide.

The title compound was obtained as a yellow solid reacting *tert*-butyl 3-(4,4,5,5-tetramethyl-1,3,2-dioxaborolan-2-yl)-6,7-dihydropyrazolo[1,5-a]pyrazine-5(4H)-carboxylate and (3-((5-bromopyridin-2-yl)methyl)-1,2,3-oxadiazol-3-ium-5-yl)((3-(trifluoromethyl)phenyl)carbamoyl)amide (prepared as reported in the synthesis of Example 6) in Step 1, crude material from Step 1 was treated with HCl (4.0 M in dioxane) to get the title compound. ¹H NMR (400 MHz, DMSO-*d*₆) δ 9.74 (s, 1H), 9.58 (br s, 2H), 8.71 (br d, *J=* 2.0 Hz, 1H), 8.20 (br s, 2H), 8.07 (s, 1H), 7.99 (dd, *J₁* = 8.1 Hz, *J₂* = 2.4 Hz, 1H), 7.75-7.71 (m, 2H), 7.44 (br t, *J=* 7.9 Hz, 1H), 7.23 (br d, *J=* 7.7 Hz, 1H), 5.96 (s, 2H), 4.72 (br s, 2H), 4.39 (br t, *J=* 5.7 Hz, 2H), 3.71 (br t, *J =* 5.6 Hz, 2H). ¹⁹F NMR (377 MHz, DMSO-*d*₆) δ -61.28 (s, 3F). LCMS (ES⁺) *m*/*z* calculated for C₂₂H₁₉F₃N₈O₂ 484.16; found 483 (M-H)⁻. HPLC *t*_{R} 1.13 min.

### Example 90: (3-(4-(2-Methoxy-4-methytpyrimidin-5-yt)benzyt)-1,2,3-oxadiazol-3-ium-5-yl)((3-(trifluoromethyl)phenyl)carbamoyl)amide

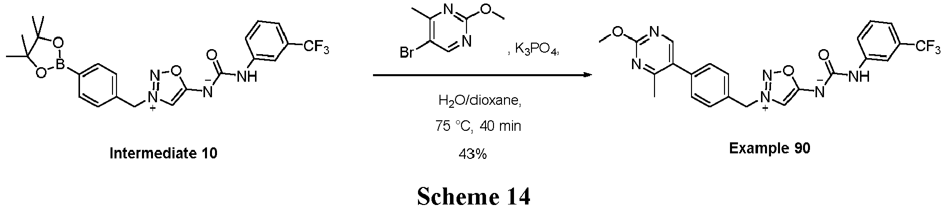

A solution of Intermediate **10** (30.0 mg, 0.07 mmol), tripotassium phosphate (42.0 mg, 0.20 mmol), bis(chloranyl)palladium cyclopentyl(diphenyl)phosphane ferrocene (5.5 mg, 0.01 mmol) and 5-bromo-2-methoxy-4-methylpyrimidine (26.8 mg, 0.13 mmol) in a mixture of H₂O/dioxane (1:10) (1.1 mL) was heated at 75 °C for 40 min. The reaction mixture was cooled at rt and filtered on a pad of solca flock. The excess of solvent was removed under reduced pressure to get a residue which was purified by chromatography on silica gel (eluting with 0-100% EtOAc in petroleum ether) to give the title compound as a white solid (14 mg, 43%). ¹H NMR (400 MHz, DMSO-*d*₆) δ 9.84 (br s, 1H), 8.53 (s, 1H), 8.39 (s, 1H), 8.32 (s, 1H), 7.84-7.76 (m, 3H), 7.64 (d, *J=* 7.9 Hz, 2H), 7.55 (br t, *J =* 8.0 Hz, 1H), 7.33 (br d, *J =* 7.5 Hz, 1H), 5.96 (s, 2H), 4.05 (s, 3H), 2.50 (s, 3H). ¹⁹F NMR (377 MHz, DMSO-*d*₆) δ -61.29 (s, 3F). LCMS (ES⁺) *m*/*z* calculated for C₂₃H₁₉F₃N₆O₃ 484.15; found 483 (M-H)⁻. HPLC *t*_{R} 1.98 min.

**Examples 80, 81, 82, 83, 84, 85, 86, 89, 91, 92, 98, 101, 107, 111, and 112** were synthesized according to the above procedure (Scheme 14) by reacting with the appropriate starting materials.

### Example 80: ((3-(trifluoromethyl)phenyl)carbamoyl)(3-(4-(4-(trifluoromethyl)pyrimidin-5-yl)benzyl)-1,2,3-oxadiazol-3-ium-5-yl)amide.

The title compound was obtained as a white solid reacting 5-bromo-4-(trifluoromethyl)pyrimidine (using XPhos Pd G₄ in THF) in Step 1. ¹H NMR (400 MHz, DMSO-*d*₆) δ 9.74 (s, 1H), 9.48 (s, 1H), 9.09 (s, 1H), 8.30 (s, 1H), 8.22 (br s, 1H), 7.73 (br d, *J=* 8.1 Hz, 3H), 7.56 (br d, *J=* 8.1 Hz, 2H), 7.44 (t, *J =* 8.2 Hz, 1H), 7.23 (br d, *J =* 7.9 Hz, 1H), 5.89 (s, 2H). ¹⁹F NMR (377 MHz, DMSO-*d*₆) δ -61.29 (s, 3F), -62.35 (s, 3F). LCMS (ES⁺) *m*/*z* calculated for C₂₂H₁₄F₆N₆O₂ 508.11; found 509 (M+H)⁺. HPLC *t*_{R} 2.42 min.

### Example 81: (3-(4-(4,6-dimethylpyrimidin-5-yl)benzyl)-1,2,3-oxadiazol-3-ium-5-yl)((3-(trifluoromethyl)phenyl)carbamoyl)amide.

The title compound was obtained as a beige solid reacting 5-bromo-4,6-dimethylpyrimidine (using XPhos Pd G₄ in THF) in Step 1, ¹H NMR (400 MHz, DMSO-*d*₆) δ 9.74 (br s, 1H), 8.89 (s, 1H), 8.32 (s, 1H), 8.22 (s, 1H), 7.73-7.70 (br m, 3H), 7.46-7.41 (m, 3H), 7.23 (br d*, J =* 7.7 Hz, 1H), 5.87 (s, 2H), 2.18 (s, 6H). ¹⁹F NMR (377 MHz, DMSO-*d*₆) δ -61.28 (s, 3F). LCMS (ES⁺) *m*/*z* calculated for C₂₃H₁₉F₃N₆O₂ 468.15; found 467 (M-H)⁻. HPLC *t*_{R} 1.69 min.

### Example 82: (R)-(3-(1-(4-(4-methylpyrimidin-5-yl)phenyl)ethyl)-1,2,3-oxadiazol-3-ium-5-yl)((3-(trifluoromethyl)phenyl)carbamoyl)amide.

The title compound was obtained as a white solid reacting 5-bromo-4-methylpyrimidine and (R)-(3-(1-(4-(4,4,5,5-tetramethyl-1,3,2-dioxaborolan-2-yl)phenyl)ethyl)-1,2,3-oxadiazol-3-ium-5-yl)((3-(trifluoromethyl)phenyl)carbamoyl)amide (prepared as reported in the synthesis of Example **60** starting from (R)-(3-(1-(4-bromophenyl)ethyl)-1,2,3-oxadiazol-3-ium-5-yl)((3-(trifluoromethyl)phenyl)carbamoyl)amide prepared as reported in Scheme 4), XPhos Pd G₄ in THF was used in Step 1. ¹H NMR (400 MHz, DMSO-*d*₆) δ 9.66 (s, 1H), 8.99 (s, 1H), 8.56 (s, 1H), 8.24 (s, 1H), 8.18 (br s, 1H), 7.69 (br d, *J=* 8.1 Hz, 2H), 7.62 (br d, *J=* 7.8 Hz, 1H), 7.53 (br d, *J* = 8.3 Hz, 2H), 7.37 (br t, *J =* 8.0 Hz, 1H), 7.16 (br d, *J =* 7.3 Hz, 1H), 6.21-6.16 (m, 1H), 2.39 (s, 3H), 2.00 (br *d, J =* 7.5 Hz, 3H). ¹⁹F NMR (377 MHz, DMSO-*d*₆) δ -61.28 (s, 3F). LCMS (ES⁺) *m*/*z* calculated for C₂₃H₁₉F₃N₆O₂ 468.15; found 467 (M-H)⁻. HPLC *t*_{R} 1.76 min.

### Example 83: (3-(3-(4-methylpyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-methylpyrimidine and (3-(3-(4,4,5,5-tetramethyl-1,3,2-dioxaborolan-2-yl)benzyl)-1,2,3-oxadiazol-3-ium-5-yl)((3-(trifluoromethyl)phenyl)carbamoyl)amide (prepared as reported in the synthesis of Example 60 starting from Example **46**), XPhos Pd G₄ in THF was used in Step 1. ¹H NMR (400 MHz, DMSO-*d*₆) δ 9.72 (s, 1H), 9.07 (s, 1H), 8.64 (s, 1H), 8.28 (s, 1H), 8.21 (br s, 1H), 7.75-7.58 (m, 5H), 7.44 (br t, *J=* 8.1 Hz, 1H), 7.22 (br d, *J=* 7.7 Hz, 1H), 5.85 (s, 2H), 2.47 (s, 3H). ¹⁹F NMR (377 MHz, DMSO-*d*₆) δ -61.29 (s, 3F). LCMS (ES⁺) *m*/*z* calculated for C₂₂H₁₇F₃N₆O₂ 454.14; found 453 (M-H)⁻. HPLC *t*_{R} 1.72 min.

### Example 84: (3-(3-(4-methoxypyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-methoxypyrimidine and (3-(3-(4,4,5,5-tetramethyl-1,3,2-dioxaborolan-2-yl)benzyl)-1,2,3-oxadiazol-3-ium-5-yl)((3-(trifluoromethyl)phenyl)carbamoyl)amide (prepared as reported in the synthesis of Example **60** starting from Example **46**). XPhos Pd G₄ in THF was used in Step 1. ¹H NMR (400 MHz, DMSO-*d*₆) δ 9.72 (s, 1H), 8.82 (s, 1H), 8.63 (s, 1H), 8.25 (s, 1H), 8.21 (br s, 1H), 7.87 (s, 1H), 7.72-7.69 (m, 2H), 7.64-7.55 (m, 2H), 7.43 (br t, *J=* 7.9 Hz, 1H), 7.22 (br d, *J=* 7.7 Hz, 1H), 5.84 (s, 2H), 3.99 (s, 3H). ¹⁹F NMR (377 MHz, DMSO-*d*₆) δ -61.30 (s, 3F). LCMS (ES⁺) *m*/*z* calculated for C₂₂H₁₇F₃N₆O₃ 470.13; found 469 (M-H)⁻. HPLC *t*_{R} 1.82 min.

### Example 85: (3-(4-(4-methylpyridin-3-yl)benzyl)-1,2,3-oxadiazol-3-ium-5-yl)((3-(trifluoromethyl)phenyl)carbamoyl)amide.

The title compound was obtained as a white solid reacting 3-bromo-4-methylpyridine, XPhos Pd G₄ in THF was used in Step 1. ¹H NMR (400 MHz, DMSO-*d*₆) δ 9.74 (s, 1H), 8.45 (br d, *J =* 5.0 Hz, 1H), 8.38 (s, 1H), 8.30 (s, 1H), 8.22 (br s, 1H), 7.73 (br d, *J=* 8.1 Hz, 1H), 7.69 (br d, *J=* 8.3 Hz, 2H), 7.52 (br d, *J=* 8.3 Hz, 2H), 7.44 (br t, *J=* 8.1 Hz, 1H), 7.35 (br d, *J=* 5.0 Hz, 1H), 7.22 (br d, *J=* 7.7 Hz, 1H), 5.86 (s, 2H), 2.27 (s, 3H). ¹⁹F NMR (377 MHz, DMSO-*d*₆) δ -61.28 (s, 3F). LCMS (ES⁺) *m*/*z* calculated for C₂₃H₁₈F₃N₅O₂ 453.14; found 452 (M-H)⁻. HPLC *t*_{R} 1.37 min.

### Example 86: (3-(4-(3-methylpyrazin-2-yl)benzyl)-1,2,3-oxadiazol-3-ium-5-yl)((3-(trifluoromethyl)phenyl)carbamoyl)amide.

The title compound was obtained as a beige solid reacting 2-bromo-3-methylpyrazine; XPhos Pd G₄ in THF was used in Step 1. ¹H NMR (400 MHz, DMSO-*d*₆) δ 9.74 (s, 1H), 8.58-8.55 (m, 2H), 8.30 (s, 1H), 8.21 (br s, 1H), 7.76-7.69 (m, 5H), 7.44 (br t, *J =* 7.9 Hz, 1H), 7.22 (br d, *J =* 7.7 Hz, 1H), 5.88 (s, 2H), 2.57 (s, 3H). ¹⁹F NMR (377 MHz, DMSO-*d*₆) δ -61.28 (s, 3F). LCMS (ES⁺) *m*/*z* calculated for C₂₂H₁₇F₃N₆O₂ 454.14; found 453 (M-H)⁻. HPLC *t*_{R} 1.76 min.

### Example 89: (3-(4-(2-(2-hydroxyethoxy)-4-methylpyrimidin-5-yl)benzyl)-1,2,3-oxadiazol-3-ium-5-yl)((3-(trifluoromethyl)phenyl)carbamoyl)amide.

The title compound was obtained as a yellow solid reacting 2-((5-bromo-4-methylpyrimidin-2-yl)oxy)ethan-1-ol in Step 1. ¹H NMR (400 MHz, DMSO-*d*₆) δ 9.65 (s, 1H), 8.34 (s, 1H), 8.20 (s, 1H), 8.14 (br s, 1H), 7.65-7.60 (m, 3H), 7.46 (br d, *J =* 8.1 Hz, 2H), 7.37 (br t, *J =* 7.8 Hz, 1H), 7.15 (br d, *J=* 7.2 Hz, 1H), 5.78 (s, 2H), 4.84-4.80 (m, 1H), 4.29-4.26 (m, 2H), 3.68-3.64 (m, 2H), 2.31 (s, 3H). ¹⁹F NMR (377 MHz, DMSO-*d*₆) δ -61.29 (s, 3F). LCMS (ES⁺) *m*/*z* calculated for C₂₄H₂₁F₃N₆O₄ 514.16; found 513 (M-H)⁻. HPLC *t*_{R} 1.68 min.

### Example 91: (3-(4-(4-cyclopropylpyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-cyclopropylpyrimidine; XPhos Pd G₄ in THF was used in Step 1. ¹H NMR (400 MHz, DMSO-*d*₆) δ 9.73 (s, 1H), 8.99 (s, 1H), 8.55 (s, 1H), 8.30 (s, 1H), 8.22 (br s, 1H), 7.75-7.71 (m, 3H), 7.62 (br d, *J=* 8.1 Hz, 2H), 7.44 (br t*, J =* 7.9 Hz, 1H), 7.23 (br d, *J =* 7.9 Hz, 1H), 5.88 (s, 2H), 2.06-1.98 (m, 1H), 1.16-1.10 (m, 2H), 1.06-1.02 (m, 2H). ¹⁹F NMR (377 MHz, DMSO-*d*₆) δ -61.29 (s, 3F). LCMS (ES⁺) *m*/*z* calculated for C₂₄H₁₉F₃N₆O₂ 480.15; found 479 (M-H)⁻. HPLC *t*_{R} 1.94 min.

### Example 92: (3-(4-(2-methylpyridin-3-yl)benzyl)-1,2,3-oxadiazol-3-ium-5-yl)((3-(trifluoromethyl)phenyl)carbamoyl)amide.

The title compound was obtained as a white solid reacting 3-bromo-2-methylpyridine; XPhos Pd G₄ in THF was used in Step 1. ¹H NMR (400 MHz, DMSO-*d*₆) δ 9.66 (s, 1H), 8.40 (dd, *J₁* = 4.8 Hz, *J₂* = 1.8 Hz, 1H), 8.21 (s, 1H), 8.14 (br s, 1H), 7.65 (br d, *J=* 8.8 Hz, 1H), 7.60 (br d, *J=* 8.1 Hz, 2H), 7.55 (dd, *J₁* = 7.7 Hz, *J₂* = 1.5 Hz, 1H), 7.43 (br d, *J =* 8.1 Hz, 2H), 7.37 (br t, *J =* 8.0 Hz, 1H), 7.24 (dd, *J₁* = 7.7 Hz, *J₂* = 4.8 Hz, 1H), 7.15 (br d, *J=* 7.5 Hz, 1H), 5.78 (s, 2H), 2.35 (s, 3H). ¹⁹F NMR (377 MHz, DMSO-*d*₆) δ -61.28 (s, 3F). LCMS (ES⁺) *m*/*z* calculated for C₂₃H₁₈F₃N₅O₂ 453.14; found 452 (M-H)⁻. HPLC *t*_{R} 1.34 min.

### Example 98: (3-(4-(1,4-dimethyl-1H-imidazol-5-yl)benzyl)-1,2,3-oxadiazol-3-ium-5-yl)((3-(trifluoromethyl)phenyl)carbamoyl)amide.

The title compound was obtained as a white solid reacting 5-bromo-1,4-dimethyl-1H-imidazole; XPhos Pd G₄ in THF was used in Step 1. ¹H NMR (400 MHz, DMSO-*d*₆) δ 9.73 (s, 1H), 8.30 (s, 1H), 8.22 (br s, 1H), 7.72 (br d, *J=* 7.9 Hz, 1H), 7.67 (br d, *J=* 8.1 Hz, 2H), 7.59 (s, 1H), 7.48 (br d, *J=* 8.1 Hz, 2H), 7.44-7.42 (m, 1H), 7.22 (br d, *J=* 7.7 Hz, 1H), 5.83 (s, 2H), 3.53 (s, 3H), 2.11 (s, 3H). ¹⁹F NMR (377 MHz, DMSO-*d*₆) δ -61.28 (s, 3F). LCMS (ES⁺) *m*/*z* calculated for C₂₂H₁₉F₃N₆O₂ 456.15; found 455 (M-H)⁻. HPLC *t*_{R} 1.31 min.

### Example 101: (3-(3-methyl-4-(4-methylpyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-methylpyrimidine and ((3-(3-methyl-4-(4,4,5,5-tetramethyl-1,3,2-dioxaborolan-2-yl)benzyl)-1,2,3-oxadiazol-3-ium-5-yl)((3-(trifluoromethyl)phenyl)carbamoyl)amide (prepared as reported in the synthesis of Example **60** starting from Intermediate **23**) in Step 1. ¹H NMR (400 MHz, DMSO-*d*₆) δ 9.72 (s, 1H), 9.08 (s, 1H), 8.53 (s, 1H), 8.28 (s, 1H), 8.22 (br s, 1H), 7.72 (br d*, J =* 7.9 Hz, 1H), 7.59 (s, 1H), 7.52 (br d, *J* = 8.1 Hz, 1H), 7.44 (br t, *J =* 7.9 Hz, 1H), 7.31 (br d, *J =* 7.7 Hz, 1H), 7.23 (br *d, J =* 7.7 Hz, 1H), 5.82 (s, 2H), 2.23 (s, 3H), 2.08 (s, 3H). ¹⁹F NMR (377 MHz, DMSO-*d*₆) δ-61.28 (s, 3F). LCMS (ES⁺) *m*/*z* calculated for C₂₃H₁₉F₃N₆O₂ 468.15; found 467 (M-H)⁻. HPLC *t*_{R} 1.72 min.

### Example 107: (3-(4-(4-fluoro-6-oxo-1,6-dihydropyrimidin-5-yl)benzyl)-1,2,3-oxadiazol-3-ium-5-yl)((3-(trifluoromethyl)phenyl)carbamoyl)amide.

The title compound was obtained as a light-pink solid reacting 5-bromo-4,6-difluoropyrimidine; XPhos Pd G₄ in THF was used in Step 1. ¹H NMR (400 MHz, DMSO-*d*₆) δ 9.65 (s, 1H), 8.16 (s, 1H), 8.13 (br s, 1H), 8.05 (br s, 1H), 7.66 (br d, *J=* 8.8 Hz, 1H), 7.50 (br s, 4H), 7.36 (br t, *J=* 7.8 Hz, 1H), 7.15 (br *d, J =* 7.7 Hz, 1H), 5.73 (s, 2H). ¹⁹F NMR (377 MHz, DMSO-*d*₆) δ -61.27 (s, 3F). LCMS (ES⁺) *m*/*z* calculated for C₂₁H₁₄F₄N₆O₃ 474.11; found 473 (M-H)⁻. HPLC *t*_{R} 1.64 min.

### Example 111: (3-(4-(4-methoxy-6-methylpyrimidin-5-yl)benzyl)-1,2,3-oxadiazol-3-ium-5-yl)((3-(trifluoromethyl)phenyl)carbamoyl)amide.

The title compound was obtained as a white solid reacting 5-bromo-4-methoxy-6-methylpyrimidine in Step 1. ¹H NMR (400 MHz, DMSO-*d*₆) δ 9.74 (s, 1H), 8.68 (s, 1H), 8.31 (s, 1H), 8.22 (br s, 1H), 7.73 (br d*, J =* 8.8 Hz, 1H), 7.65 (br d, *J=* 8.1 Hz, 2H), 7.46-7.40 (m, 3H), 7.23 (br d*, J =* 7.9 Hz, 1H), 5.84 (s, 2H), 3.84 (s, 3H), 2.21 (s, 3H). ¹⁹F NMR (377 MHz, DMSO-*d*₆) δ -61.28 (s, 3F). LCMS (ES⁺) *m*/*z* calculated for C₂₃H₁₉F₃N₆O₃ 484.15; found 483 (M-H)⁻. HPLC *t*_{R} 1.82 min.

### Example 112: (3-(4-(4-(2-hydroxyethoxy)-6-methylpyrimidin-5-yl)benzyl)-1,2,3-oxadiazol-3-ium-5-yl)((3-(trifluoromethyl)phenyl)carbamoyl)amide.

The title compound was obtained as a white solid reacting 2-((5-bromo-6-methylpyrimidin-4-yl)oxy)ethan-1-ol in Step 1. ¹H NMR (400 MHz, DMSO-*d*₆) δ 9.74 (s, 1H), 8.64 (s, 1H), 8.62 (br s, 1H), 8.32 (s, 1H), 8.22 (br s, 1H), 7.73 (br d, *J=* 9.0 Hz, 1H), 7.64 (br d, *J=* 8.1 Hz, 2H), 7.46-7.42 (m, 3H), 7.23 (br d, *J=* 7.7 Hz, 1H), 5.85 (s, 2H), 4.36-4.31 (m, 2H), 3.62-3.58 (m, 2H), 2.22 (s, 3H). ¹⁹F NMR (377 MHz, DMSO-*d*₆) δ -61.28 (s, 3F). LCMS (ES⁺) *m*/*z* calculated for C₂₄H₂₁F₃N₆O₄ 514.16; found 513 (M-H)⁻. HPLC *t*_{R} 1.63 min.

### Example 123: ((3-((5-(4-(2-(metheyliumyl(methyl)ammonio)ethoxy)-6-methylpyrimidin-5-yl)pyridin- 2-yl)methyl)-1,2,3-oxadiazol-3-ium-5-yl)((3-(trifluoromethyl)vhenyl)carbamoyl)amide) (2,2,2-trifluoroacetate)

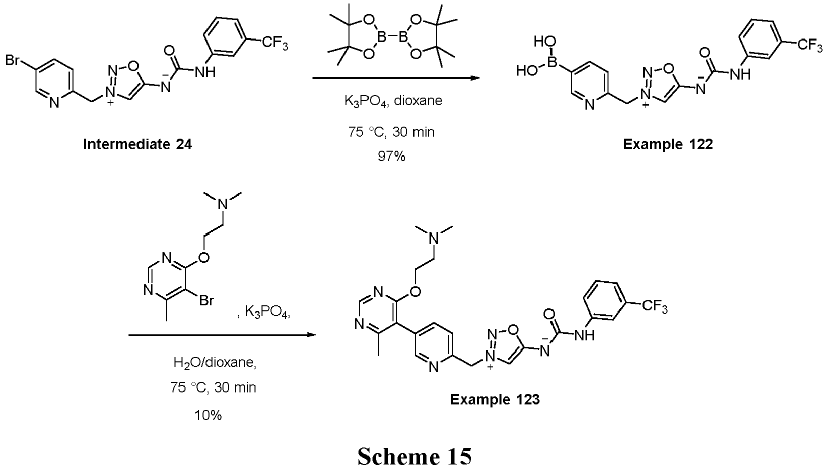

### Step 1: (3-((5-boronopyridin-2-yl)methyl)-1,2,3-oxadiazol-3-ium-5-yl) ((3-(trifluoromethyl)phenyl)carbamoyl)amide (Example 122).

A solution of bis(chloranyl)palladium; cyclopentyl(diphenyl)phosphane ferrocene (5.6 mg, 0.01 mmol), (3-((5-bromopyridin-2-yl)methyl)-1,2,3-oxadiazol-3-ium-5-yl)((3-(trifluoromethyl)phenyl)carbamoyl)amide (Intermediate **24**) (prepared as described in the synthesis of **Example 6**) (30.0 mg, 0.07 mmol), potassium acetate (20.0 mg, 0.20 mmol) and 4,4,5,5-tetramethyl-2-(4,4,5,5-tetramethyl-1,3,2-dioxaborolan-2-yl)-1,3,2-dioxaborolane (34.5 mg, 0.14 mmol) in dioxane (7.0 mL) was heated at 75 °C for 30 min. The reaction mixture was cooled at rt and filtered on a pad of solca flock. The excess of solvent was removed under reduced pressure to afford the title compound (27.0 mg, 97%) as a brown oil which was used as such in the next step. ¹H NMR (400 MHz, DMSO-*d*₆) δ 9.73 (s, 1H), 8.75 (s, 1H), 8.21-8.18 (m, 3H), 7.74 (d, *J =* 8.0 Hz, 1H), 7.59 (d, *J =* 8.0 Hz, 1H), 7.44 (t, *J =* 8.0 Hz, 1H), 7.22 (d, *J =* 8.0 Hz, 1H), 5.94 (s, 2H). LCMS (ES⁺) *m*/*z* calculated for C₁₆H₁₃BF₃N₅O₄ 407.10; found 408 (M+H)⁺. HPLC *t*_{R} 1.37 min.

*Step 2: ((3-((5-(4-(2-(metheyliumyl(methyl)ammonio)ethoxy)-6-methylpyrimidin-5-yl)pyridin-2-yl)methyl)-1,2,3-oxadiazol-3-ium-5-yl) ((3-(trifluoromethyl)phenyl)carbamoyl)amide) (2,2,2-trifluoroacetate) (**Example 123**)*

The title compound was prepared using same conditions reported for the synthesis of **Example 90** using **Example 122** (48 mg, 0.12 mmol) and 2-((5-bromo-6-methylpyrimidin-4-yl)oxy)-*N*,*N-*dimethylethan-1-amine (61 mg, 0.24 mmol). The title compound was obtained after purification on silica gel (eluting with 10% MeOH in DCM) and lyophilization from H₂O/MeCN + 0.1% TFA as a brown gummy (6.7 mg, 10%). ¹H NMR (400 MHz, DMSO-*d*₆) δ 9.75 (s, 1H), 9.48 (br s, 1H), 8.75 (s, 1H), 8.63 (d, *J=* 1.8 Hz, 1H), 8.23 (s, 1H), 8.21 (br s, 1H), 7.98 (dd, *J₁* = 8.1 Hz, *J₂* = 2.2 Hz, 1H), 7.76 (d, *J =* 8.1 Hz, 1H), 7.72 (br d, *J =* 8.8 Hz, 1H), 7.44 (t, *J =* 7.9 Hz, 1H), 7.23 (d, *J* = 7.7 Hz, 1H), 6.01 (s, 2H), 4.64-4.62 (m, 2H), 3.45-3.41 (m, 2H), 2.68 (s, 3H), 2.67 (s, 3H), 2.28 (s, 3H); ¹⁹F NMR (377 MHz, DMSO-*d*₆) δ -61.28 (s, 3F). LCMS (ES⁺) *m*/*z* calculated for C₂₅H₂₅F₃N₈O₃ 542.20; found 543 (M+H)⁺. HPLC *t*_{R} 1.20 min.

**Examples 43 and 118** were synthesized according to the above procedure (Scheme 15) by reacting with the appropriate starting materials.

### Example 43: (3-((5-(2-(2-(dimethylamino)ethoxy)-4-methylpyrimidin-5-yl)pyridin-2-yl)methyl)-1,2,3-oxadiazol-3-ium-5-yl)((3-(trifluoromethyl)phenyl)carbamoyl)amide.

The title compound was obtained as a yellow solid reacting 2-((5-bromo-4-methylpyrimidin-2-yl)oxy)-*N,N-*dimethylethan-1-amine in Step 2. ¹H NMR (400 MHz, DMSO-*d*₆) δ 9.74 (s, 1H), 9.48 (br s, 1H), 8.68 (br d, *J=* 1.8 Hz, 1H), 8.48 (s, 1H), 8.25 (s, 1H), 8.20 (br s, 1H), 8.05 (dd, *J₁* = 8.0 Hz, *J₂* = 2.3 Hz, 1H), 7.79-7.73 (m, 2H), 7.44 (t, *J =* 7.9 Hz, 1H), 7.22 (d, *J =* 7.7 Hz, 1H), 6.01 (s, 2H), 4.43 (t, *J=* 5.8 Hz, 2H), 2.66-2.63 (m, 2H), 2.40 (s, 3H), 2.22 (s, 6H); ¹⁹F NMR (377 MHz, DMSO-*d*₆) δ -61.28 (s, 3F). LCMS (ES⁺) *m*/*z* calculated for C₂₅H₂₅F₃N₈O₃ 542.20; found 541 (M-H)⁻. HPLC *t*_{R} 1.22 min.

### Example 118: (3-((5-(1,4-dimethyl-1H-imidazol-5-yl)pyridin-2-yl)methyl)-1,2,3-oxadiazol-3-ium-5-yl)((3-(trifluoromethyl)phenyl)carbamoyl)amide.

The title compound was obtained as a white solid reacting 5-bromo-1,4-dimethyl-1H-imidazole in Step 2. ¹H NMR (400 MHz, DMSO-*d*₆) δ 9.73 (s, 1H), 8.65 (s, 1H), 8.26 (s, 1H), 8.20 (br s, 1H), 8.00-7.97 (m, 1H), 7.77-7.68 (m, 3H), 7.47-7.42 (m, 1H), 7.24-7.22 (m, 1H), 6.00 (s, 2H), 3.56 (s, 3H), 2.13 (s, 3H). ¹⁹F NMR (377 MHz, DMSO-*d*₆) δ -61.28 (s, 3F). LCMS (ES⁺) *m*/*z* calculated for C₂₁H₁₈F₃N₇O₂ 457.15; found 456 (M-H)⁻. HPLC *t*_{R} 1.27 min.

### Example 31: (S)-(3-(2-hydroxy-1-phenylethyl)-1,2,3-oxadiazol-3-ium-5-yl)((3-(trifluoromethyl)phenyl)carbamoyl)amide

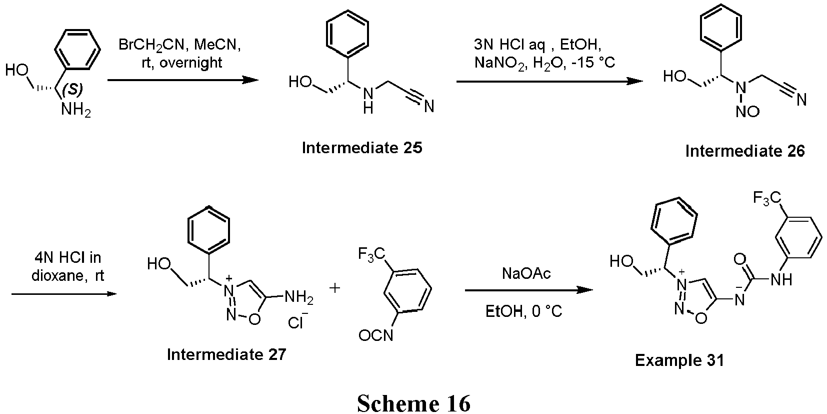

### Step 1: (S)-2-((2-hydroxy-1-phenylethyl)amino)acetonitrile (Intermediate 25).

To a suspension of (S)-2-phenylglycinol (343 mg, 2.5 mmol) and *N,N*-diisopropylethylamine (0.52 mL, 3 mmol) in MeCN (4 mL), 2-bromoacetonitrile (0.12 mL, 1.67 mmol) was added at rt. The reaction was stirred overnight at rt and then concentrated under reduced pressure. The obtained oil was treated with water (2 mL) and extracted with Et₂O. The organic phase was washed with water (3 × 3 mL) and brine, then was dried over Na₂SO₄, filtered, and concentrated under reduced pressure to obtain the title compound (268 mg, 91%) as an oil. ¹H NMR (300 MHz, DMSO-*d*₆) δ 7.43-7.16 (m, 5H), 5.02 (t, *J=* 5.5 Hz, 1H), 3.84-3.76 (m, 1H), 3.75-3.65 (m, 1H), 3.54-3.35 (m, 2H), 3.25-3.11 (m, 1H), 2.99 (ddd, *J =* 10.6, 4.6, 1.7 Hz, 1H). LCMS (ES⁺) *m*/*z* calculated for C₁₀H₁₂N₂O 176.09; found 177 (M+H)⁺. HPLC *t*_{R} 1.35 min.

### Step 2: (S)-N-(cyanomethyl)-N-(2-hydroxy-1-phenylethyl)nitrous amide (Intermediate 26).

A cold mixture (ice bath) of Intermediate **25** (268 mg, 1.52 mmol) in 3 N hydrogen chloride (2.03 mL, 6.08 mmol) in ethanol (2 mL) was added dropwise to a stirred and cooled (ice-salt) solution of sodium nitrite (210 mg, 3.04 mmol) in water (1.5 mL). The reaction was warmed up until rt for 1 h. Then, it was added brine and extracted with Et₂O. The organic phase was washed with water, brine and dried over Na₂SO₄, filtered and concentrated under reduced pressure to yield the title compound as an oil that as used as such in the next synthetic step ¹H NMR (300 MHz, DMSO-*d*₆) δ 7.56-7.26 (m, 5H), 5.78 (dd, *J =* 9.2, 5.1 Hz, 1H), 5.33 (dd, *J* = 5.5, 5.0 Hz, 1H), 4.68-4.54 (m, 2H), 4.32-4.23 (m, 1H), 4.03 (dt, *J =* 11.7, 5.0 Hz, 1H). HPLC *t*_{R} 2.25 min.

### Step 3: (S)-5-amino-3-(2-hydroxy-1-phenylethyl)-1,2,3-oxadiazol-3-ium chloride (Intermediate 27).

Intermediate **26** was directly treated with 4 N HCl in dioxane (1.5 mL) at rt to give a yellowish solution. After 10 min the reaction mixture was diluted with Et₂O (3 mL) and concentrated under reduced pressure. The crude was stripped with DCM and was dried under vacuum pump overnight to give the title compound (286 mg, 78% over two steps) as a white foam. ¹H NMR (300 MHz, DMSO-*d*₆) δ 9.73 (s, 2H), 8.22 (s, 1H), 7.71-7.53 (m, 2H), 7.52-7.41 (m, 3H), 6.21 (dd, *J =* 9.3, 4.2 Hz, 1H), 5.77 (br s, 1H), 4.44 (dd, *J =* 12.2, 9.4 Hz, 1H), 4.10 (dd, *J =* 12.2, 4.3 Hz, 1H). LCMS (ES⁺) m/z calculated for C₁₀H₁₂N₃O₂⁺ Exact Mass: 206.09; found 206 [M]+. HPLC t_{R} 1.44 min.

### Step 4: (S)-(3-(2-hydroxy-1-phenylethyl)-1,2,3-oxadiazol-3-ium-5-yl) ((3-(trifluoromethyl)phenyl) carbamoyl)amide (Example 31)

Intermediate **27** (222 mg, 0.92 mmol) in dry methanol (3 mL) was cooled at -10 °C (ice-salt bath) and sodium acetate (82 mg, 1.01 mmol) was added. The mixture was stirred at the same temperature for 15 min and 1-isocyanato-3-(trifluoromethyl)benzene (0.13 mL, 0.92 mmol) was added. The reaction was stirred further at -10 °C for 1 hr and then was diluted with water. The precipitated product was filtered and washed with water. The crude was purified on silica gel eluting with DCM/MeOH 99.8:0.2 to obtain the title compound (140 mg, 39%) as white solid. ¹H NMR (300 MHz, DMSO-*d*₆) δ 9.72 (s, 1H), 8.35 (s, 1H), 8.25 (s, 1H), 7.70 (br d, *J =* 8.6 Hz, 1H), 7.66-7.56 (m, 2H), 7.54-7.36 (m, 4H), 7.22 (br d, *J =* 7.7 Hz, 1H), 6.02 (dd, *J =* 9.4, 4.2 Hz, 1H), 5.69 (br s, 1H), 4.52 (br t, *J =* 10.7 Hz, 1H), 4.07 (br dd, *J =* 11.9, 4.1 Hz, 1H). LCMS (ES⁺) *m*/*z* calculated for C₁₈H₁₅F₃N₄O₃ 392.11; found 393 (M+H)⁺. HPLC *t*_{R} 3.12 min.

**Examples 30, 32, and 45** were synthesized according to the above procedure (Scheme 16) by reacting with the appropriate starting materials.

### Example 30: (R)-(3-(2-hydroxy-1-phenylethyl)-1,2,3-oxadiazol-3-ium-5-yl)((3-(trifluoromethyl)phenyl)carbamoyl)amide.

The title compound was obtained as a white solid starting from (*R*)-2-phenylglycinol. ¹H NMR (300 MHz, DMSO-*d*₆) δ 9.72 (s, 1H), 8.35 (s, 1H), 8.25 (s, 1H), 7.70 (br d, *J =* 8.4 Hz, 1H), 7.62 (dd, *J =* 6.7, 2.9 Hz, 2H), 7.53-7.38 (m, 4H), 7.22 (d, *J =* 7.8 Hz, 1H), 6.02 (dd, *J =* 9.5, 4.2 Hz, 1H), 5.67 (t*, J* = 5.5 Hz, 1H), 4.52 (ddd, *J =* 12.0, 9.6, 5.8 Hz, 1H), 4.15-3.98 (m, 1H). LCMS (ES⁺) *m*/*z* calculated for C₁₈H₁₅F₃N₄O₃ 392.11; found 393 (M+H)⁺. HPLC *t*_{R} 3.11 min.

### Example 32: (S)-(3-(1-hydroxy-3-phenylpropan-2-yl)-1,2,3-oxadiazol-3-ium-5-yl)((3-(trifluoromethyl)phenyl)carbamoyl)amide.

The title compound was obtained as a white solid starting from Boc-*L*-Phenylalaninol. ¹H NMR (300 MHz, DMSO-*d*₆) δ 9.68 (s, 1H), 8.33 (s, 1H), 8.30-8.23 (m, 1H), 7.73-7.65 (m, 1H), 7.44 (t, *J =* 8.0 Hz, 1H), 7.34-7.16 (m, 6H), 5.89-5.16 (m, 1H), 5.10-4.94 (m, 1H), 4.05-3.94 (m, 1H), 3.93-3.84 (m, 1H), 3.34-3.27 (m, 2H). LCMS (ES+) m/z calculated for C₁₉H₁₇F₃N₄O₃ 406.13; found 407 (M+H)+. HPLC *t*_{R} 3.20 min.

### Example 45: (R)-(3-(1-amino-3-phenylpropan-2-yl)-1,2,3-oxadiazol-3-ium-5-yl)((3-(trifluoromethyl)phenyl)carbamoyl)amide.

The title compound was obtained as a white solid starting from Boc-D-Phenylalaninol. ¹H NMR (300 MHz, DMSO-*d*₆) δ 9.68 (s, 1H), 8.34 (s, 1H), 8.30-8.25 (m, 1 H), 7.74-7.65 (m, 1H), 7.44 (t, *J =* 8.0 Hz, 1H), 7.34-7.18 (m, 6H), 5.14-4.93 (m, 1H), 3.92-3.84 (m, 1H), 3.34-3.26 (m, 2H). LCMS (ES+) m/z calculated for C₁₉H₁₇F₃N₄O₃ 406.13; found 407 (M+H)⁺. HPLC *t*_{R} 3.20 min.

### Example 35: (3-(1-Phenylvinyl)-1,2,3-oxadiazol-3-ium-5-yl)((3-(trifluoromethyl)phenyl)carbamoyl)amide

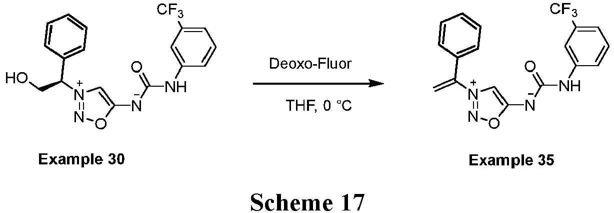

To an ice-cooled solution of **Example 30** (20 mg,0.05 mmol) in dry THF (0.25 mL, 0.003 mol) was added 1,1,1-trifluoro-*N,N*-bis(2-methoxyethyl)-14-sulfanamine (0.04 mL, 0.06 mmol) dropwise under nitrogen atmosphere. The reaction was stirred at 0 °C for 40 min. More 1,1,1-trifluoro-*N*,*N*-bis(2-methoxyethyl)-14-sulfanamine (0.04 mL, 0.06 mmol) was added and stirred for another 30 min. Then a second addition of 1,1,1-trifluoro-*N*,*N-*bis(2-methoxyethyl)-14-sulfanamine (0.07 mL) was done at 0 °C. The reaction was stirred at 0 °C for 40 min and then was allowed to warm-up slowly overnight. The reaction mixture was quenched with NaHCO₃ (0.3 mL, sat. sol.) and water and extracted with EtOAc. The organic layer was washed with brine, dried over Na₂SO₄, filtered, and concentrated under reduced pressure to give a yellow oil that was purified on silica gel column (eluent gradient from 100% petroleum ether to 70/30 petroleum ether/ethyl acetate) then reverse phase chromatography (eluent gradient 15-70% MeCN + 0.1% HCOOH). The pure fractions were combined and lyophilized to afford the title compound (1.41 mg, 8%) as a yellow solid. ¹H NMR (300 MHz, DMSO-*d*₆) δ 9.81 (br s, 1H), 8.37-8.13 (m, 2H), 7.74 (br d, *J =* 7.9 Hz, 1H), 7.66-7.37 (m, 6H), 7.23 (br d, *J =* 7.4 Hz, 1H), 6.45-6.12 (m, 2H). LCMS (ES⁺) *m*/*z* calculated for C₁₈H₁₅F₃N₄O₃ 392.11; found 393 (M+H)⁺. HPLC *t*_{R} 3.64 min.

### (3-(3-phenylprop-1-en-2-yl)-1,2,3-oxadiazol-3-ium-5-yl)((3-(trifluoromethyl)phenyl) carbamoyl)amide (Example 36) and (S)-(3-(1-fluoro-3-phenylpropan-2-yl)-1,2,3-oxadiazol-3-ium-5-yl)((3-(trifluoromethyl)phenyl)carbamoyl)amide(Example37)

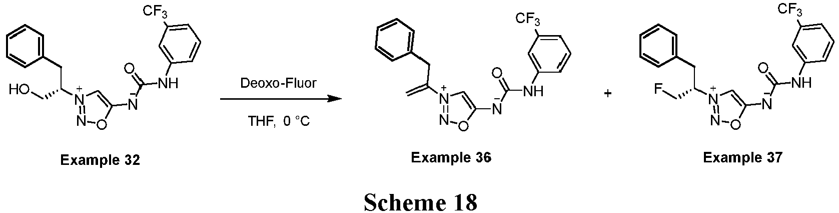

To an ice-cooled solution of **Example 32** (20 mg, 0.05 mmol) in dry THF (0.25 mL, 0.003 mol), 1,1,1-trifluoro-*N,N-*bis(2-methoxyethyl)-14-sulfanamine (0.22 mL, 0.3 mmol) was added dropwise. The reaction mixture was stirred for 40 min at 0 °C. More 1,1,1-trifluoro-*N*,*N*-bis(2-methoxyethyl)-14-sulfanamine was added (0.22 mL, 0.3 mmol) and the reaction was stirred at 0 °C for 1 hr. Then potassium fluoride (2.9 mg, 0.05 mmol) was added, and the reaction was stirred at rt overnight. The next day the reaction was quenched by water and extracted with EtOAc. The organic layer was washed additionally with water, 1 N HCl and brine, dried over Na₂SO₄, filtered, and concentrated to yield a yellow oil. The crude was purified by direct phase silica gel chromatography (eluent DCM + 0.5% MeOH). The first eluted product was additionally purified by reverse phase chromatography (eluent gradient 10-70% MeCN+0.1% HCOOH) to yield (3-(3-phenylprop-1-en-2-yl)-1,2,3-oxadiazol-3-ium-5-yl)((3-(trifluoromethyl)phenyl)carbamoyl)amide **(Example 36**) (0.4 mg, 2%) as a white powder. ¹H NMR (300 MHz, DMSO-*d*₆) δ 9.91-9.58 (m, 1H), 8.46 (s, 1H), 8.26 (br s, 1H), 7.70 (br d, *J =* 8.2 Hz, 1H), 7.52-7.15 (m, 7H), 6.28 (s, 1H), 5.75 (s, 1H), 4.13 (br s, 2H). LCMS (ES⁺) *m*/*z* calculated for C₁₉H₁₅F₃N₄O₂ 388.11; found 389 (M+H)⁺. HPLC *t*_{R} 3.66 min.

The second eluted product was additionally purified by reverse phase chromatography (eluent gradient 10-70% MeCN + 0.1% HCOOH) to yield (*S*)-(3-(1-fluoro-3-phenylpropan-2-yl)-1,2,3-oxadiazol-3-ium-5-yl)((3-(trifluoromethyl)phenyl)carbamoyl)amide **(Example 37**) (2.64 mg, 12%) as a white powder. ¹H NMR (300 MHz, DMSO-*d*₆) δ 9.73 (s, 1H), 8.43 (s, 1H), 8.27 (s, 1H), 7.70 (br d, *J=* 8.71 Hz, 1H), 7.45 (t, *J=* 7.9 Hz, 1H), 7.36-7.19 (m, 6H), 5.65-5.39 (m, 1H) , 5.18-5.03 (m, 1H), 5.01-4.86 (m, 1H), 3.45-3.34 (m, 2H); ¹⁹F NMR (282 MHz, DMSO-*d*₆) δ-224.60 (s, 1F) -61.29 (s, 3F). LCMS (ES⁺) *m*/*z* calculated for C₁₉H₁₆F₄N₄O₂ 408.12; found 409 (M+H)⁺. HPLC *t*_{R} 3.62 min.

### Example 44: (3-(2,2,2-trifluoro-1-phenylethyl)-1,2,3-oxadiazol-3-ium-5-yl)((3-(trifluoromethyl)phenyl)carbamoyl)amide

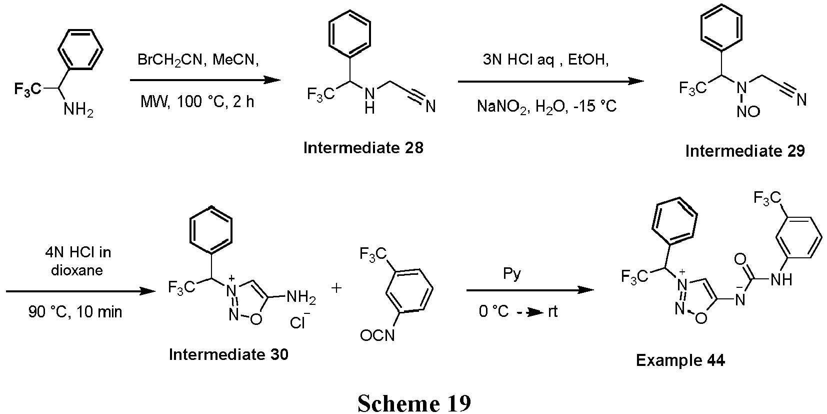

### Step 1: 2-((2,2,2-trifluoro-1-phenylethyl)amino)acetonitrile (Intermediate 28).

A microwave vail was charged with 2-((2,2,2-trifluoro-1-phenylethyl)amino)acetonitrile (0.25 mg, 1.44 mmol), *N,N*-diisopropylethylamine (0.3 mL, 1.73 mmol) and 2-bromoacetonitrile (0.07 mL, 0.96 mmol) in MeCN (2 mL). The reaction mixture was heated under microwave irradiation for 90 min at 100 °C; then more DIPEA (300 uL) and 2-bromoacetonitrile (0.07 mL,0.96 mmol) were added and the reaction was heated additionally for 30 min at 100 °C, then was directly diluted with EtOAc and washed with small amount of water and brine. The organic phase was dried over Na₂SO₄, filtered, and concentrated under reduced pressure to give dark brown oil which was purified by chromatography on silica gel (eluent DCM 100%) to give the title compound (87 mg, 28%) was as a white powder. ¹H NMR (300 MHz, DMSO-*d*₆) δ 7.87-7.26 (m, 5H), 4.50 (q, *J =* 7.7 Hz, 1H), 3.90 (br s, 1 H), 3.75-3.61 (m, 1H), 3.56-3.44 (m, 1H). LCMS (ES⁺) *m*/*z* calculated for C₁₀H₉F₃N₂ 214.07; found 215 (M+H)⁺. HPLC *t*_{R} 1.78 min.

### Step 2: N-(cyanomethyl)-N-(2,2,2-trifluoro-1-phenylethyl)nitrous amide (Intermediate 29).

The title compound was prepared using the procedure described in **Example 31,** step 2 starting from intermediate **28**. The obtained crude as a yellow oil was used as such in the next synthetic step. LCMS (ES⁺) *m*/*z* calculated for C₁₀H₈F₃N₃O243.06; found 244 (M+H)⁺. HPLC *t*_{R} 1.91 min. *Step 3: 5-amino-3-(2,2,2-trifluoro-1-phenylethyl)-1,2,3-oxadiazol-3-ium chloride (**Intermediate 30**).*

The title compound was prepared using the procedure described in **Example 32,** step 3 but heating at 90 °C for 10 min starting from intermediate **29**. The impure crude product (30 mg) was obtained as a white solid and was used as such in the next synthetic step. LCMS (ES⁺) *m*/*z* calculated for C₁₀H₉F₃N₃O⁺244.07; found 244 [M]⁺. HPLC *t*_{R} 1.09 min.

### Step 4: (3-(2, 2, 2-trifluoro-1-phenylethyl)-1,2,3-oxadiazol-3-ium-5-yl) ((3-(trifluoromethyl) phenyl)carbamoyl)amide (Example 44).

A mixture of Intermediate **30** (30 mg, 0.11 mmol) in pyridine (1 mL) was treated with 1-isocyanato-3-(trifluoromethyl)benzene (0.04 mL, 0.27 mmol) at 0 °C. The reaction was stirred at RT for 20 min; then was diluted with DCM. The organic phase was washed with water, 1 N HCl and brine; the was dried over Na₂SO₄, filtered, and concentrated under reduced pressure. The title compound (0.62 mg, 1.25%) was obtained after purification by reverse phase chromatography (MeCN / H₂O + 0.1% HCOOH). Fractions containing product were lyophilized to give the title compound as a white powder. ¹H NMR (300 MHz, DMSO-*d*₆) δ 9.87 (s, 1H), 8.36 (s, 1H), 8.18 (s, 1H), 7.75 (br d, *J=* 8.0 Hz, 3H), 7.67-7.50 (m, 4H), 7.46 (t, *J=* 8.0 Hz, 1H), 7.25 (d, *J=* 7.70 Hz, 1H). LCMS (ES⁺) *m*/*z* calculated for C₁₈H₁₂F₆N₄O₂ 430.09; found 431 (M+H)⁺. HPLC *t*_{R} 4.02 min.

### Example 70: (S)-(3-(1-(1,1'-biphenyl-4-yl)-3-acetamidopropan-2-yl)-1,2,3-oxadiazol-3-ium-5-yl)((3-(trifluoromethyl)phenyl)carbamoyl)amide

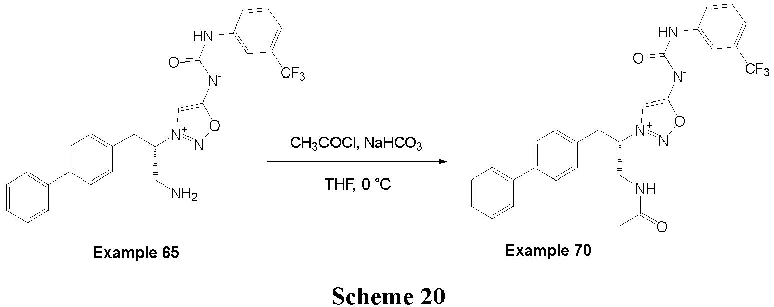

To a solution of **Example 65** (3.6 mg, 0.01 mmol) and sodium bicarbonate (0.3 mL, sat. sol.) in THF (0.5 mL) at 0 °C, acetyl chloride (10 uL) was added. The reaction was stirred for 5 min at rt, then was diluted with EtOAc and the organic phase was washed with sat. sol. KHSO₄ and brine. The organic layer was dried over Na₂SO₄, filtered, and concentrated under reduced pressure. The product was dissolved in MeCN/water and after frizz-drying the title compound was obtained (2.3 mg, 40%). ¹H NMR (300 MHz, DMSO-*d*₆) δ 9.70 (s, 1 H), 8.47 (s, 1 H), 8.21 (br t*, J =* 5.6 Hz, 1H), 7.76-7.59 (m, 6H), 7.52-7.40 (m, 4H), 7.39-7.30 (m, 4H), 7.23 (br d, *J=* 8.0 Hz, 1H), 5.12-4.93 (m, 1H), 3.89-3.76 (m, 1H), 3.74-3.60 (m, 1H), 1.79 (s, 3H). LCMS (ES⁺) *m*/*z* calculated for C₂₇H₂₄F₃N₅O₃ 523.18; found 524 (M+H)⁺. HPLC *t*_{R} 3.66 min.

### Example 71: (R)-(3-(1-([1,1'-biphenyl]-4-yl)-3-acetamidopropan-2-yl)-1,2,3-oxadiazol-3-ium-5-yl)((3-(trifluoromethyl)phenyl)carbamoyl)amide.

The title compound was synthesized according to the above procedure (Scheme 20) by reacting with the appropriate starting materials. ¹H NMR (300 MHz, DMSO-*d*₆) δ 9.70 (s, 1H), 8.47 (s, 1H), 8.21 (br t, *J =* 5.6 Hz, 1H), 7.76-7.59 (m, 6H), 7.52-7.40 (m, 4H), 7.39-7.30 (m, 4H), 7.23 (br d, *J =* 8.0 Hz, 1H), 5.12-4.93 (m, 1H), 3.89-3.76 (m, 1H), 3.74-3.60 (m, 1H), 1.79 (s, 3H). LCMS (ES⁺) *m*/*z* calculated for C₂₇H₂₄F₃N₅O₃ 523.18; found 524 (M+H)⁺. HPLC *t*_{R} 3.66 min.

### Biology

### ZIKV protease expression and purification

The sequence coding for NS2B (aa 49-95, R95A) and NS3 (aa 1-170, R29G) were synthesized (GenScript, Piscataway, New Jersey, United States), cloned in pET15b vector downstream the sequence for 6 x His tag and fused by a not cleavable linker (G₄SG₄). The protease was expressed in *E. coli* BL21 (DE3) cells: bacteria were grown in LB medium at 37 °C up to 0.8 O.D. 600, then the temperature was lowered to 20 °C and protein expression induced with 0.5 mM IPTG (cat. N. 15502, Sigma Aldrich) for 18 h. Cells were collected by centrifugation (4000 *x g,* 4 °C for 30 min), then re-suspended (7 ml/g of cell paste) in lysis buffer (25 mM Tris pH 8.0, 100 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bars by PANDA homogenizer (GEA) then cell extract was cleared by centrifugation (32914 *x* g, 4 °C, 1h), brought to 500 mM NaCl then loaded on 1 ml HisTrap HP column (cat. N. 17-52407-01, Cytiva, Sweden) equilibrated in 25 mM Tris pH 8.5, 500 mM NaCl, 5% glycerol. After three wash steps at 20, 30 and 50 mM imidazole, recombinant protein was eluted by a linear gradient up to 350 mM imidazole (cat. N. 1202, Sigma Aldrich). Fractions containing ZIKV protease were pooled and treated with 2 mM TCEP (cat. N. C4706, Sigma Aldrich) at 4 °C for 40 min, then concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 25 mM Tris pH 8.5, 5% glycerol and 1 mM DTT (cat. N. A2948, PanReac AppliChem) as mobile phase. Monomer peak fractions were collected, divided in small aliquots and frozen in liquid nitrogen.

### DEN2V protease expression and purification

The expression of DEN2V serine protease was performed as previously described (Acta Cryst. (2006). F62, 157-162), with few changes. The sequence coding for NS2B (aa 1394-1440, Uniprot Q91H74) and NS3 (aa 1476-1660, Uniprot Q91H74) were synthesized (GenScript, Piscataway, New Jersey, United States), cloned in pET15b vector downstream the sequence for 6 x His tag and fused by a not cleavable linker (G4SG4). The protease was expressed in E. coli BL21 (DE3) cells: bacteria were grown in MDG minimal not inducing medium at 25 °C for 24 h, with shaking at 300 RPM. Then the bacterial starter culture was diluted 1:20 in ZYM-5052 auto-inducing medium and let grow for additional 24 h at 25 °C with 200 RPM. Cells were collected by centrifugation (4000 x g, 4 °C for 30 min), then re-suspended (7 ml/g of cell paste) in lysis buffer (20 mM Tris pH 8.5, 50 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bar by PANDA homogenizer (GEA) then cell extract was cleared by centrifugation (32914 x g, 4 °C, 1 h), brought to 500 mM NaCl and loaded on 5 ml HisTrap HP column (cat. N. 17525501, Cytiva, Sweden) equilibrated in 20 mM Tris pH 8.5, 500 mM NaCl, 5% glycerol. After two wash steps at 10 and 20 mM imidazole, recombinant protein was eluted by a linear gradient up to 440 mM imidazole (cat. N. 1202, Sigma Aldrich). Fractions containing DEN2V protease were pooled, concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 20 mM Tris pH 8.5, 50 mM NaCl, 5 % glycerol as mobile phase. Monomer peak fractions were collected, divided in small aliquots and frozen in liquid nitrogen.

### JEV protease expression and purification

The expression of JEV serine protease was performed as previously described (PLoS ONE 7(5): e36872), with few changes. The sequence coding for NS2B (aa 51-95, JEV genotype III strain JaOArS 982, Genebank accession number: M18370) and NS3 (aa 1-180, JEV genotype III strain JaOArS 982, Genebank accession number: M18370) were synthesized (GenScript, Piscataway, New Jersey, United States), cloned in pET15b vector downstream the sequence for 6 x His tag and fused by a cleavable linker derived from NS2B sequence (aa 121-131). The protease was expressed in *E. coli* BL21 (DE3) cells: bacteria were grown in MDG minimal not inducing medium at 25 °C for 24 h, with shaking at 300 RPM. Then the bacterial starter culture was diluted 1:20 in ZYM-5052 auto-inducing medium and let grow for additional 24 h at 25 °C with 200 RPM. Cells were collected by centrifugation (4000 *xg,* 4 °C for 30 min), then re-suspended (7 ml/g of cell paste) in lysis buffer (25 mM Tris pH 8.5, 100 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bars by PANDA homogenizer (GEA) then cell extract was cleared by centrifugation (32914 *x g*, 4 °C, 1 h), brought to 500 mM NaCl then loaded on 5 ml HisTrap HP column (cat. N. 17525501, Cytiva, Sweden) equilibrated in 25 mM Tris pH 8.5, 500 mM NaCl, 5% glycerol. After two wash steps at 10 and 30 mM imidazole, recombinant protein was eluted by a linear gradient up to 500 mM imidazole (cat. N. 1202, Sigma Aldrich). Fractions containing JEV protease were pooled then treated with 2 mM TCEP (cat. N. C4706, Sigma Aldrich) at 4 °C for 40 min, finally concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 25 mM Tris pH 9.0, 5% glycerol, 1 mM TCEP as mobile phase. Monomer peak fractions were collected, divided in small aliquots and frozen in liquid nitrogen.

### ZIKV protease inhibition assay

The enzyme inhibition assay was performed in 384-well flat bottom black polystyrene microplates (cat. N. 781900, Greiner,) in a reaction volume of 20 µl. ZIKV NS2B-G₄SG₄-NS3 serine protease (MGSSHHHHHHSSGLVPRGSHMVDMYIERAGDITWEKDAEVTGNSPRLDVALDESGDF SLVEDDGPPMAGGGGSGGGGSGALWDVPAPKEVKKGETTDGVYRVMTRGLLGSTQV GVGVMQEGVFHTMWHVTKGSALRSGEGRLDPYWGDVKQDLVSYCGPWKLDAAWD GHSEVQLLAVPPGERARNIQTLPGIFKTKDGDIGAVALDYPAGTSGSPILDKCGRVIGLY GNGVVIKNGSYVSAITQGRR; SEQ. ID. No. 1) (1.25 nM) was incubated with increasing inhibitor concentrations (0.097-100 µM or 0.0009 -1 µM for the most active compounds) in assay buffer (50 mM Tris-HCl, pH 8.5, 1% glycerol, 1 mM CHAPS, 1% DMSO) for 10 minutes at 25 °C. 10 µM of Bz-Nle-KRR-AMC substrate (cat. N. 4055312, Bachem) was added and the reaction mixture incubated for additional 30 minutes at 25 °C. Product formation was evaluated by fluorescence measurement (excitation 360 nm, emission 465 nM), using a SPARK TM10 Tecan instrument. Results were analysed using Prism (GraphPad, San Diego, CA) and Vortex software (Dotmatics, Bioshops Stortford, UK). Dose-response curves were fitted by four-parameter logistic regression.

### DEN2V protease inhibition assay

The enzyme inhibition assay was performed in 384-well flat bottom black polystyrene microplates (cat. N. 781900, Greiner,) in a reaction volume of 20 µl. DEN2V NS2B-G4SG4-NS3 serine protease (MGSSHHHHHHSSGLVPRGSHMADLELERAADVRWEEQAEISGSSPILSITISED GSMSIKNEEEEQTLGGGGSGGGGAGVLWDVPSPPPVGKAELEDGAYRIKQKGILGYSQI GAGVYKEGTFHTMWHVTRGAVLMHKGKRIEPSWADVKKDLISYGGGWKLEGEWKE GEEVQVLALEPGKNPRAVQTKPGLFKTNTGTIGAVSLDFSPGTSGSPIVDKKGKVVGLY GNGVVTRSGAYVSAIAQTEKSIEDNPEIEDDIFRK; SEQ ID No 2) (10 nM) was incubated with increasing inhibitor concentrations (0.097-100 µM or 0.0009-1 µM for the most active compounds) in assay buffer (50 mM Tris-HCl, pH 8.5, 1% glycerol, 1 mM CHAPS, 1% DMSO) for 10 minutes at 25 °C. 15 µM of Bz-Nle-KRR-AMC substrate (cat. N. 4055312, Bachem) was added and the reaction mixture incubated for additional 30 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### WNV protease inhibition assay

The enzyme inhibition assay was performed in 384-well flat bottom black polystyrene microplates (cat. N. 781900, Greiner,) in a reaction volume of 20 µl. West Nile Virus (WNV) NS2B-NS3 protease (R&D, cat. N. 2907-SE-020) (2 nM) was incubated with increasing inhibitor concentrations (0.097-100 µM or 0.0009-1 µM for the most active compounds) in assay buffer (50 mM Tris-HCl, pH 9.0, 30% glycerol, 1% DMSO) for 10 minutes at 25 °C. 10 µM of pERTKR-AMC substrate (R&D, cat. N. ES013) was added and the reaction mixture incubated for additional 30 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### JEV protease inhibition assay

The enzyme inhibition assay was performed in 384-well flat bottom black polystyrene microplates (cat. N. 781900, Greiner,) in a reaction volume of 20 µl. Japanese encephalitis virus (JEV)-NS2B-NS3 protease (600 nM) was incubated with increasing inhibitor concentrations (0.097-100 µM or 0.009-1 µM for the most active compounds) in assay buffer (50 mM Tris-HCl, pH 9.0, 0.1% BSA, 0.1% Triton X-100, 1% DMSO) for 10 minutes at 25 °C. 10 µM of Pyr-RTKR-AMC substrate (Bachem, cat. N.4018149) was added and the reaction mixture incubated for additional 120 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### ZIKV replicon generation

To construct the subgenomic replicon we used the Natal RGN isolate of Asian lineage (GenBank: KU527068.1). In the replicon, the coding sequences of structural genes were removed with the exception of 31 amino acids at the N-terminus of the capsid protein fused with 32 amino acids at the C-terminus of the E protein. These sequences were retained to preserve the correct processing and translocation of NS1and of the non-structural polyprotein across the endothelium reticulum membrane. An EMCV IRES was placed after the stop codon of the polyprotein. The IRES drives the translation of a downstream fusion protein consisting of a luciferase (Nano-Luc) gene followed by the ubiquitin gene (UBI), and the neomycin phosphotransferase resistance gene (NEO). This fusion protein is required for reporting purposes and to confer resistance to G-418. The subgenomic replicon was transcribed as RNA via an upstream T7 promoter and transfected in Vero cells. The stable cell line used for the ZIKV replication assay was generated via selection with G-418.

### ZIKV replication inhibition assay and cell viability assay

A green monkey cell line (Vero) with a stable ZIKV replicon was grown and maintained in Dulbecco's modified eagle's medium (DMEM) with high glucose and pyruvate (GIBCO #41966) completed with 10% fetal bovine serum (FBS) (Gibco #10270), 1% Penicillin Streptomycin (10 mg/ml) (Gibco #14140). 0.760 µg/mL G418 solution (Sigma-Aldrich, Cat 4727894001) were used to maintain the ZIKV replication in the cells. The day of the experiment, compounds previously dissolved in 100% dimethyl sulfoxide (DMSO, Sigma-Aldrich, D5879-1L) were transferred to a 384 black plate (Greiner #781086) using an acoustic system (ATS-100 EDC). Compounds were tested in dose response. 50 µM of mycophenolic acid (Sigma-Aldrich, M3536) was used in as positive control for the ZIKV replication assay while 32 µM of Gambogic Acid (Sigma-Aldrich, G8171) was used as positive control for the cell viability assay. In both cases, in the negative control wells were added with 0.5% DMSO. 8000 cells/well were plated on the compound containing plates in 40 µL growth medium. 72 h after the treatment at 37 °C, 5% CO₂ and 90% humidity, 20 µL Nano-Glo (Promega #N1150) were added to reveal the NanoLuc signal, while 20 µL CellTiter-Glo (Promega #G7573) were added to determine the cell viability. The luminescent signal for both assays was detected reading the plate on the Envision plate reading 10 minutes post detection reagent addition (luminescence was measured at 0.5 sec/well). Data were normalized between 0 and 100% inhibition (negative control and positive control respectively). For EC₅₀ determinations, the dose-response was fitted with a 4-p logistic regression approach using the Dotmatics Studies software.

### Results

The exemplified compounds described herein were tested in the assays described above. Results from IC₅₀ calculation for inhibition of ZIKV protease and replicon and for the inhibition of Dengue Virus, West Nile Virus proteases are reported in the following Table 1, using the following letter codes: A: IC₅₀ or EC₅₀ lower than 1 µM; B: IC₅₀ or EC₅₀ from 1 µM to 5 µM; IC₅₀ or EC₅₀ higher than 5 µM; n.a.: not available.

| **Ex** | **Structure** | **Zika Protease IC₅₀ (nM)** | **Zika Replicon EC₅₀ (nM)** | **Dengue Protease IC₅₀ (nM)** | **WNV Protease IC₅₀ (nM)** | **JEV Protease IC₅₀ (nM)** |
|---|---|---|---|---|---|---|
| **1** | 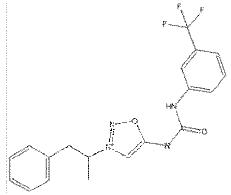 | B | C | n.a. | n.a. | n.a. |
| **2** | 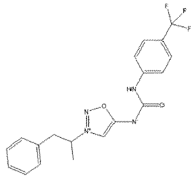 | C | C | n.a. | n.a. | n.a. |
| **3** | 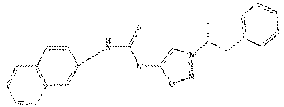 | C | C | n.a. | n.a. | n.a. |
| **4** | 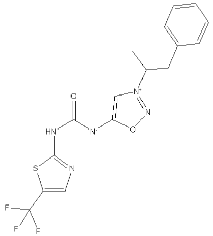 | C | C | n.a. | n.a. | n.a. |
| **5** | 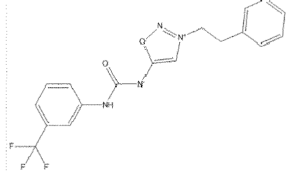 | B | A | n.a. | B | C |
| **6** | 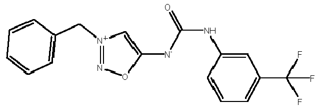 | A | B | C | C | B |
| **7** | 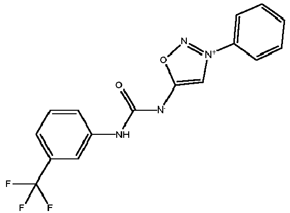 | C | C | n.a. | n.a. | n.a. |
| **8** | 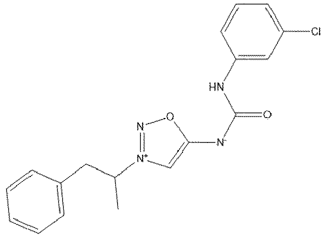 | C | C | n.a. | n.a. | n.a. |
| **9** | 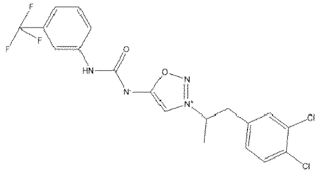 | A | C | n.a. | n.a. | n.a. |
| **10** | 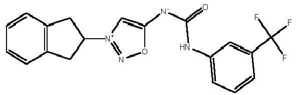 | A | C | n.a. | n.a. | n.a. |
| **11** | 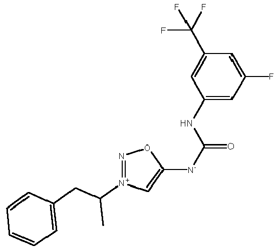 | B | C | n.a. | n.a. | n.a. |
| **12** | 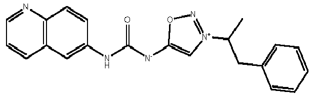 | C | C | n.a. | n.a. | n.a. |
| **13** | 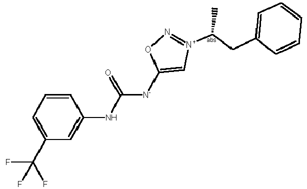 | A | B | n.a. | n.a. | n.a. |
| **14** | 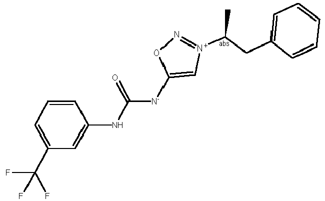 | C | C | n.a. | n.a. | n.a. |
| **15** | 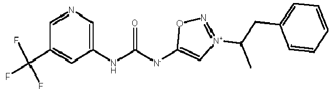 | C | C | n.a. | n.a. | n.a. |
| **16** | 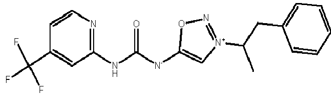 | C | C | n.a. | n.a. | n.a. |
| **17** | 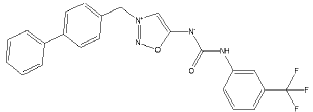 | A | A | n.a. | n.a. | n.a. |
| **18** | 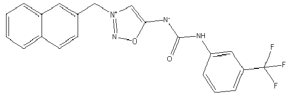 | A | B | n.a. | n.a. | n.a. |
| **19** | 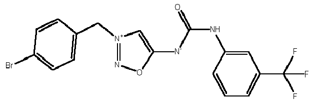 | A | A | n.a. | A | C |
| **20** | 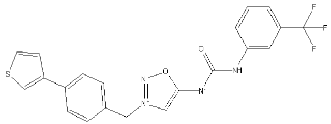 | A | A | n.a. | n.a. | n.a. |
| **21** | 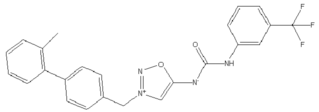 | A | A | C | n.a. | C |
| **22** | 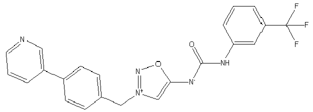 | A | A | C | A | B |
| **23** | 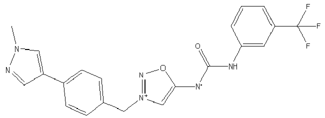 | A | A | B | A | n.a. |
| **24** | 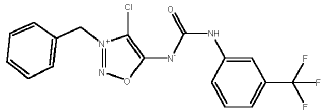 | C | C | n.a. | n.a. | n.a. |
| **25** | 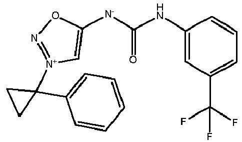 | B | C | n.a. | n.a. | n.a. |
| **26** | 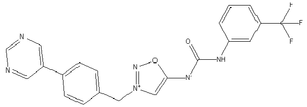 | A | A | C | A | B |
| **27** | 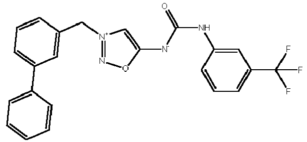 | A | B | n.a. | n.a. | C |
| **28** | 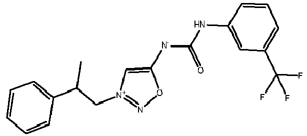 | A | A | n.a. | B | C |
| **29** | 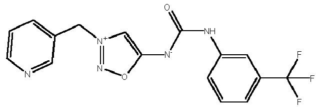 | B | B | n.a. | B | B |
| **30** | 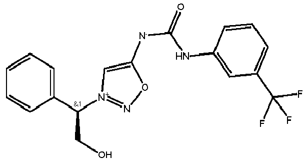 | B | B | n.a. | n.a. | C |
| **31** | 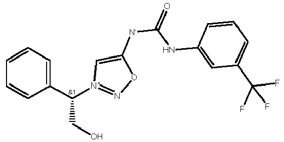 | C | C | n.a. | n.a. | n.a. |
| **32** | 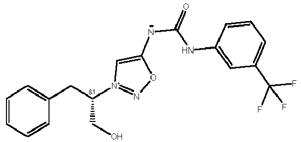 | C | C | n.a. | n.a. | n.a. |
| **33** | 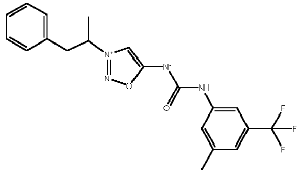 | C | C | n.a. | n.a. | n.a. |
| **34** | 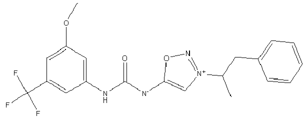 | C | C | n.a. | n.a. | n.a. |
| **35** | 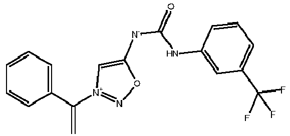 | B | C | n.a. | n.a. | n.a. |
| **36** | 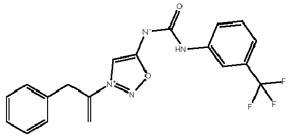 | C | C | n.a. | n.a. | n.a. |
| **37** | 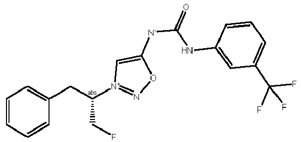 | C | C | n.a. | n.a. | n.a. |
| **38** | 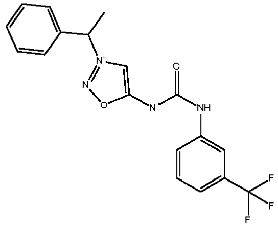 | A | B | n.a. | n.a. | C |
| **39** | 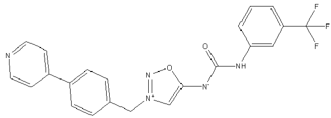 | A | A | n.a. | A | B |
| **40** | 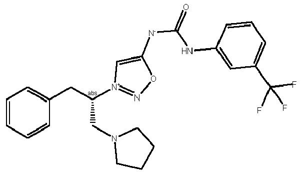 | C | C | n.a. | C | n.a. |
| **41** | 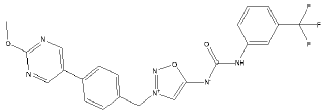 | A | A | n.a. | A | C |
| **42** | 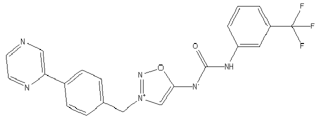 | A | A | n.a. | A | C |
| **43** | 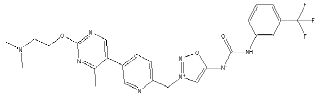 | A | A | C | n.a. | n.a. |
| **44** | 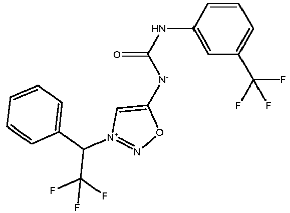 | C | C | C | C | n.a. |
| **45** | 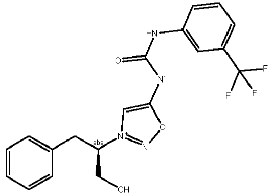 | B | C | n.a. | C | n.a. |
| **46** | 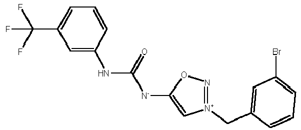 | A | B | C | B | C |
| **47** | 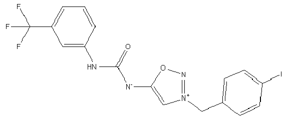 | A | A | C | A | C |
| **48** | 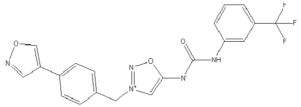 | A | A | B | A | B |
| **49** | 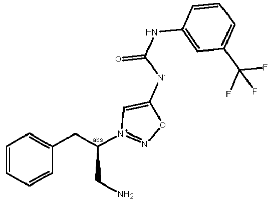 | C | C | C | n.a. | n.a. |
| **50** | 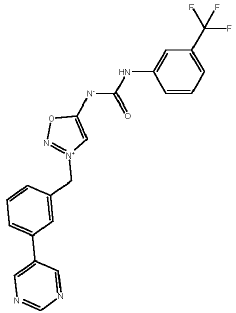 | A | A | C | A | B |
| **51** | 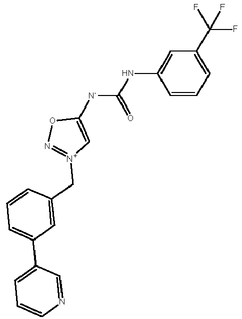 | A | A | n.a. | A | B |
| **52** | 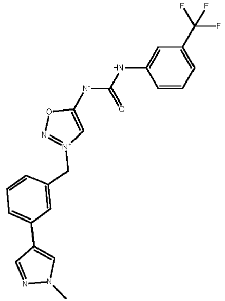 | A | A | B | A | B |
| **53** | 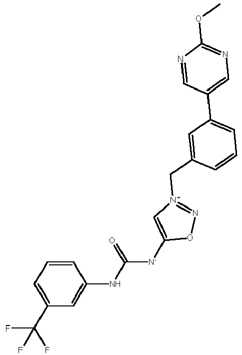 | A | A | C | A | B |
| **54** | 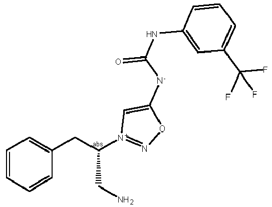 | C | C | n.a. | n.a. | n.a. |
| **55** | 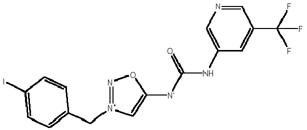 | A | A | B | A | n.a. |
| **56** | 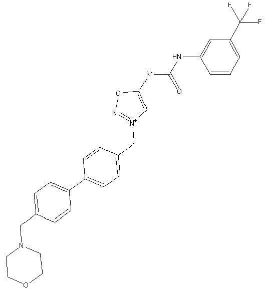 | B | A | C | A | n.a. |
| **57** | 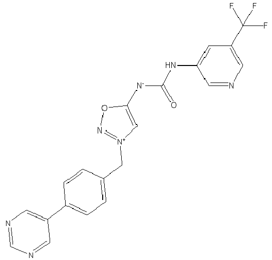 | A | A | n.a. | A | n.a. |
| **58** | 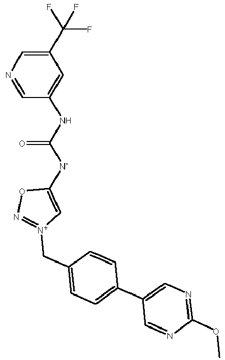 | A | A | n.a. | A | n.a. |
| **59** | 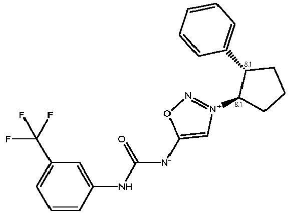 | A | B | B | B | n.a. |
| **60** | 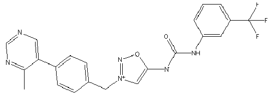 | A | A | B | A | A |
| **61** | 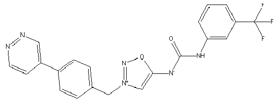 | A | A | n.a. | A | B |
| **62** | 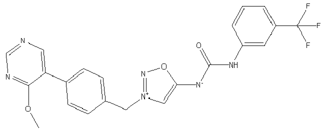 | A | A | B | A | C |
| **63** | 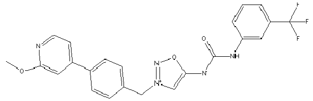 | A | A | n.a. | A | C |
| **64** | 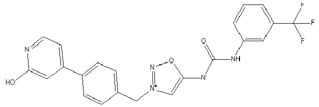 | A | A | C | A | n.a. |
| **65** | 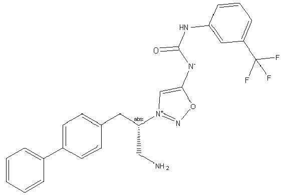 | C | C | C | n.a. | n.a. |
| **66** | 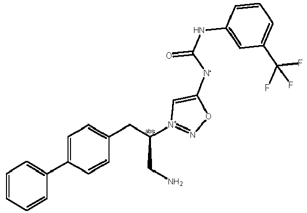 | C | C | C | n.a. | n.a. |
| **67** | 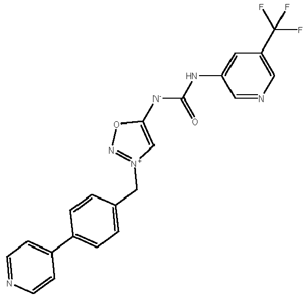 | A | A | n.a. | A | n.a. |
| **68** | 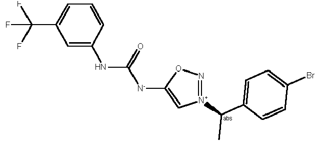 | B | B | n.a. | B | n.a. |
| **69** | 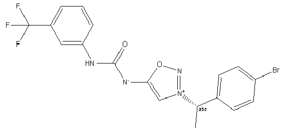 | A | A | n.a. | B | n.a. |
| **70** | 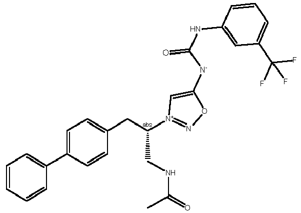 | C | C | n.a. | n.a. | n.a. |
| **71** | 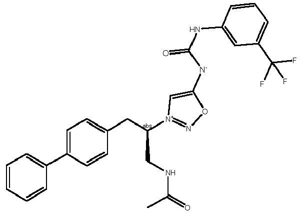 | C | C | n.a. | n.a. | n.a. |
| **72** | 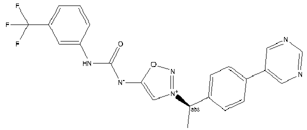 | A | A | B | A | n.a. |
| **73** | 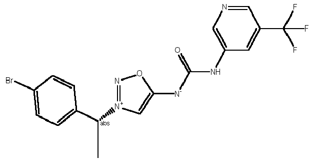 | A | B | n.a. | B | n.a. |
| **74** | 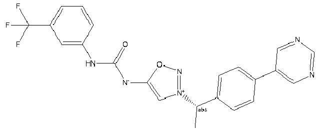 | A | A | B | A | n.a. |
| **75** | 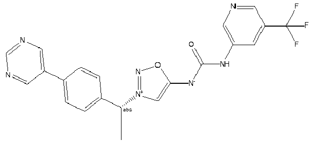 | A | A | C | A | n.a. |
| **76** | 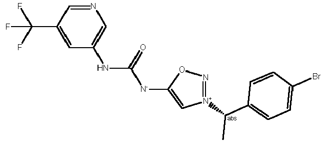 | A | A | n.a. | n.a. | n.a. |
| **77** | 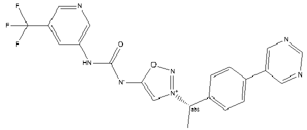 | A | A | B | n.a. | n.a. |
| **78** | 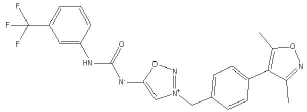 | A | A | B | n.a. | n.a. |
| **79** | 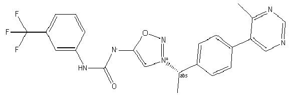 | A | A | B | n.a. | n.a. |
| **80** | 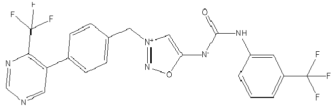 | A | A | C | n.a. | n.a. |
| **81** | 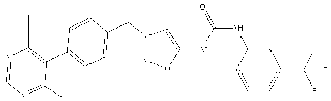 | A | A | A | n.a. | n.a. |
| **82** | 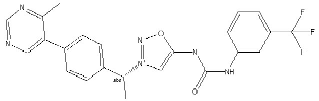 | A | A | C | n.a. | n.a. |
| **83** | 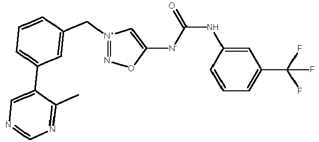 | A | A | C | n.a. | n.a. |
| **84** | 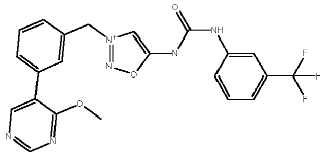 | A | A | C | n.a. | n.a. |
| **85** | 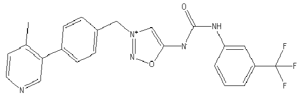 | A | A | B | n.a. | n.a. |
| **86** | 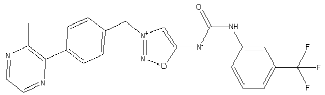 | A | A | C | n.a. | n.a. |
| **87** | 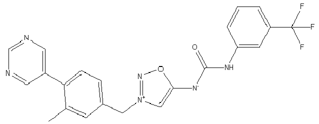 | A | A | B | n.a. | n.a. |
| **88** | 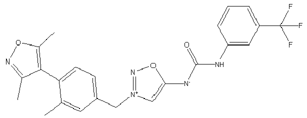 | A | A | A | n.a. | n.a. |
| **89** | 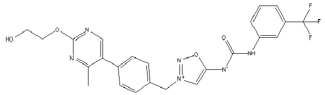 | A | A | B | n.a. | n.a. |
| **90** | 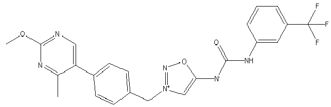 | A | A | A | n.a. | n.a. |
| **91** | 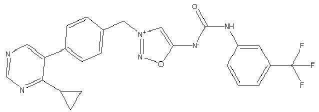 | A | A | A | n.a. | n.a. |
| **92** | 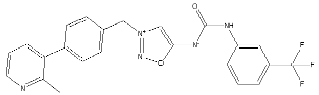 | A | A | A | n.a. | n.a. |
| **93** | 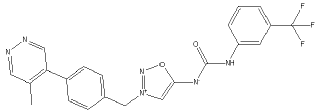 | A | A | B | n.a. | n.a. |
| **94** | 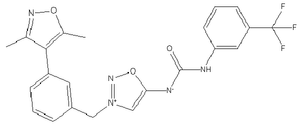 | A | A | C | n.a. | n.a. |
| **95** | 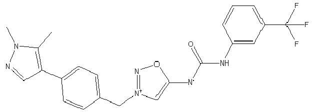 | A | A | B | n.a. | n.a. |
| **96** | 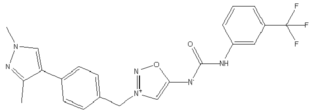 | A | A | B | n.a. | n.a. |
| **97** | 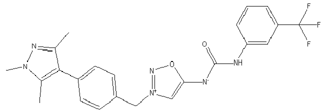 | A | A | A | n.a. | n.a. |
| **98** | 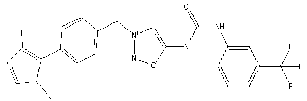 | A | A | B | n.a. | n.a. |
| **99** | 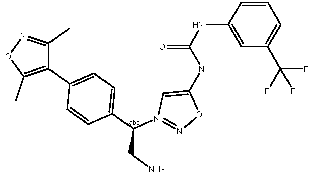 | A | A | B | n.a. | n.a. |
| **100** | 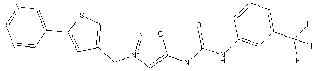 | A | A | C | n.a. | n.a. |
| **101** | 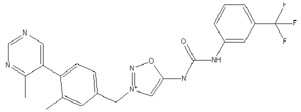 | A | A | B | n.a. | n.a. |
| **102** | 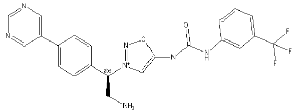 | A | A | C | n.a. | n.a. |
| **103** | 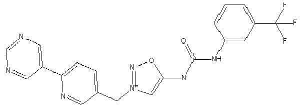 | A | A | C | n.a. | B |
| **104** | 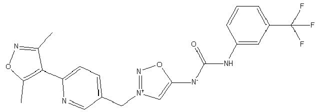 | A | A | B | n.a. | n.a. |
| **105** | 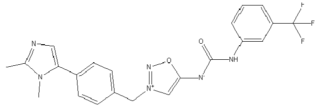 | A | A | B | n.a. | n.a. |
| **106** | 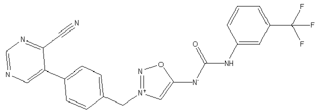 | A | A | B | n.a. | n.a. |
| **107** | 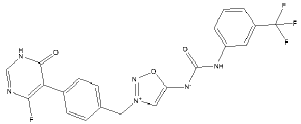 | A | B | B | n.a. | n.a. |
| **108** | 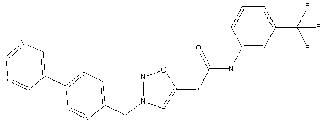 | A | A | B | n.a. | n.a. |
| **109** | 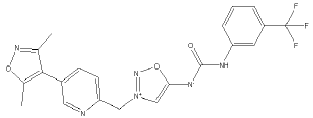 | A | A | B | n.a. | n.a. |
| **110** | 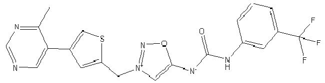 | A | A | C | n.a. | n.a. |
| **111** | 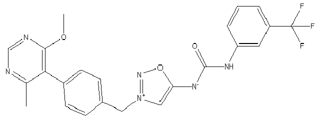 | A | A | A | n.a. | n.a. |
| **112** | 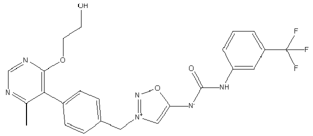 | A | A | C | n.a. | n.a. |
| **113** | 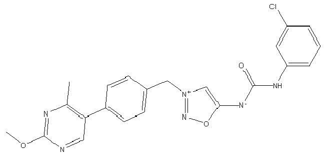 | A | A | C | n.a. | n.a. |
| **114** | 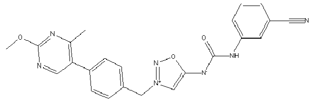 | B | B | n.a. | n.a. | n.a. |
| **115** | 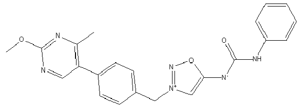 | C | C | n.a. | n.a. | n.a. |
| **116** | 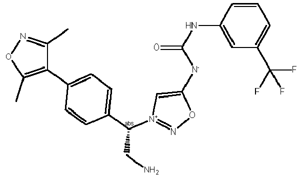 | A | A | B | n.a. | n.a. |
| **117** | 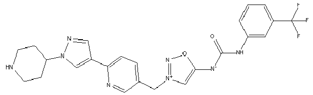 | A | C | C | n.a. | n.a. |
| **118** | 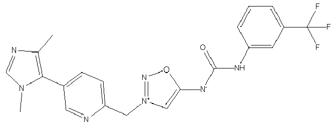 | A | A | B | n.a. | n.a. |
| **119** | 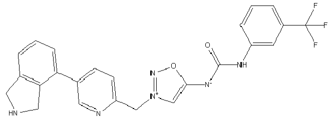 | A | A | B | n.a. | n.a. |
| **120** | 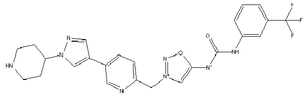 | A | C | C | n.a. | n.a. |
| **121** | 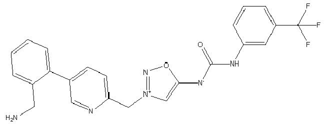 | A | A | B | n.a. | n.a. |
| **122** | 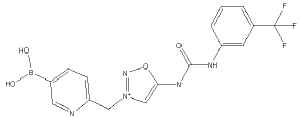 | C | C | n.a. | n.a. | n.a. |
| **123** | 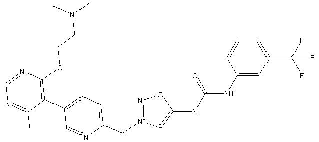 | A | A | B | n.a. | n.a. |
| **124** | 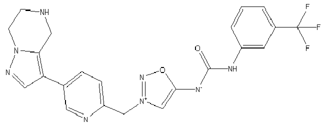 | A | A | B | n.a. | n.a. |

## Claims

1. A compound of general formula (I): 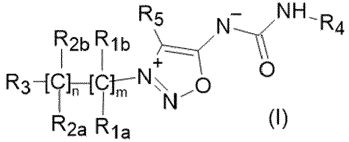 wherein:
m and n are each independently selected from 0, 1 and 2;
R₁ₐ and R_{1b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₁ₐ and R_{1b} are linked together to form a C₃₋₆-cycloalkyl ring or a C₄₋₆- heterocycloalkyl ring;
or R₁ₐ is linked to R₃ to form a partially unsaturated aromatic or heteroaromatic bicycle;
or R_{1b} doesn't exist and C-R₁ₐ is C=CH₂;
R₂ₐ and R_{2b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₂ₐ and R_{2b} are linked together to form a C₃₋₆-cycloalkyl ring or a C₄₋₆-heterocycloalkyl ring;
or any two of R₁ₐ and R₂ₐ or of R_{1b} and R_{2b} are linked together to form a C₃₋₆cycloalkyl or a C₃₋₆heterocycloalkyl;
R₃ is heterocycloalkyl, aromatic or heteroaromatic ring, each of said heterocycloalkyl, aromatic or heteroaromatic ring being optionally substituted with one or more substituents independently selected from halogen, OH, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, haloC₁₋₆alkyl, C₁₋₆alkoxy, cyano, B(OH)₂, aryl, heteroaryl wherein said aryl or heteroaryl ring are optionally substituted with one or more substituents each independently selected from halogen, hydroxy, cyano, NH₂, NHCOCH₃, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, N-methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and N-methylpyperazine;
R₄ is a ring of formula (II): 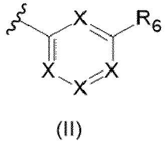
wherein
- R₆ is CF₃, F, Cl, CN or C₁₋₃alkyl;
- X is CR₇ or N;
- R₇ is at each occurrence independently selected from H, halogen, C₁₋₆alkyl, C₁₋₆alkoxy,
haloC₁₋₆alkyl, hydroxy;
or R₄ is 4-trifluoromethylphenyl, thiophene, thiazole, imidazole, pyrazole, oxazole, oxadiazole, naphthalene, quinoline, isoquinoline, quinazoline or napthyridine, wherein each of said ring is optionally substituted with one or more substituents selected from halogen, C₁₋₆alkyl, hydroxy, haloC₁₋₆alkyl, C₁₋₆alkoxy, NH₂, NHC(O)C₁₋₆alkyl, CN;
or R₄ is phenyl and R₃ is a phenyl-heteroaryl system optionally substituted with one or more substituents selected from halogen, C₁₋₆alkyl, C₁₋₆alkoxy;
R₅ is H, C₁₋₆alkyl, C₁₋₆haloalkyl or halogen;
or a pharmaceutically acceptable salt, tautomer, solvate, or stereoisomer thereof; provided that compounds indicated below are not included:

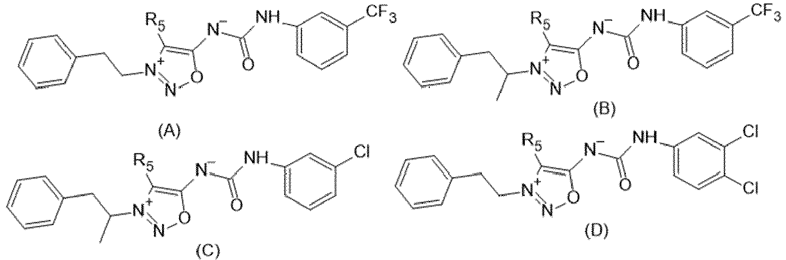

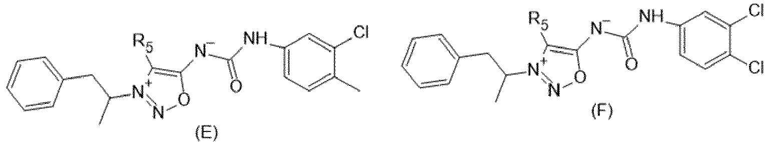

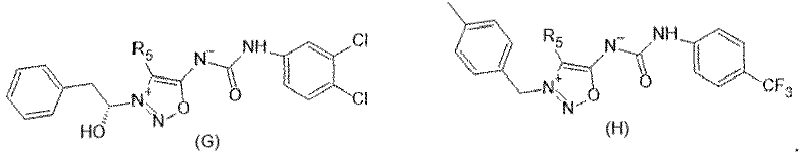

2. The compound according to claim 1 wherein:
- R_{1b} and R_{2b} are H; and/or
- m and n are each independently selected from 0, 1.

3. The compound according to any one of previous claims wherein R₃ is phenyl, naphtyl, biphenyl, phenyl-heteroaryl or bis-heteroaryl wherein each of said phenyl, naphtyl or heteroaryl ring is optionally substituted with one or more substituents each independently selected from halogen, hydroxy, cyano, B(OH)₂, NH₂, NHCOCH₃, NHCOOC₁₋₆alkyl, NHC₁₋₃alkyl, N(C₁₋₃alkyl)₂, C₁₋₆alkyl, C₁₋₆alkoxy, pyrrolidine, pyperidine, morpholine, cyclopropyl, cyclobutyl, cyclopentyl, cyclohexyl, wherein said C₁₋₆alkyl, C₁₋₆alkoxy are optionally substituted with one or more halogen, hydroxy, NH₂, N(CH₃)₂, morpholine or pyrrolidine.

4. The compound according to any one of previous claims wherein R₄ is 3-trifluoromethylphenyl, 4-trifluoromethylphenyl, naphthyl, 5-(trifluoromethyl)thiazol-2-yl, 3-chlorophenyl, 3-fluoro-5-trifluoromethylphenyl, quinolinyl, 5-(trifluoromethyl)pyridin-3-yl, 4-(trifluoromethyl)pyridin-2-yl, 3-methyl-5-trifluoromethylphenyl, 3-methoxy-5-trifluoromethylphenyl, 3-cyanophenyl.

5. A compound of general formula (I) according to any one of claims 1 to 4 selected from the following list:
- (3-(1-phenylpropan-2-yl)-1,2,3-oxadiazol-3-ium-5-yl)((4-(trifluoromethyl)phenyl) carbamoyl)amide;
- (naphthalen-2-ylcarbamoyl)(3-(1-phenylpropan-2-yl)-1,2,3-oxadiazol-3-ium-5-yl)amide;
- (3-(1-phenylpropan-2-yl)-1,2,3-oxadiazol-3-ium-5-yl)((5-(trifluoromethyl)thiazol-2-yl)carbamoyl)amide;
- (3-benzyl-1,2,3-oxadiazol-3-ium-5-yl)((3-(trifluoromethyl)phenyl)carbamoyl)amide;
- (3-phenyl-1,2,3-oxadiazol-3-ium-5-yl)((3-(trifluoromethyl)phenyl)carbamoyl)amide;
- ((3-chlorophenyl)carbamoyl)(3-(1-phenylpropan-2-yl)-1,2,3-oxadiazol-3-ium-5-yl)amide
- (3-(1-(3,4-dichlorophenyl)propan-2-yl)-1,2,3-oxadiazol-3-ium-5-yl)((3-(trifluoromethyl) phenyl)carbamoyl)amide;
- (3-(2,3-dihydro-1*H*-inden-2-yl)-1,2,3-oxadiazol-3-ium-5-yl)((3-(trifluoromethyl)phenyl) carbamoyl)amide;
- ((3-fluoro-5-(trifluoromethyl)phenyl)carbamoyl)(3-(1-phenylpropan-2-yl)-1,2,3-oxadiazol-3-ium-5-yl)amide;
- (3-(1-phenylpropan-2-yl)-1,2,3-oxadiazol-3-ium-5-yl)(quinolin-6-ylcarbamoyl)amide;
- (*R*)-(3-(1-phenylpropan-2-yl)-1,2,3 -oxadiazol -3 -ium-5 -yl)((3 -(trifluoromethyl)phenyl) carbamoyl)amide;
- (*S*)-(3-(1-phenylpropan-2-yl)-1,2,3-oxadiazol-3 -ium-5-yl)((3-(trifluoromethyl)phenyl) carbamoyl)amide;
- (3-(1-phenylpropan-2-yl)-1,2,3-oxadiazol-3-ium-5-yl)((5-(trifluoromethyl)pyridin-3-yl)carbamoyl)amide;
- (3-(1-phenylpropan-2-yl)-1,2,3-oxadiazol-3-ium-5-yl)((4-(trifluoromethyl)pyridin-2-yl)carbamoyl)amide;
- (3-([1,1'-biphenyl]-4-ylmethyl)-1,2,3-oxadiazol-3 -ium-5-yl)((3-(trifluoromethyl)phenyl) carbamoyl)amide;
- (3-(naphthalen-2-ylmethyl)-1,2,3-oxadiazol-3-ium-5-yl)((3-(trifluoromethyl)phenyl) carbamoyl)amide;
- (3-(4-bromobenzyl)-1,2,3-oxadiazol-3-ium-5-yl)((3-(trifluoromethyl)phenyl)carbamoyl) amide;
- (3-(4-(thiophen-3-yl)benzyl)-1,2,3-oxadiazol-3 -ium-5-yl)((3-(trifluoromethyl)phenyl) carbamoyl)amide;
- (3-((2'-methyl-[1,1'-biphenyl]-4-yl)methyl)-1,2,3-oxadiazol-3 -ium-5-yl)((3-(trifluoromethyl)phenyl)carbamoyl)amide;
- (3-(4-(pyridin-3-yl)benzyl)-1,2,3-oxadiazol-3-ium-5-yl)((3-(trifluoromethyl)phenyl) carbamoyl)amide;
- (3-(4-(1-methyl-1*H*-pyrazol-4-yl)benzyl)-1,2,3-oxadiazol-3 -ium-5-yl)((3-(trifluoromethyl)phenyl)carbamoyl)amide;
- (3-benzyl-4-chloro-1,2,3-oxadiazol-3-ium-5-yl)((3-(trifluoromethyl)phenyl)carbamoyl) amide;
- (3-(1-phenylcyclopropyl)-1,2,3-oxadiazol-3-ium-5 -yl)((3 -(trifluoromethyl)phenyl) carbamoyl)amide;
- (3-(4-(pyrimidin-5-yl)benzyl)-1,2,3-oxadiazol-3 -ium-5-yl)((3-(trifluoromethyl)phenyl) carbamoyl)amide;
- (3-([1,1'-biphenyl]-3-ylmethyl)-1,2,3-oxadiazol-3 -ium-5-yl)((3-(trifluoromethyl)phenyl) carbamoyl)amide;
- (3-(2-phenylpropyl)-1,2,3-oxadiazol-3-ium-5-yl)((3-(trifluoromethyl)phenyl)carbamoyl) amide;
- (3-(pyridin-3-ylmethyl)-1,2,3-oxadiazol-3-ium-5-yl)((3-(trifluoromethyl)phenyl) carbamoyl)amide;
- (*R*)-(3-(2-hydroxy-1-phenylethyl)-1,2,3-oxadiazol-3-ium-5-yl)((3-(trifluoromethyl) phenyl)carbamoyl)amide;
- (*S*)-(3-(2-hydroxy-1-phenylethyl)-1,2,3-oxadiazol-3-ium-5-yl)((3-(trifluoromethyl) phenyl)carbamoyl)amide;
- (*R*)-(3-(1-hydroxy-3-phenylpropan-2-yl)-1,2,3-oxadiazol-3-ium-5 -yl)((3 - (trifluoromethyl)phenyl)carbamoyl)amide;
- ((3-methyl-5-(trifluoromethyl)phenyl)carbamoyl)(3-(1-phenylpropan-2-yl)-1,2,3-oxadiazol-3 -ium-5 -yl)amide;
- ((3-methoxy-5-(trifluoromethyl)phenyl)carbamoyl)(3-(1-phenylpropan-2-yl)-1,2,3-oxadiazol-3-ium-5-yl)amide;
- (3-(1-phenylvinyl)-1,2,3-oxadiazol-3-ium-5-yl)((3-(trifluoromethyl)phenyl)carbamoyl) amide;
- (3-(3-phenylprop-1-en-2-yl)-1,2,3 -oxadiazol -3 -ium-5 -yl)((3-(trifluoromethyl)phenyl) carbamoyl)amide;
- (*S*)-(3-(1-fluoro-3 -phenylpropan-2-yl)-1,2,3 -oxadiazol-3 -ium-5 -yl)((3 -(trifluoromethyl) phenyl)carbamoyl)amide;
- (3-(1-phenylethyl)-1,2,3-oxadiazol-3-ium-5-yl)((3-(trifluoromethyl)phenyl)carbamoyl) amide;
- (3-(4-(pyridin-4-yl)benzyl)-1,2,3-oxadiazol-3-ium-5-yl)((3-(trifluoromethyl)phenyl) carbamoyl)amide;
- (*S*)-(3-(1-phenyl-3-(pyrrolidin-1-yl)propan-2-yl)-1,2,3-oxadiazol-3-ium-5-yl)((3-(trifluoromethyl)phenyl)carbamoyl)amide;
- (3-(4-(2-methoxypyrimidin-5-yl)benzyl)-1,2,3-oxadiazol-3-ium-5-yl)((3-(trifluoromethyl)phenyl)carbamoyl)amide;
- (3-(4-(pyrazin-2-yl)benzyl)-1,2,3-oxadiazol-3-ium-5-yl)((3-(trifluoromethyl)phenyl) carbamoyl)amide;
- (3-(2,2,2-trifluoro-1-phenylethyl)-1,2,3-oxadiazol-3-ium-5-yl)((3-(trifluoromethyl) phenyl)carbamoyl)amide;
- (*R*)-(3-(1-hydroxy-3-phenylpropan-2-yl)-1,2,3 -oxadiazol-3 -ium-5 -yl)((3 - (trifluoromethyl)phenyl)carbamoyl)amide;
- (3-(3-bromobenzyl)-1,2,3-oxadiazol-3-ium-5-yl)((3-(trifluoromethyl)phenyl)carbamoyl) amide;
- (3-(4-iodobenzyl)-1,2,3-oxadiazol-3-ium-5-yl)((3-(trifluoromethyl)phenyl)carbamoyl) amide;
- (3-(4-(isoxazol-4-yl)benzyl)-1,2,3-oxadiazol-3-ium-5-yl)((3-(trifluoromethyl)phenyl) carbamoyl)amide;
- (*R*)-(3-(1-amino-3-phenylpropan-2-yl)-1,2,3 -oxadiazol-3 -ium-5 -yl)((3 -(trifluoromethyl) phenyl)carbamoyl)amide;
- (3-(3-(pyrimidin-5-yl)benzyl)-1,2,3-oxadiazol-3 -ium-5-yl)((3-(trifluoromethyl)phenyl) carbamoyl)amide;
- (3-(3-(pyridin-3-yl)benzyl)-1,2,3-oxadiazol-3-ium-5-yl)((3-(trifluoromethyl)phenyl) carbamoyl)amide;
- (3 -(3 -(1-methyl-1*H*-pyrazol-4-yl)benzyl)-1,2,3 -oxadiazol-3 -ium-5 -yl)((3 - (trifluoromethyl)phenyl)carbamoyl)amide;
- (3-(3-(2-methoxypyrimidin-5-yl)benzyl)-1,2,3-oxadiazol-3-ium-5-yl)((3-(trifluoromethyl)phenyl)carbamoyl)amide;
- (*S*)-(3-(1-amino-3-phenylpropan-2-yl)-1,2,3-oxadiazol-3-ium-5 -yl)((3 -(trifluoromethyl) phenyl)carbamoyl)amide;
- (3-(4-iodobenzyl)-1,2,3-oxadiazol-3-ium-5-yl)((5-(trifluoromethyl)pyridin-3-yl) carbamoyl)amide;
- (3-((4'-(morpholinomethyl)-[1,1'-biphenyl]-4-yl)methyl)-1,2,3-oxadiazol-3 -ium-5-yl)((3-(trifluoromethyl)phenyl)carbamoyl)amide;
- (3-(4-(pyrimidin-5-yl)benzyl)-1,2,3-oxadiazol-3 -ium-5-yl)((5-(trifluoromethyl)pyridin-3-yl)carbamoyl)amide;
- (3-(4-(2-methoxypyrimidin-5-yl)benzyl)-1,2,3-oxadiazol-3-ium-5-yl)((5-(trifluoromethyl)pyridin-3-yl)carbamoyl)amide;
- (3-((1*R*,2*S*)-2-phenylcyclopentyl)-1,2,3-oxadiazol-3-ium-5-yl)((3-(trifluoromethyl) phenyl)carbamoyl)amide;
- (3-(4-(4-methylpyrimidin-5-yl)benzyl)-1,2,3-oxadiazol-3 -ium-5-yl)((3-(trifluoromethyl) phenyl)carbamoyl)amide;
- (3-(4-(pyridazin-4-yl)benzyl)-1,2,3-oxadiazol-3 -ium-5-yl)((3-(trifluoromethyl)phenyl) carbamoyl)amide;
- (3-(4-(4-methoxypyrimidin-5-yl)benzyl)-1,2,3-oxadiazol-3-ium-5-yl)((3-(trifluoromethyl)phenyl)carbamoyl)amide;
- (3-(4-(2-methoxypyridin-4-yl)benzyl)-1,2,3-oxadiazol-3 -ium-5-yl)((3-(trifluoromethyl) phenyl)carbamoyl)amide;
- (3-(4-(2-hydroxypyridin-4-yl)benzyl)-1,2,3-oxadiazol-3-ium-5-yl)((3-(trifluoromethyl) phenyl)carbamoyl)amide;
- (*S*)-(3-(1-([1,1'-biphenyl]-4-yl)-3 -aminopropan-2-yl)-1,2,3-oxadiazol-3-ium-5-yl)((3-(trifluoromethyl)phenyl)carbamoyl)amide;
- (*R*)-(3-(1-([1,1'-biphenyl]-4-yl)-3-aminopropan-2-yl)-1,2,3-oxadiazol-3-ium-5-yl)((3-(trifluoromethyl)phenyl)carbamoyl)amide;
- (3-(4-(pyridin-4-yl)benzyl)-1,2,3-oxadiazol-3-ium-5-yl)((5-(trifluoromethyl)pyridin-3-yl)carbamoyl)amide;
- (*R*)-(3-(1-(4-bromophenyl)ethyl)-1,2,3 -oxadiazol-3 -ium-5 -yl)((3 -(trifluoromethyl) phenyl)carbamoyl)amide;
- (*S*)-(3-(1-(4-bromophenyl)ethyl)-1,2,3-oxadiazol-3-ium-5 -yl)((3 -(trifluoromethyl) phenyl)carbamoyl)amide;
- (*S*)-(3-(1-([1,1'-biphenyl]-4-yl)-3 -acetamidopropan-2-yl)-1,2,3-oxadiazol-3 -ium-5-yl)((3-(trifluoromethyl)phenyl)carbamoyl)amide;
- (*R*)-(3-(1-([1,1'-biphenyl]-4-yl)-3-acetamidopropan-2-yl)-1,2,3-oxadiazol-3 -ium-5-yl)((3-(trifluoromethyl)phenyl)carbamoyl)amide;
- (*R*)-(3 -(1-(4-(pyrimidin-5-yl)phenyl)ethyl)-1,2,3-oxadiazol-3-ium-5 -yl)((3 - (trifluoromethyl)phenyl)carbamoyl)amide;
- (*R*)-(3-(1-(4-bromophenyl)ethyl)-1,2,3 -oxadiazol-3 -ium-5 -yl)((5 -(trifluoromethyl) pyridin-3-yl)carbamoyl)amide;
- (*S*)-(3-(1-(4-(pyrimidin-5-yl)phenyl)ethyl)-1,2,3-oxadiazol-3-ium-5 -yl)((3 - (trifluoromethyl)phenyl)carbamoyl)amide;
- (*R*)-(3-(1-(4-(pyrimidin-5-yl)phenyl)ethyl)-1,2,3-oxadiazol-3 -ium-5-yl)((5-(trifluoromethyl)pyridin-3-yl)carbamoyl)amide;
- (*S*)-(3-(1-(4-bromophenyl)ethyl)-1,2,3-oxadiazol-3-ium-5 -yl)((5 -(trifluoromethyl) pyridin-3-yl)carbamoyl)amide;
- (*S*)-(3-(1-(4-(pyrimidin-5-yl)phenyl)ethyl)-1,2,3-oxadiazol-3 -ium-5-yl)((5-(trifluoromethyl)pyridin-3-yl)carbamoyl)amide;
- (3-(4-(3,5-dimethylisoxazol-4-yl)benzyl)-1,2,3-oxadiazol-3 -ium-5-yl)((3-(trifluoromethyl)phenyl)carbamoyl)amide;
- (*S*)-(3-(1-(4-(4-methylpyrimidin-5-yl)phenyl)ethyl)-1,2,3-oxadiazol-3-ium-5 -yl)((3 - (trifluoromethyl)phenyl)carbamoyl)amide;
- ((3-(trifluoromethyl)phenyl)carbamoyl)(3-(4-(4-(trifluoromethyl)pyrimidin-5-yl)benzyl)-1,2,3-oxadiazol-3-ium-5-yl)amide;
- (3-(4-(4,6-dimethylpyrimidin-5-yl)benzyl)-1,2,3-oxadiazol-3 -ium-5-yl)((3-(trifluoromethyl)phenyl)carbamoyl)amide;
- (R)-(3 -(1-(4-(4-methylpyrimidin-5-yl)phenyl)ethyl)-1,2,3-oxadiazol-3-ium-5 -yl)((3 - (trifluoromethyl)phenyl)carbamoyl)amide;
- (3-(3-(4-methylpyrimidin-5-yl)benzyl)-1,2,3-oxadiazol-3 -ium-5-yl)((3-(trifluoromethyl) phenyl)carbamoyl)amide;
- (3-(3-(4-methoxypyrimidin-5-yl)benzyl)-1,2,3-oxadiazol-3-ium-5-yl)((3-(trifluoromethyl)phenyl)carbamoyl)amide;
- (3-(4-(4-methylpyridin-3-yl)benzyl)-1,2,3-oxadiazol-3-ium-5-yl)((3-(trifluoromethyl) phenyl)carbamoyl)amide;
- (3-(4-(3-methylpyrazin-2-yl)benzyl)-1,2,3-oxadiazol-3-ium-5-yl)((3-(trifluoromethyl) phenyl)carbamoyl)amide;
- (3-(3-methyl-4-(pyrimidin-5-yl)benzyl)-1,2,3-oxadiazol-3-ium-5-yl)((3-(trifluoromethyl) phenyl)carbamoyl)amide;
- (3-(4-(3,5-dimethylisoxazol-4-yl)-3 -methylbenzyl)-1,2,3-oxadiazol-3 -ium-5-yl)((3-(trifluoromethyl)phenyl)carbamoyl)amide;
- (3-(4-(2-(2-hydroxyethoxy)-4-methylpyrimidin-5-yl)benzyl)-1,2,3-oxadiazol-3-ium-5-yl)((3-(trifluoromethyl)phenyl)carbamoyl)amide;
- (3-(4-(2-methoxy-4-methylpyrimidin-5-yl)benzyl)-1,2,3-oxadiazol-3 -ium-5-yl)((3-(trifluoromethyl)phenyl)carbamoyl)amide;
- (3-(4-(4-cyclopropylpyrimidin-5-yl)benzyl)-1,2,3-oxadiazol-3 -ium-5-yl)((3-(trifluoromethyl)phenyl)carbamoyl)amide;
- (3-(4-(2-methylpyridin-3-yl)benzyl)-1,2,3-oxadiazol-3-ium-5-yl)((3-(trifluoromethyl) phenyl)carbamoyl)amide;
- (3-(4-(5-methylpyridazin-4-yl)benzyl)-1,2,3-oxadiazol-3 -ium-5-yl)((3-(trifluoromethyl) phenyl)carbamoyl)amide;
- (3-(3-(3,5-dimethylisoxazol-4-yl)benzyl)-1,2,3-oxadiazol-3-ium-5-yl)((3-(trifluoromethyl)phenyl)carbamoyl)amide;
- (3-(4-(1,5-dimethyl-1*H*-pyrazol-4-yl)benzyl)-1,2,3-oxadiazol-3-ium-5-yl)((3-(trifluoromethyl)phenyl)carbamoyl)amide;
- (3-(4-(1,3-dimethyl-1*H*-pyrazol-4-yl)benzyl)-1,2,3-oxadiazol-3-ium-5-yl)((3-(trifluoromethyl)phenyl)carbamoyl)amide;
- ((3 -(trifluoromethyl)phenyl)carbamoyl)(3 -(4-(1,3 ,5 -trimethyl-1*H*-pyrazol-4-yl)benzyl)-1,2,3-oxadiazol-3-ium-5-yl)amide;
- (3-(4-(1,4-dimethyl-1*H*-imidazol-5-yl)benzyl)-1,2,3-oxadiazol-3-ium-5-yl)((3-(trifluoromethyl)phenyl)carbamoyl)amide;
- (*R*)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide;
- (3-((5-(pyrimidin-5-yl)thiophen-3-yl)methyl)-1,2,3-oxadiazol-3-ium-5-yl)((3-(trifluoromethyl)phenyl)carbamoyl)amide;
- (3-(3-methyl-4-(4-methylpyrimidin-5-yl)benzyl)-1,2,3-oxadiazol-3-ium-5-yl)((3-(trifluoromethyl)phenyl)carbamoyl)amide;
- (*R*)-(3-(2-amino-1-(4-(pyrimidin-5-yl)phenyl)ethyl)-1,2,3 -oxadiazol-3 -ium-5 -yl)((3 - (trifluoromethyl)phenyl)carbamoyl)amide;
- (3-((6-(pyrimidin-5-yl)pyridin-3-yl)methyl)-1,2,3-oxadiazol-3-ium-5-yl)((3-(trifluoromethyl)phenyl)carbamoyl)amide;
- (3-((6-(3,5-dimethylisoxazol-4-yl)pyridin-3-yl)methyl)-1,2,3-oxadiazol-3-ium-5-yl)((3-(trifluoromethyl)phenyl)carbamoyl)amide;
- (3-(4-(1,2-dimethyl-1*H*-imidazol-5-yl)benzyl)-1,2,3-oxadiazol-3-ium-5-yl)((3-(trifluoromethyl)phenyl)carbamoyl)amide;
- (3-(4-(4-cyanopyrimidin-5-yl)benzyl)-1,2,3-oxadiazol-3-ium-5-yl)((3-(trifluoromethyl)phenyl)carbamoyl)amide;
- (3-(4-(4-fluoro-6-oxo-1,6-dihydropyrimidin-5-yl)benzyl)-1,2,3-oxadiazol-3-ium-5 - yl)((3-(trifluoromethyl)phenyl)carbamoyl)amide;
- (3-((5-(pyrimidin-5-yl)pyridin-2-yl)methyl)-1,2,3-oxadiazol-3-ium-5-yl)((3-(trifluoromethyl)phenyl)carbamoyl)amide;
- (3-((5-(3,5-dimethylisoxazol-4-yl)pyridin-2-yl)methyl)-1,2,3-oxadiazol-3-ium-5-yl)((3-(trifluoromethyl)phenyl)carbamoyl)amide;
- (3-((4-(4-methylpyrimidin-5-yl)thiophen-2-yl)methyl)-1,2,3-oxadiazol-3-ium-5-yl)((3-(trifluoromethyl)phenyl)carbamoyl)amide;
- (3-(4-(4-methoxy-6-methylpyrimidin-5-yl)benzyl)-1,2,3-oxadiazol-3-ium-5-yl)((3-(trifluoromethyl)phenyl)carbamoyl)amide;
- (3-(4-(4-(2-hydroxyethoxy)-6-methylpyrimidin-5-yl)benzyl)-1,2,3-oxadiazol-3-ium-5-yl)((3-(trifluoromethyl)phenyl)carbamoyl)amide;
- ((3-chlorophenyl)carbamoyl)(3-(4-(2-methoxy-4-methylpyrimidin-5-yl)benzyl)-1,2,3-oxadiazol-3 -ium-5 -yl)amide;
- ((3-cyanophenyl)carbamoyl)(3-(4-(2-methoxy-4-methylpyrimidin-5-yl)benzyl)-1,2,3-oxadiazol-3 -ium-5-yl);
- (3-(4-(2-methoxy-4-methylpyrimidin-5-yl)benzyl)-1,2,3-oxadiazol-3-ium-5-yl)(phenylcarbamoyl)amide amide;
- (*S*)-(3-(2-amino-1-(4-(3,5-dimethylisoxazol-4-yl)phenyl)ethyl)-1,2,3-oxadiazol-3-ium-5-yl)((3-(trifluoromethyl)phenyl)carbamoyl)amide;
- (3-((6-(1-(piperidin-4-yl)-1*H*-pyrazol-4-yl)pyridin-3-yl)methyl)-1,2,3-oxadiazol-3 -ium-5-yl)((3-(trifluoromethyl)phenyl)carbamoyl)amide;
- (3-((5-(1,4-dimethyl-1*H*-imidazol-5-yl)pyridin-2-yl)methyl)-1,2,3-oxadiazol-3-ium-5-yl)((3-(trifluoromethyl)phenyl)carbamoyl)amide;
- (3-((5-(isoindolin-4-yl)pyridin-2-yl)methyl)-1,2,3-oxadiazol-3 -ium-5-yl)((3-(trifluoromethyl)phenyl)carbamoyl)amide;
- (3-((5-(1-(piperidin-4-yl)-1*H*-pyrazol-4-yl)pyridin-2-yl)methyl)-1,2,3-oxadiazol-3 -ium-5-yl)((3-(trifluoromethyl)phenyl)carbamoyl)amide;
- (3-((5-(2-(aminomethyl)phenyl)pyridin-2-yl)methyl)-1,2,3-oxadiazol-3-ium-5-yl)((3-(trifluoromethyl)phenyl)carbamoyl)amide;
- (3-((5-boronopyridin-2-yl)methyl)-1,2,3-oxadiazol-3-ium-5-yl)((3-(trifluoromethyl)phenyl)carbamoyl)amide;
- (3-((5-(4-(2-(dimethylamino)ethoxy)-6-methylpyrimidin-5-yl)pyridin-2-yl)methyl)-1,2,3-oxadiazol-3-ium-5-yl)((3-(trifluoromethyl)phenyl)carbamoyl)amide;
- (3-((5-(4,5,6,7-tetrahydropyrazolo[1,5-a]pyrazin-3 -yl)pyridin-2-yl)methyl)-1,2,3-oxadiazol-3-ium-5-yl)((3-(trifluoromethyl)phenyl)carbamoyl)amide;
- (3-((5-(2-(2-(dimethylamino)ethoxy)-4-methylpyrimidin-5-yl)pyridin-2-yl)methyl)-1,2,3-oxadiazol-3-ium-5-yl)((3-(trifluoromethyl)phenyl)carbamoyl)amide.

6. The compound according to any one of claims 1 to 5 being an inhibitor of NS2B-NS3 protease of a Flavivirus, preferably an inhibitor of the NS2B-NS3 protease of Zika and/or Dengue and/or West Nile and/or Japan Encephalitis virus.

7. The compound according to any one of previous claims for medical use.

8. The compound according to claim 7 for use in treatment and/or prevention of a Flavivirus infection, preferably wherein the Flavivirus is selected from the group consisting of Dengue, West Nile, Zika and Japan Encephalitis virus.

9. A compound of general Formula (I): 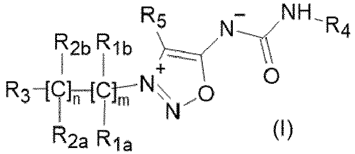 wherein:
m and n are each independently selected from 0, 1 and 2;
R₁ₐ and R_{1b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₁ₐ and R_{1b} are linked together to form a C₃₋₆-cycloalkyl ring or a C₄₋₆-heterocycloalkyl ring;
or R₁ₐ is linked to R₃ to form a partially unsaturated aromatic or heteroaromatic bicycle;
or R_{1b} doesn't exist and C-R₁ₐ is C=CH₂;
R₂ₐ and R_{2b} are independently selected from H and C₁₋₆alkyl optionally substituted with one or more substituents selected from: OH, OC₁₋₃alkyl, halogen, NH₂, NHCOCH₃, NHCOOC₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₂ₐ and R_{2b} are linked together to form a C₃₋₆-cycloalkyl ring or a C₄₋₆- heterocycloalkyl ring;
or any two of R₁ₐ and R₂ₐ or of R_{1b} and R_{2b} are linked together to form a C₃₋₆cycloalkyl or a C₃₋₆heterocycloalkyl;
R₃ is heterocycloalkyl, aromatic or heteroaromatic ring, each of said heterocycloalkyl, aromatic or heteroaromatic ring being optionally substituted with one or more substituents independently selected from halogen, OH, NH₂, NHCOCH₃, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, haloC₁₋₆alkyl, C₁₋₆alkoxy, cyano, B(OH)₂, aryl, heteroaryl wherein said aryl or heteroaryl ring are optionally substituted with one or more substituents each independently selected from halogen, hydroxy, cyano, NH₂, NHCOCH₃, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, N-methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and N-methylpyperazine;
R₄ is an aromatic or heteroaromatic ring optionally substituted with one or more substituents each independently selected from halogen, OH, C₁₋₆alkyl, haloC₁₋₆alkyl, C₁₋₆alkoxy, cyano, NH₂, NHC(O)C₁₋₆alkyl, NHC₁₋₃alkyl, N(C₁₋₃alkyl)₂, aziridine, azetidine, pyrrolidine, pyperidine, morpholine, piperazine and N-methylpyperazine;
R₅ is H, C₁₋₆alkyl, C₁₋₆haloalkyl or halogen;
or a pharmaceutically acceptable salt, tautomer, solvate, or stereoisomer thereof for use in the treatment of Flavivirus infection, preferably wherein the Flavivirus is selected from the group consisting of Dengue, West Nile, Zika and Japan Encephalitis virus.

10. The compound for use according to claim 9 wherein:
- R_{1b} and R_{2b} are H; and/or
- m and n are each independently selected from 0, 1; and/or
- R₃ is a phenyl, naphtyl, biphenyl, phenyl-heteroaryl or bis-heteroaryl wherein each of said phenyl, naphtyl or heteroaryl ring is optionally substituted with one or more substituents each independently selected from halogen, hydroxy, cyano, B(OH)₂, NH₂, NHCOCH₃, NHCOOC₁₋₆alkyl, NHC₁₋₃alkyl, N(C₁₋₃alkyl)₂, C₁₋₆alkyl, C₁₋₆alkoxy, pyrrolidine, pyperidine, morpholine, cyclopropyl, cyclobutyl, cyclopentyl, cyclohexyl, wherein said C₁₋₆alkyl, C₁₋₆alkoxy are optionally substituted with one or more halogen, hydroxy, NH₂, N(CH₃)₂, morpholine or pyrrolidine; and/or
- R₄ is selected from 3-trifluoromethylphenyl, naphthyl, 5-(trifluoromethyl)thiazol-2-yl, 3-chlorophenyl, 3-fluoro-5-trifluoromethylphenyl, quinolinyl, 5-(trifluoromethyl)pyridin-3-yl, 4-(trifluoromethyl)pyridin-2-yl, 3-methyl-5-trifluoromethylphenyl, 3-methoxy-5-trifluoromethylphenyl, 3-cyanophenyl.

11. The compound for use according to any one of claims 9 and 10 selected from:
- (3-(1-phenylpropan-2-yl)-1,2,3 -oxadiazol -3 -ium-5 -yl)((3 -(trifluoromethyl) phenyl)carbamoyl)amide;
- (3-phenethyl-1,2,3-oxadiazol-3-ium-5-yl)((3-(trifluoromethyl)phenyl) carbamoyl)amide.

12. The compound according to any one of claims 7-11 for use in combination with at least one further therapeutic agent.

13. A pharmaceutical composition comprising the compound according to any one of claims 1 to 8 or the compound for use according to any one of claims 9 to 12, and at least one pharmaceutically acceptable excipient.

14. The pharmaceutical composition of claim 13 for use in the treatment and/or prevention of a Flavivirus infection, preferably wherein the Flavivirus is selected from the group consisting of Dengue, West Nile, Zika and Japan Encephalitis virus.
